# EUROPEAN PATENT SPECIFICATION

(11) **EP 3 331 915 B1**
(45) Date of publication and mention of the grant of the patent: **17.02.2021**
(21) Application number: 16757165.2
(22) Date of filing: 05.08.2016
(51) Int. Cl.: C07K 16/28, G01N 33/574, A61K 39/395, A61P 35/02

(54) **IMMUNE-CHECKPOINT INHIBITORS FOR USE IN THE TREATMENT OF BLOOD-BORNE CANCERS**
IMMUN-CHECKPOINT-INHIBITOREN ZUR BEHANDLUNG VON BLUTKREBS
INHIBITEURS DE POINTS DE CONTRÔLE IMMUNITAIRES DESTINÉS À ÊTRE UTILISÉS DANS LE TRAITEMENT DE CANCERS À DIFFUSION HÉMATOGÈNE

(30) Priority: 05.08.2015 US 201562201461 P
(43) Date of publication of application: 13.06.2018
(73) Proprietor: Amgen Research (Munich) GmbH, 81477 München (DE)
(72) Inventor: FIEDLER, Walter, 20251 Hamburg (DE); WELLBROCK, Jasmin, 22083 Hamburg (DE); STAMM, Hauke, 20146 Hamburg (DE); KLINGLER, Felix, 21244 Buchholz (DE)
(74) Representative: Schiweck Weinzierl Koch Patentanwälte Partnerschaft mbB
(86) International application number: PCT/EP2016/068726
(87) International publication number: WO 2017/021526

(56) References cited:
- EP-A1- 2 067 791
- WO-A1-2012/177788
- WO-A1-2015/048312
- WO-A1-2015/117002
- WO-A2-2004/074324
- WO-A2-2009/097394
- WO-A2-2011/159877
- WO-A2-2015/009856
- PENDE DANIELA ET AL: "PVR (CD155) and Nectin-2 (CD112) as ligands of the human DNAM-1 (CD226) activating receptor: involvement in tumor cell lysis.", MOLECULAR IMMUNOLOGY FEB 2005, vol. 42, no. 4, February 2005 (2005-02), pages 463-469, XP002763679, ISSN: 0161-5890
- HOU SHENGKE ET AL: "Recombinant soluble CD226 protein directly inhibits cancer cell proliferation in vitro.", INTERNATIONAL IMMUNOPHARMACOLOGY MAR 2014, vol. 19, no. 1, March 2014 (2014-03), pages 119-126, XP002763680, ISSN: 1878-1705
- KONG YAXIAN ET AL: "T-Cell Immunoglobulin and ITIM Domain (TIGIT) Associates with CD8+ T-Cell Exhaustion and Poor Clinical Outcome in AML Patients.", CLINICAL CANCER RESEARCH : AN OFFICIAL JOURNAL OF THE AMERICAN ASSOCIATION FOR CANCER RESEARCH 15 JUN 2016, vol. 22, no. 12, 15 June 2016 (2016-06-15) , pages 3057-3066, XP002763681, ISSN: 1078-0432
- STAMM HAUKE ET AL: "Expression of Novel Immune Checkpoint Molecules PVR and PVRL2 Confers a Negative Prognosis to Patients with Acute Myeloid Leukemia and Their Blockade Augments T-Cell Mediated Lysis of AML Cells Alone or in Combination with the BiTE Antibody Construct AMG 330", BLOOD, vol. 126, no. 23, December 2015 (2015-12), XP002763682, & 57TH ANNUAL MEETING OF THE AMERICAN-SOCIETY-OF-HEMATOLOGY; ORLANDO, FL, USA; DECEMBER 05 -08, 2015
- KRUPKA C ET AL: "Blockade of the PD-1/PD-L1 axis augments lysis of AML cells by the CD33/CD3 BiTE antibody construct AMG 330: reversing a T-cell-induced immune escape mechanism.", LEUKEMIA FEB 2016, vol. 30, no. 2, 4 August 2015 (2015-08-04) , pages 484-491, XP002763683, ISSN: 1476-5551
- LANDRY BREANNE ET AL: "Progress in RNAi-mediated Molecular Therapy of Acute and Chronic Myeloid Leukemia.", MOLECULAR THERAPY. NUCLEIC ACIDS 2015, vol. 4, 12 May 2015 (2015-05-12), page e240, XP002763684, ISSN: 2162-2531
- Magee: "https://www.ncbi.nlm.nih.gov/pmc/articles /PMC3991216/pdf/2051-1426-1-S1-P22.pdf", https://www.ncbi.nlm.nih.gov/pmc/articles/ PMC3991216/pdf/2051-1426-1-S1-P22.pdf , 7 November 2013 (2013-11-07), XP002763686, Retrieved from the Internet: URL:https://www.ncbi.nlm.nih.gov/pmc/artic les/PMC3991216/pdf/2051-1426-1-S1-P22.pdf [retrieved on 2016-11-01]
- JOHN LIZA B ET AL: "Blockade of PD-1 immunosuppression boosts CAR T-cell therapy", ONCOIMMUNOLOGY, vol. 2, no. 10, October 2013 (2013-10), XP002765631,
- SANCHEZ-CORREA BEATRIZ ET AL: "Natural killer cell immunosenescence in acute myeloid leukaemia patients: new targets for immunotherapeutic strategies?", CANCER IMMUNOLOGY, IMMUNOTHERAPY : CII APR 2016, vol. 65, no. 4, 10 June 2015 (2015-06-10), pages 453-463, XP002763685, ISSN: 1432-0851
- ZHU YUWEN ET AL: "Identification of CD112R as a novel checkpoint for human T cells.", THE JOURNAL OF EXPERIMENTAL MEDICINE 8 FEB 2016, vol. 213, no. 2, 8 February 2016 (2016-02-08), pages 167-176, XP002763687, ISSN: 1540-9538
- Q. ZHOU ET AL: "Coexpression of Tim-3 and PD-1 identifies a CD8+ T-cell exhaustion phenotype in mice with disseminated acute myelogenous leukemia", BLOOD, vol. 117, no. 17, 8 March 2011 (2011-03-08), pages 4501-4510, XP055147794, ISSN: 0006-4971, DOI: 10.1182/blood-2010-10-310425
- KURODA JUNYA ET AL: "Targeting activating transcription factor 3 by Galectin-9 induces apoptosis and overcomes various types of treatment resistance in chronic myelogenous leukemia.", MOLECULAR CANCER RESEARCH : MCR JUL 2010, vol. 8, no. 7, July 2010 (2010-07), pages 994-1001, XP002765632, ISSN: 1557-3125
- YOSHIKANE KIKUSHIGE ET AL: "TIM-3 as a therapeutic target for malignant stem cells in acute myelogenous leukemia", ANNALS OF THE NEW YORK ACADEMY OF SCIENCES, vol. 1266, no. 1, 17 August 2012 (2012-08-17), pages 118-123, XP055087894, ISSN: 0077-8923, DOI: 10.1111/j.1749-6632.2012.06550.x
- DATABASE BIOSIS [Online] BIOSCIENCES INFORMATION SERVICE, PHILADELPHIA, PA, US; December 2014 (2014-12), VARGHESE BINDU ET AL: "A Novel CD20xCD3 Bispecific Fully Human Antibody Induces Potent Anti-Tumor Effects Against B Cell Lymphoma in Mice", XP002765634, Database accession no. PREV201500281422 & BLOOD, vol. 124, no. 21, December 2014 (2014-12), 56TH ANNUAL MEETING OF THE AMERICAN-SOCIETY-OF-HEMATOLOGY; SAN FRANCISCO, CA, USA; DECEMBER 06 -09, 2014 ISSN: 0006-4971(print)
- STAMM HAUKE ET AL: "Immune checkpoints PVR and PVRL2 are prognostic markers in AML and their blockade represents a new therapeutic option", ONCOGENE, NATURE PUBLISHING GROUP UK, LONDON, vol. 37, no. 39, 31 May 2018 (2018-05-31), pages 5269-5280, XP036601975, ISSN: 0950-9232, DOI: 10.1038/S41388-018-0288-Y [retrieved on 2018-05-31]

### SEQUENCE LISTING

<110> Amgen Research (Munich) GmbH
<120> IMMUNE-CHECKPOINT INHIBITORS FOR USE IN THE TREATMENT OF BLOOD-BORNE
   CANCERS
<130> AMG15434PCT
<150> US 62/201,461
   <151> 2015-08-05
<160> 37
<170> Patent In version 3.5
<210> 1
   <211> 20
   <212> DNA
   <213> Artificial Sequence
<220>
   <223> Synthetic Primer
<400> 1
   agcaggagcg tggatatctg 20
<210> 2
   <211> 20
   <212> DNA
   <213> Artificial Sequence
<220>
   <223> Synthetic Primer
<400> 2
   gactgtgcca gacaggaacc 20
<210> 3
   <211> 20
   <212> DNA
   <213> Artificial Sequence
<220>
   <223> Synthetic Primer
<400> 3
   gaggacgagg gcaactacac 20
<210> 4
   <211> 20
   <212> DNA
   <213> Artificial Sequence
<220>
   <223> Synthetic Primer
<400> 4
   agggatgaga gccaggagat 20
<210> 5
   <211> 20
   <212> DNA
   <213> Artificial Sequence
<220>
   <223> Synthetic Primer
<400> 5
   gtctccagga cggacttcag 20
<210> 6
   <211> 20
   <212> DNA
   <213> Artificial Sequence
<220>
   <223> Synthetic Primer
<400> 6
   caggaagcag aggtcaaagg 20
<210> 7
   <211> 20
   <212> DNA
   <213> Artificial Sequence
<220>
   <223> Synthetic Primer
<400> 7
   gtcagtggtg gacctgacct 20
<210> 8
   <211> 20
   <212> DNA
   <213> Artificial Sequence
<220>
   <223> Synthetic Primer
<400> 8
   tgctgtagcc aaattcgttg 20
<210> 9
   <211> 5
   <212> PRT
   <213> Artificial Sequence
<220>
   <223> Synthetic Linker
<400> 9
   Gly Gly Gly Gly Ser
   1 5
<210> 10
   <211> 4
   <212> PRT
   <213> Artificial Sequence
<220>
   <223> Synthetic Linker
<400> 10
   Gly Gly Gly Gly
   1
<210> 11
   <211> 5
   <212> PRT
   <213> Artificial Sequence
<220>
   <223> Synthetic Linker
<400> 11
   Gly Gly Gly Gly Gln
   1 5
<210> 12
   <211> 6
   <212> PRT
   <213> Artificial Sequence
<220>
   <223> Synthetic Linker
<400> 12
   Pro Gly Gly Gly Gly Ser
   1 5
<210> 13
   <211> 6
   <212> PRT
   <213> Artificial Sequence
<220>
   <223> Synthetic Linker
<400> 13
   Pro Gly Gly Asp Gly Ser
   1               5
<210> 14
   <211> 6
   <212> PRT
   <213> Artificial Sequence
<220>
   <223> Synthetic Linker
<400> 14
   Ser Gly Gly Gly Gly Ser
   1               5
<210> 15
   <211> 505
   <212> PRT
   <213> artificial
<220>
   <223> CD33xCD3 bispecific antibody construct
<400> 15
   Gln Val Gln Leu Val Gln Ser Gly Ala Glu Val Lys Lys Pro Gly Glu
   1               5                   10                  15
   Ser Val Lys Val Ser Cys Lys Ala Ser Gly Tyr Thr Phe Thr Asn Tyr
               20                  25                  30
   Gly Met Asn Trp Val Lys Gln Ala Pro Gly Gln Gly Leu Glu Trp Met
           35                  40                  45
   Gly Trp Ile Asn Thr Tyr Thr Gly Glu Pro Thr Tyr Ala Asp Lys Phe
       50                  55                  60
   Gln Gly Arg Val Thr Met Thr Thr Asp Thr Ser Thr Ser Thr Ala Tyr
   65                  70                  75                  80
   Met Glu Ile Arg Asn Leu Gly Gly Asp Asp Thr Ala Val Tyr Tyr Cys
                   85                  90                  95
   Ala Arg Trp Ser Trp Ser Asp Gly Tyr Tyr Val Tyr Phe Asp Tyr Trp
               100                 105                 110
   Gly Gln Gly Thr Ser Val Thr Val Ser Ser Gly Gly Gly Gly Ser Gly
           115                 120                 125
   Gly Gly Gly Ser Gly Gly Gly Gly Ser Asp Ile Val Met Thr Gln Ser
       130                 135                 140
   Pro Asp Ser Leu Thr Val Ser Leu Gly Glu Arg Thr Thr Ile Asn Cys
   145                 150                 155                 160
   Lys Ser Ser Gln Ser Val Leu Asp Ser Ser Thr Asn Lys Asn Ser Leu
                   165                 170                 175
   Ala Trp Tyr Gln Gln Lys Pro Gly Gln Pro Pro Lys Leu Leu Leu Ser
               180                 185                 190
   Trp Ala Ser Thr Arg Glu Ser Gly Ile Pro Asp Arg Phe Ser Gly Ser
           195                 200                 205
   Gly Ser Gly Thr Asp Phe Thr Leu Thr Ile Asp Ser Pro Gln Pro Glu
       210                 215                 220
   Asp Ser Ala Thr Tyr Tyr Cys Gln Gln Ser Ala His Phe Pro Ile Thr
   225                 230                 235                 240
   Phe Gly Gln Gly Thr Arg Leu Glu Ile Lys Ser Gly Gly Gly Gly Ser
                   245                 250                 255
   Glu Val Gln Leu Val Glu Ser Gly Gly Gly Leu Val Gln Pro Gly Gly
               260                 265                 270
   Ser Leu Lys Leu Ser Cys Ala Ala Ser Gly Phe Thr Phe Asn Lys Tyr
           275                 280                 285
   Ala Met Asn Trp Val Arg Gln Ala Pro Gly Lys Gly Leu Glu Trp Val
       290                 295                 300
   Ala Arg Ile Arg Ser Lys Tyr Asn Asn Tyr Ala Thr Tyr Tyr Ala Asp
   305                 310                 315                 320
   Ser Val Lys Asp Arg Phe Thr Ile Ser Arg Asp Asp Ser Lys Asn Thr
                   325                 330                 335
   Ala Tyr Leu Gln Met Asn Asn Leu Lys Thr Glu Asp Thr Ala Val Tyr
               340                 345                 350
   Tyr Cys Val Arg His Gly Asn Phe Gly Asn Ser Tyr Ile Ser Tyr Trp
           355                 360                 365
   Ala Tyr Trp Gly Gln Gly Thr Leu Val Thr Val Ser Ser Gly Gly Gly
       370                 375                 380
   Gly Ser Gly Gly Gly Gly Ser Gly Gly Gly Gly Ser Gln Thr Val Val
   385                 390                 395                 400
   Thr Gln Glu Pro Ser Leu Thr Val Ser Pro Gly Gly Thr Val Thr Leu
                   405                 410                 415
   Thr Cys Gly Ser Ser Thr Gly Ala Val Thr Ser Gly Asn Tyr Pro Asn
               420                 425                 430
   Trp Val Gln Gln Lys Pro Gly Gln Ala Pro Arg Gly Leu Ile Gly Gly
                   435                 440                 445
   Thr Lys Phe Leu Ala Pro Gly Thr Pro Ala Arg Phe Ser Gly Ser Leu
       450                 455                 460
   Leu Gly Gly Lys Ala Ala Leu Thr Leu Ser Gly Val Gln Pro Glu Asp
   465                 470                 475                 480
   Glu Ala Glu Tyr Tyr Cys Val Leu Trp Tyr Ser Asn Arg Trp Val Phe
                   485                 490                 495
   Gly Gly Gly Thr Lys Leu Thr Val Leu
               500                 505
<210> 16
   <211> 993
   <212> PRT
   <213> artificial
<220>
   <223> CD33xCD3 bispecific HLE 1
<400> 16
   Gln Val Gln Leu Val Gln Ser Gly Ala Glu Val Lys Lys Pro Gly Glu
   1               5                   10                  15
   Ser Val Lys Val Ser Cys Lys Ala Ser Gly Tyr Thr Phe Thr Asn Tyr
               20                  25                  30
   Gly Met Asn Trp Val Lys Gln Ala Pro Gly Gln Gly Leu Glu Trp Met
           35                  40                  45
   Gly Trp Ile Asn Thr Tyr Thr Gly Glu Pro Thr Tyr Ala Asp Lys Phe
       50                  55                  60
   Gln Gly Arg Val Thr Met Thr Thr Asp Thr Ser Thr Ser Thr Ala Tyr
   65                  70                  75                  80
   Met Glu Ile Arg Asn Leu Gly Gly Asp Asp Thr Ala Val Tyr Tyr Cys
                   85                  90                  95
   Ala Arg Trp Ser Trp Ser Asp Gly Tyr Tyr Val Tyr Phe Asp Tyr Trp
               100                 105                 110
   Gly Gln Gly Thr Ser Val Thr Val Ser Ser Gly Gly Gly Gly Ser Gly
           115                 120                 125
   Gly Gly Gly Ser Gly Gly Gly Gly Ser Asp Ile Val Met Thr Gln Ser
       130                 135                 140
   Pro Asp Ser Leu Thr Val Ser Leu Gly Glu Arg Thr Thr Ile Asn Cys
   145                 150                 155                 160
   Lys Ser Ser Gln Ser Val Leu Asp Ser Ser Thr Asn Lys Asn Ser Leu
                   165                 170                 175
   Ala Trp Tyr Gln Gln Lys Pro Gly Gln Pro Pro Lys Leu Leu Leu Ser
               180                 185                 190
   Trp Ala Ser Thr Arg Glu Ser Gly Ile Pro Asp Arg Phe Ser Gly Ser
           195                 200                 205
   Gly Ser Gly Thr Asp Phe Thr Leu Thr Ile Asp Ser Pro Gln Pro Glu
       210                 215                 220
   Asp Ser Ala Thr Tyr Tyr Cys Gln Gln Ser Ala His Phe Pro Ile Thr
   225                 230                 235                 240
   Phe Gly Gln Gly Thr Arg Leu Glu Ile Lys Ser Gly Gly Gly Gly Ser
                   245                 250                 255
   Glu Val Gln Leu Val Glu Ser Gly Gly Gly Leu Val Gln Pro Gly Gly
               260                 265                 270
   Ser Leu Lys Leu Ser Cys Ala Ala Ser Gly Phe Thr Phe Asn Lys Tyr
           275                 280                 285
   Ala Met Asn Trp Val Arg Gln Ala Pro Gly Lys Gly Leu Glu Trp Val
       290                 295                 300
   Ala Arg Ile Arg Ser Lys Tyr Asn Asn Tyr Ala Thr Tyr Tyr Ala Asp
   305                 310                 315                 320
   Ser Val Lys Asp Arg Phe Thr Ile Ser Arg Asp Asp Ser Lys Asn Thr
                   325                 330                 335
   Ala Tyr Leu Gln Met Asn Asn Leu Lys Thr Glu Asp Thr Ala Val Tyr
               340                 345                 350
   Tyr Cys Val Arg His Gly Asn Phe Gly Asn Ser Tyr Ile Ser Tyr Trp
           355                 360                 365
   Ala Tyr Trp Gly Gln Gly Thr Leu Val Thr Val Ser Ser Gly Gly Gly
       370                 375                 380
   Gly Ser Gly Gly Gly Gly Ser Gly Gly Gly Gly Ser Gln Thr Val Val
   385 390 395 400
   Thr Gln Glu Pro Ser Leu Thr Val Ser Pro Gly Gly Thr Val Thr Leu
   405 410 415
   Thr Cys Gly Ser Ser Thr Gly Ala Val Thr Ser Gly Asn Tyr Pro Asn
   420 425 430
   Trp Val Gln Gln Lys Pro Gly Gln Ala Pro Arg Gly Leu Ile Gly Gly
   435 440 445
   Thr Lys Phe Leu Ala Pro Gly Thr Pro Ala Arg Phe Ser Gly Ser Leu
   450 455 460
   Leu Gly Gly Lys Ala Ala Leu Thr Leu Ser Gly Val Gln Pro Glu Asp
   465 470 475 480
   Glu Ala Glu Tyr Tyr Cys Val Leu Trp Tyr Ser Asn Arg Trp Val Phe
   485 490 495
   Gly Gly Gly Thr Lys Leu Thr Val Leu Gly Gly Gly Gly Asp Lys Thr
   500 505 510
   His Thr Cys Pro Pro Cys Pro Ala Pro Glu Leu Leu Gly Gly Pro Ser
   515 520 525
   Val Phe Leu Phe Pro Pro Lys Pro Lys Asp Thr Leu Met Ile Ser Arg
   530 535 540
   Thr Pro Glu Val Thr Cys Val Val Val Asp Val Ser His Glu Asp Pro
   545 550 555 560
   Glu Val Lys Phe Asn Trp Tyr Val Asp Gly Val Glu Val His Asn Ala
   565 570 575
   Lys Thr Lys Pro Cys Glu Glu Gln Tyr Gly Ser Thr Tyr Arg Cys Val
   580 585 590
   Ser Val Leu Thr Val Leu His Gln Asp Trp Leu Asn Gly Lys Glu Tyr
   595 600 605
   Lys Cys Lys Val Ser Asn Lys Ala Leu Pro Ala Pro Ile Glu Lys Thr
   610 615 620
   Ile Ser Lys Ala Lys Gly Gln Pro Arg Glu Pro Gln Val Tyr Thr Leu
   625 630 635 640
   Pro Pro Ser Arg Glu Glu Met Thr Lys Asn Gln Val Ser Leu Thr Cys
                   645                 650                 655
   Leu Val Lys Gly Phe Tyr Pro Ser Asp Ile Ala Val Glu Trp Glu Ser
               660                 665                 670
   Asn Gly Gln Pro Glu Asn Asn Tyr Lys Thr Thr Pro Pro Val Leu Asp
           675                 680                 685
   Ser Asp Gly Ser Phe Phe Leu Tyr Ser Lys Leu Thr Val Asp Lys Ser
       690                 695                 700
   Arg Trp Gln Gln Gly Asn Val Phe Ser Cys Ser Val Met His Glu Ala
   705                 710                 715                 720
   Leu His Asn His Tyr Thr Gln Lys Ser Leu Ser Leu Ser Pro Gly Lys
                   725                 730                 735
   Gly Gly Gly Gly Ser Gly Gly Gly Gly Ser Gly Gly Gly Gly Ser Gly
               740                 745                 750
   Gly Gly Gly Ser Gly Gly Gly Gly Ser Gly Gly Gly Gly Ser Asp Lys
           755                 760                 765
   Thr His Thr Cys Pro Pro Cys Pro Ala Pro Glu Leu Leu Gly Gly Pro
       770                 775                 780
   Ser Val Phe Leu Phe Pro Pro Lys Pro Lys Asp Thr Leu Met Ile Ser
   785                 790                 795                 800
   Arg Thr Pro Glu Val Thr Cys Val Val Val Asp Val Ser His Glu Asp
                   805                 810                 815
   Pro Glu Val Lys Phe Asn Trp Tyr Val Asp Gly Val Glu Val His Asn
               820                 825                 830
   Ala Lys Thr Lys Pro Cys Glu Glu Gln Tyr Gly Ser Thr Tyr Arg Cys
           835                 840                 845
   Val Ser Val Leu Thr Val Leu His Gln Asp Trp Leu Asn Gly Lys Glu
       850                 855                 860
   Tyr Lys Cys Lys Val Ser Asn Lys Ala Leu Pro Ala Pro Ile Glu Lys
   865                 870                 875                 880
   Thr Ile Ser Lys Ala Lys Gly Gln Pro Arg Glu Pro Gln Val Tyr Thr
                   885                 890                 895
   Leu Pro Pro Ser Arg Glu Glu Met Thr Lys Asn Gln Val Ser Leu Thr
   900 905 910
   Cys Leu Val Lys Gly Phe Tyr Pro Ser Asp Ile Ala Val Glu Trp Glu
   915 920 925
   Ser Asn Gly Gln Pro Glu Asn Asn Tyr Lys Thr Thr Pro Pro Val Leu
   930 935 940
   Asp Ser Asp Gly Ser Phe Phe Leu Tyr Ser Lys Leu Thr Val Asp Lys
   945 950 955 960
   Ser Arg Trp Gln Gln Gly Asn Val Phe Ser Cys Ser Val Met His Glu
   965 970 975
   Ala Leu His Asn His Tyr Thr Gln Lys Ser Leu Ser Leu Ser Pro Gly
   980 985 990
   Lys
<210> 17
   <211> 991
   <212> PRT
   <213> artificial
<220>
   <223> CD33xCD3 bispecific HLE 2
<400> 17
   Gln Val Gln Leu Val Gln Ser Gly Ala Glu Val Lys Lys Pro Gly Glu
   1 5 10 15
   Ser Val Lys Val Ser Cys Lys Ala Ser Gly Tyr Thr Phe Thr Asn Tyr
   20 25 30
   Gly Met Asn Trp Val Lys Gln Ala Pro Gly Gln Gly Leu Glu Trp Met
   35 40 45
   Gly Trp Ile Asn Thr Tyr Thr Gly Glu Pro Thr Tyr Ala Asp Lys Phe
   50 55 60
   Gln Gly Arg Val Thr Met Thr Thr Asp Thr Ser Thr Ser Thr Ala Tyr
   65 70 75 80
   Met Glu Ile Arg Asn Leu Gly Gly Asp Asp Thr Ala Val Tyr Tyr Cys
   85 90 95
   Ala Arg Trp Ser Trp Ser Asp Gly Tyr Tyr Val Tyr Phe Asp Tyr Trp
               100                 105                 110
   Gly Gln Gly Thr Ser Val Thr Val Ser Ser Gly Gly Gly Gly Ser Gly
           115                 120                 125
   Gly Gly Gly Ser Gly Gly Gly Gly Ser Asp Ile Val Met Thr Gln Ser
       130                 135                 140
   Pro Asp Ser Leu Thr Val Ser Leu Gly Glu Arg Thr Thr Ile Asn Cys
   145                 150                 155                 160
   Lys Ser Ser Gln Ser Val Leu Asp Ser Ser Thr Asn Lys Asn Ser Leu
                   165                 170                 175
   Ala Trp Tyr Gln Gln Lys Pro Gly Gln Pro Pro Lys Leu Leu Leu Ser
               180                 185                 190
   Trp Ala Ser Thr Arg Glu Ser Gly Ile Pro Asp Arg Phe Ser Gly Ser
           195                 200                 205
   Gly Ser Gly Thr Asp Phe Thr Leu Thr Ile Asp Ser Pro Gln Pro Glu
       210                 215                 220
   Asp Ser Ala Thr Tyr Tyr Cys Gln Gln Ser Ala His Phe Pro Ile Thr
   225                 230                 235                 240
   Phe Gly Gln Gly Thr Arg Leu Glu Ile Lys Ser Gly Gly Gly Gly Ser
                   245                 250                 255
   Glu Val Gln Leu Val Glu Ser Gly Gly Gly Leu Val Gln Pro Gly Gly
               260                 265                 270
   Ser Leu Lys Leu Ser Cys Ala Ala Ser Gly Phe Thr Phe Asn Lys Tyr
           275                 280                 285
   Ala Met Asn Trp Val Arg Gln Ala Pro Gly Lys Gly Leu Glu Trp Val
       290                 295                 300
   Ala Arg Ile Arg Ser Lys Tyr Asn Asn Tyr Ala Thr Tyr Tyr Ala Asp
   305                 310                 315                 320
   Ser Val Lys Asp Arg Phe Thr Ile Ser Arg Asp Asp Ser Lys Asn Thr
                   325                 330                 335
   Ala Tyr Leu Gln Met Asn Asn Leu Lys Thr Glu Asp Thr Ala Val Tyr
               340                 345                 350
   Tyr Cys Val Arg His Gly Asn Phe Gly Asn Ser Tyr Ile Ser Tyr Trp
               355                 360                 365
   Ala Tyr Trp Gly Gln Gly Thr Leu Val Thr Val Ser Ser Gly Gly Gly
           370                 375                 380
   Gly Ser Gly Gly Gly Gly Ser Gly Gly Gly Gly Ser Gln Thr Val Val
   385                 390                 395                 400
   Thr Gln Glu Pro Ser Leu Thr Val Ser Pro Gly Gly Thr Val Thr Leu
                   405                 410                 415
   Thr Cys Gly Ser Ser Thr Gly Ala Val Thr Ser Gly Asn Tyr Pro Asn
               420                 425                 430
   Trp Val Gln Gln Lys Pro Gly Gln Ala Pro Arg Gly Leu Ile Gly Gly
           435                 440                 445
   Thr Lys Phe Leu Ala Pro Gly Thr Pro Ala Arg Phe Ser Gly Ser Leu
       450                 455                 460
   Leu Gly Gly Lys Ala Ala Leu Thr Leu Ser Gly Val Gln Pro Glu Asp
   465                 470                 475                 480
   Glu Ala Glu Tyr Tyr Cys Val Leu Trp Tyr Ser Asn Arg Trp Val Phe
                   485                 490                 495
   Gly Gly Gly Thr Lys Leu Thr Val Leu Gly Gly Gly Gly Asp Lys Thr
               500                 505                 510
   His Thr Cys Pro Pro Cys Pro Ala Pro Glu Leu Leu Gly Gly Pro Ser
           515                 520                 525
   Val Phe Leu Phe Pro Pro Lys Pro Lys Asp Thr Leu Met Ile Ser Arg
       530                 535                 540
   Thr Pro Glu Val Thr Cys Val Val Val Asp Val Ser His Glu Asp Pro
   545                 550                 555                 560
   Glu Val Lys Phe Asn Trp Tyr Val Asp Gly Val Glu Val His Asn Ala
                   565                 570                 575
   Lys Thr Lys Pro Cys Glu Glu Gln Tyr Gly Ser Thr Tyr Arg Cys Val
               580                 585                 590
   Ser Val Leu Thr Val Leu His Gln Asp Trp Leu Asn Gly Lys Glu Tyr
           595                 600                 605
   Lys Cys Lys Val Ser Asn Lys Ala Leu Pro Ala Pro Ile Glu Lys Thr
       610                 615                 620
   Ile Ser Lys Ala Lys Gly Gln Pro Arg Glu Pro Gln Val Tyr Thr Leu
   625                 630                 635                 640
   Pro Pro Ser Arg Glu Glu Met Thr Lys Asn Gln Val Ser Leu Thr Cys
                   645                 650                 655
   Leu Val Lys Gly Phe Tyr Pro Ser Asp Ile Ala Val Glu Trp Glu Ser
               660                 665                 670
   Asn Gly Gln Pro Glu Asn Asn Tyr Lys Thr Thr Pro Pro Val Leu Asp
           675                 680                 685
   Ser Asp Gly Ser Phe Phe Leu Tyr Ser Lys Leu Thr Val Asp Lys Ser
       690                 695                 700
   Arg Trp Gln Gln Gly Asn Val Phe Ser Cys Ser Val Met His Glu Ala
   705                 710                 715                 720
   Leu His Asn His Tyr Thr Gln Lys Ser Leu Ser Leu Ser Pro Gly Gly
                   725                 730                 735
   Gly Gly Ser Gly Gly Gly Gly Ser Gly Gly Gly Gly Ser Gly Gly Gly
               740                 745                 750
   Gly Ser Gly Gly Gly Gly Ser Gly Gly Gly Gly Ser Asp Lys Thr His
           755                 760                 765
   Thr Cys Pro Pro Cys Pro Ala Pro Glu Leu Leu Gly Gly Pro Ser Val
       770                 775                 780
   Phe Leu Phe Pro Pro Lys Pro Lys Asp Thr Leu Met Ile Ser Arg Thr
   785                 790                 795                 800
   Pro Glu Val Thr Cys Val Val Val Asp Val Ser His Glu Asp Pro Glu
                   805                 810                 815
   Val Lys Phe Asn Trp Tyr Val Asp Gly Val Glu Val His Asn Ala Lys
               820                 825                 830
   Thr Lys Pro Cys Glu Glu Gln Tyr Gly Ser Thr Tyr Arg Cys Val Ser
           835                 840                 845
   Val Leu Thr Val Leu His Gln Asp Trp Leu Asn Gly Lys Glu Tyr Lys
   850 855 860
   Cys Lys Val Ser Asn Lys Ala Leu Pro Ala Pro Ile Glu Lys Thr Ile
   865 870 875 880
   Ser Lys Ala Lys Gly Gln Pro Arg Glu Pro Gln Val Tyr Thr Leu Pro
   885 890 895
   Pro Ser Arg Glu Glu Met Thr Lys Asn Gln Val Ser Leu Thr Cys Leu
   900 905 910
   Val Lys Gly Phe Tyr Pro Ser Asp Ile Ala Val Glu Trp Glu Ser Asn
   915 920 925
   Gly Gln Pro Glu Asn Asn Tyr Lys Thr Thr Pro Pro Val Leu Asp Ser
   930 935 940
   Asp Gly Ser Phe Phe Leu Tyr Ser Lys Leu Thr Val Asp Lys Ser Arg
   945 950 955 960
   Trp Gln Gln Gly Asn Val Phe Ser Cys Ser Val Met His Glu Ala Leu
   965 970 975
   His Asn His Tyr Thr Gln Lys Ser Leu Ser Leu Ser Pro Gly Lys
   980 985 990
<210> 18
   <211> 505
   <212> PRT
   <213> artificial
<220>
   <223> CD33xCD3 bispecific antibody construct
<400> 18
   Gln Val Gln Leu Val Gln Ser Gly Ala Glu Val Lys Lys Pro Gly Glu
   1               5                   10                  15
   Ser Val Lys Val Ser Cys Lys Ala Ser Gly Tyr Thr Phe Thr Asn Tyr
               20                  25                  30
   Gly Met Asn Trp Val Lys Gln Ala Pro Gly Gln Cys Leu Glu Trp Met
           35                  40                  45
   Gly Trp Ile Asn Thr Tyr Thr Gly Glu Pro Thr Tyr Ala Asp Lys Phe
       50                  55                  60
   Gln Gly Arg Val Thr Met Thr Thr Asp Thr Ser Thr Ser Thr Ala Tyr
   65                  70                  75                  80
   Met Glu Ile Arg Asn Leu Gly Gly Asp Asp Thr Ala Val Tyr Tyr Cys
   85 90 95
   Ala Arg Trp Ser Trp Ser Asp Gly Tyr Tyr Val Tyr Phe Asp Tyr Trp
   100 105 110
   Gly Gln Gly Thr Ser Val Thr Val Ser Ser Gly Gly Gly Gly Ser Gly
   115 120 125
   Gly Gly Gly Ser Gly Gly Gly Gly Ser Asp Ile Val Met Thr Gln Ser
   130 135 140
   Pro Asp Ser Leu Thr Val Ser Leu Gly Glu Arg Thr Thr Ile Asn Cys
   145 150 155 160
   Lys Ser Ser Gln Ser Val Leu Asp Ser Ser Thr Asn Lys Asn Ser Leu
   165 170 175
   Ala Trp Tyr Gln Gln Lys Pro Gly Gln Pro Pro Lys Leu Leu Leu Ser
   180 185 190
   Trp Ala Ser Thr Arg Glu Ser Gly Ile Pro Asp Arg Phe Ser Gly Ser
   195 200 205
   Gly Ser Gly Thr Asp Phe Thr Leu Thr Ile Asp Ser Pro Gln Pro Glu
   210 215 220
   Asp Ser Ala Thr Tyr Tyr Cys Gln Gln Ser Ala His Phe Pro Ile Thr
   225 230 235 240
   Phe Gly Cys Gly Thr Arg Leu Glu Ile Lys Ser Gly Gly Gly Gly Ser
   245 250 255
   Glu Val Gln Leu Val Glu Ser Gly Gly Gly Leu Val Gln Pro Gly Gly
   260 265 270
   Ser Leu Lys Leu Ser Cys Ala Ala Ser Gly Phe Thr Phe Asn Lys Tyr
   275 280 285
   Ala Met Asn Trp Val Arg Gln Ala Pro Gly Lys Gly Leu Glu Trp Val
   290 295 300
   Ala Arg Ile Arg Ser Lys Tyr Asn Asn Tyr Ala Thr Tyr Tyr Ala Asp
   305 310 315 320
   Ser Val Lys Asp Arg Phe Thr Ile Ser Arg Asp Asp Ser Lys Asn Thr
   325 330 335
   Ala Tyr Leu Gln Met Asn Asn Leu Lys Thr Glu Asp Thr Ala Val Tyr
   340 345 350
   Tyr Cys Val Arg His Gly Asn Phe Gly Asn Ser Tyr Ile Ser Tyr Trp
   355 360 365
   Ala Tyr Trp Gly Gln Gly Thr Leu Val Thr Val Ser Ser Gly Gly Gly
   370 375 380
   Gly Ser Gly Gly Gly Gly Ser Gly Gly Gly Gly Ser Gln Thr Val Val
   385 390 395 400
   Thr Gln Glu Pro Ser Leu Thr Val Ser Pro Gly Gly Thr Val Thr Leu
   405 410 415
   Thr Cys Gly Ser Ser Thr Gly Ala Val Thr Ser Gly Asn Tyr Pro Asn
   420 425 430
   Trp Val Gln Gln Lys Pro Gly Gln Ala Pro Arg Gly Leu Ile Gly Gly
   435 440 445
   Thr Lys Phe Leu Ala Pro Gly Thr Pro Ala Arg Phe Ser Gly Ser Leu
   450 455 460
   Leu Gly Gly Lys Ala Ala Leu Thr Leu Ser Gly Val Gln Pro Glu Asp
   465 470 475 480
   Glu Ala Glu Tyr Tyr Cys Val Leu Trp Tyr Ser Asn Arg Trp Val Phe
   485 490 495
   Gly Gly Gly Thr Lys Leu Thr Val Leu
   500 505
<210> 19
   <211> 993
   <212> PRT
   <213> artificial
<220>
   <223> CD33xCD3 HLE 1
<400> 19
   Gln Val Gln Leu Val Gln Ser Gly Ala Glu Val Lys Lys Pro Gly Glu
   1               5                   10                  15
   Ser Val Lys Val Ser Cys Lys Ala Ser Gly Tyr Thr Phe Thr Asn Tyr
               20                  25                  30
   Gly Met Asn Trp Val Lys Gln Ala Pro Gly Gln Cys Leu Glu Trp Met
           35                  40                  45
   Gly Trp Ile Asn Thr Tyr Thr Gly Glu Pro Thr Tyr Ala Asp Lys Phe
       50                  55                  60
   Gln Gly Arg Val Thr Met Thr Thr Asp Thr Ser Thr Ser Thr Ala Tyr
   65                  70                  75                  80
   Met Glu Ile Arg Asn Leu Gly Gly Asp Asp Thr Ala Val Tyr Tyr Cys
                   85                  90                  95
   Ala Arg Trp Ser Trp Ser Asp Gly Tyr Tyr Val Tyr Phe Asp Tyr Trp
               100                 105                 110
   Gly Gln Gly Thr Ser Val Thr Val Ser Ser Gly Gly Gly Gly Ser Gly
           115                 120                 125
   Gly Gly Gly Ser Gly Gly Gly Gly Ser Asp Ile Val Met Thr Gln Ser
       130                 135                 140
   Pro Asp Ser Leu Thr Val Ser Leu Gly Glu Arg Thr Thr Ile Asn Cys
   145                 150                 155                 160
   Lys Ser Ser Gln Ser Val Leu Asp Ser Ser Thr Asn Lys Asn Ser Leu
                   165                 170                 175
   Ala Trp Tyr Gln Gln Lys Pro Gly Gln Pro Pro Lys Leu Leu Leu Ser
               180                 185                 190
   Trp Ala Ser Thr Arg Glu Ser Gly Ile Pro Asp Arg Phe Ser Gly Ser
           195                 200                 205
   Gly Ser Gly Thr Asp Phe Thr Leu Thr Ile Asp Ser Pro Gln Pro Glu
       210                 215                 220
   Asp Ser Ala Thr Tyr Tyr Cys Gln Gln Ser Ala His Phe Pro Ile Thr
   225                 230                 235                 240
   Phe Gly Cys Gly Thr Arg Leu Glu Ile Lys Ser Gly Gly Gly Gly Ser
                   245                 250                 255
   Glu Val Gln Leu Val Glu Ser Gly Gly Gly Leu Val Gln Pro Gly Gly
               260                 265                 270
   Ser Leu Lys Leu Ser Cys Ala Ala Ser Gly Phe Thr Phe Asn Lys Tyr
           275                 280                 285
   Ala Met Asn Trp Val Arg Gln Ala Pro Gly Lys Gly Leu Glu Trp Val
       290                 295                 300
   Ala Arg Ile Arg Ser Lys Tyr Asn Asn Tyr Ala Thr Tyr Tyr Ala Asp
   305                 310                 315                 320
   Ser Val Lys Asp Arg Phe Thr Ile Ser Arg Asp Asp Ser Lys Asn Thr
                   325                 330                 335
   Ala Tyr Leu Gln Met Asn Asn Leu Lys Thr Glu Asp Thr Ala Val Tyr
               340                 345                 350
   Tyr Cys Val Arg His Gly Asn Phe Gly Asn Ser Tyr Ile Ser Tyr Trp
           355                 360                 365
   Ala Tyr Trp Gly Gln Gly Thr Leu Val Thr Val Ser Ser Gly Gly Gly
       370                 375                 380
   Gly Ser Gly Gly Gly Gly Ser Gly Gly Gly Gly Ser Gln Thr Val Val
   385                 390                 395                 400
   Thr Gln Glu Pro Ser Leu Thr Val Ser Pro Gly Gly Thr Val Thr Leu
                   405                 410                 415
   Thr Cys Gly Ser Ser Thr Gly Ala Val Thr Ser Gly Asn Tyr Pro Asn
               420                 425                 430
   Trp Val Gln Gln Lys Pro Gly Gln Ala Pro Arg Gly Leu Ile Gly Gly
           435                 440                 445
   Thr Lys Phe Leu Ala Pro Gly Thr Pro Ala Arg Phe Ser Gly Ser Leu
       450                 455                 460
   Leu Gly Gly Lys Ala Ala Leu Thr Leu Ser Gly Val Gln Pro Glu Asp
   465                 470                 475                 480
   Glu Ala Glu Tyr Tyr Cys Val Leu Trp Tyr Ser Asn Arg Trp Val Phe
                   485                 490                 495
   Gly Gly Gly Thr Lys Leu Thr Val Leu Gly Gly Gly Gly Asp Lys Thr
               500                 505                 510
   His Thr Cys Pro Pro Cys Pro Ala Pro Glu Leu Leu Gly Gly Pro Ser
           515                 520                 525
   Val Phe Leu Phe Pro Pro Lys Pro Lys Asp Thr Leu Met Ile Ser Arg
       530                 535                 540
   Thr Pro Glu Val Thr Cys Val Val Val Asp Val Ser His Glu Asp Pro
   545                 550                 555                 560
   Glu Val Lys Phe Asn Trp Tyr Val Asp Gly Val Glu Val His Asn Ala
                   565                 570                 575
   Lys Thr Lys Pro Cys Glu Glu Gln Tyr Gly Ser Thr Tyr Arg Cys Val
               580                 585                 590
   Ser Val Leu Thr Val Leu His Gln Asp Trp Leu Asn Gly Lys Glu Tyr
           595                 600                 605
   Lys Cys Lys Val Ser Asn Lys Ala Leu Pro Ala Pro Ile Glu Lys Thr
       610                 615                 620
   Ile Ser Lys Ala Lys Gly Gln Pro Arg Glu Pro Gln Val Tyr Thr Leu
   625                 630                 635                 640
   Pro Pro Ser Arg Glu Glu Met Thr Lys Asn Gln Val Ser Leu Thr Cys
                   645                 650                 655
   Leu Val Lys Gly Phe Tyr Pro Ser Asp Ile Ala Val Glu Trp Glu Ser
               660                 665                 670
   Asn Gly Gln Pro Glu Asn Asn Tyr Lys Thr Thr Pro Pro Val Leu Asp
           675                 680                 685
   Ser Asp Gly Ser Phe Phe Leu Tyr Ser Lys Leu Thr Val Asp Lys Ser
       690                 695                 700
   Arg Trp Gln Gln Gly Asn Val Phe Ser Cys Ser Val Met His Glu Ala
   705                 710                 715                 720
   Leu His Asn His Tyr Thr Gln Lys Ser Leu Ser Leu Ser Pro Gly Lys
                   725                 730                 735
   Gly Gly Gly Gly Ser Gly Gly Gly Gly Ser Gly Gly Gly Gly Ser Gly
               740                 745                 750
   Gly Gly Gly Ser Gly Gly Gly Gly Ser Gly Gly Gly Gly Ser Asp Lys
           755                 760                 765
   Thr His Thr Cys Pro Pro Cys Pro Ala Pro Glu Leu Leu Gly Gly Pro
       770                 775                 780
   Ser Val Phe Leu Phe Pro Pro Lys Pro Lys Asp Thr Leu Met Ile Ser
   785 790 795 800
   Arg Thr Pro Glu Val Thr Cys Val Val Val Asp Val Ser His Glu Asp
   805 810 815
   Pro Glu Val Lys Phe Asn Trp Tyr Val Asp Gly Val Glu Val His Asn
   820 825 830
   Ala Lys Thr Lys Pro Cys Glu Glu Gln Tyr Gly Ser Thr Tyr Arg Cys
   835 840 845
   Val Ser Val Leu Thr Val Leu His Gln Asp Trp Leu Asn Gly Lys Glu
   850 855 860
   Tyr Lys Cys Lys Val Ser Asn Lys Ala Leu Pro Ala Pro Ile Glu Lys
   865 870 875 880
   Thr Ile Ser Lys Ala Lys Gly Gln Pro Arg Glu Pro Gln Val Tyr Thr
   885 890 895
   Leu Pro Pro Ser Arg Glu Glu Met Thr Lys Asn Gln Val Ser Leu Thr
   900 905 910
   Cys Leu Val Lys Gly Phe Tyr Pro Ser Asp Ile Ala Val Glu Trp Glu
   915 920 925
   Ser Asn Gly Gln Pro Glu Asn Asn Tyr Lys Thr Thr Pro Pro Val Leu
   930 935 940
   Asp Ser Asp Gly Ser Phe Phe Leu Tyr Ser Lys Leu Thr Val Asp Lys
   945 950 955 960
   Ser Arg Trp Gln Gln Gly Asn Val Phe Ser Cys Ser Val Met His Glu
   965 970 975
   Ala Leu His Asn His Tyr Thr Gln Lys Ser Leu Ser Leu Ser Pro Gly
   980 985 990
   Lys
<210> 20
   <211> 991
   <212> PRT
   <213> artificial
<220>
   <223> CD33xCD3 HLE 2
<400> 20
   Gln Val Gln Leu Val Gln Ser Gly Ala Glu Val Lys Lys Pro Gly Glu
   1               5                   10                  15
   Ser Val Lys Val Ser Cys Lys Ala Ser Gly Tyr Thr Phe Thr Asn Tyr
               20                  25                  30
   Gly Met Asn Trp Val Lys Gln Ala Pro Gly Gln Cys Leu Glu Trp Met
           35                  40                  45
   Gly Trp Ile Asn Thr Tyr Thr Gly Glu Pro Thr Tyr Ala Asp Lys Phe
       50                  55                  60
   Gln Gly Arg Val Thr Met Thr Thr Asp Thr Ser Thr Ser Thr Ala Tyr
   65                  70                  75                  80
   Met Glu Ile Arg Asn Leu Gly Gly Asp Asp Thr Ala Val Tyr Tyr Cys
                   85                  90                  95
   Ala Arg Trp Ser Trp Ser Asp Gly Tyr Tyr Val Tyr Phe Asp Tyr Trp
               100                 105                 110
   Gly Gln Gly Thr Ser Val Thr Val Ser Ser Gly Gly Gly Gly Ser Gly
           115                 120                 125
   Gly Gly Gly Ser Gly Gly Gly Gly Ser Asp Ile Val Met Thr Gln Ser
       130                 135                 140
   Pro Asp Ser Leu Thr Val Ser Leu Gly Glu Arg Thr Thr Ile Asn Cys
   145                 150                 155                 160
   Lys Ser Ser Gln Ser Val Leu Asp Ser Ser Thr Asn Lys Asn Ser Leu
                   165                 170                 175
   Ala Trp Tyr Gln Gln Lys Pro Gly Gln Pro Pro Lys Leu Leu Leu Ser
               180                 185                 190
   Trp Ala Ser Thr Arg Glu Ser Gly Ile Pro Asp Arg Phe Ser Gly Ser
           195                 200                 205
   Gly Ser Gly Thr Asp Phe Thr Leu Thr Ile Asp Ser Pro Gln Pro Glu
       210                 215                 220
   Asp Ser Ala Thr Tyr Tyr Cys Gln Gln Ser Ala His Phe Pro Ile Thr
   225                 230                 235                 240
   Phe Gly Cys Gly Thr Arg Leu Glu Ile Lys Ser Gly Gly Gly Gly Ser
   245 250 255
   Glu Val Gln Leu Val Glu Ser Gly Gly Gly Leu Val Gln Pro Gly Gly
   260 265 270
   Ser Leu Lys Leu Ser Cys Ala Ala Ser Gly Phe Thr Phe Asn Lys Tyr
   275 280 285
   Ala Met Asn Trp Val Arg Gln Ala Pro Gly Lys Gly Leu Glu Trp Val
   290 295 300
   Ala Arg Ile Arg Ser Lys Tyr Asn Asn Tyr Ala Thr Tyr Tyr Ala Asp
   305 310 315 320
   Ser Val Lys Asp Arg Phe Thr Ile Ser Arg Asp Asp Ser Lys Asn Thr
   325 330 335
   Ala Tyr Leu Gln Met Asn Asn Leu Lys Thr Glu Asp Thr Ala Val Tyr
   340 345 350
   Tyr Cys Val Arg His Gly Asn Phe Gly Asn Ser Tyr Ile Ser Tyr Trp
   355 360 365
   Ala Tyr Trp Gly Gln Gly Thr Leu Val Thr Val Ser Ser Gly Gly Gly
   370 375 380
   Gly Ser Gly Gly Gly Gly Ser Gly Gly Gly Gly Ser Gln Thr Val Val
   385 390 395 400
   Thr Gln Glu Pro Ser Leu Thr Val Ser Pro Gly Gly Thr Val Thr Leu
   405 410 415
   Thr Cys Gly Ser Ser Thr Gly Ala Val Thr Ser Gly Asn Tyr Pro Asn
   420 425 430
   Trp Val Gln Gln Lys Pro Gly Gln Ala Pro Arg Gly Leu Ile Gly Gly
   435 440 445
   Thr Lys Phe Leu Ala Pro Gly Thr Pro Ala Arg Phe Ser Gly Ser Leu
   450 455 460
   Leu Gly Gly Lys Ala Ala Leu Thr Leu Ser Gly Val Gln Pro Glu Asp
   465 470 475 480
   Glu Ala Glu Tyr Tyr Cys Val Leu Trp Tyr Ser Asn Arg Trp Val Phe
   485 490 495
   Gly Gly Gly Thr Lys Leu Thr Val Leu Gly Gly Gly Gly Asp Lys Thr
   500 505 510
   His Thr Cys Pro Pro Cys Pro Ala Pro Glu Leu Leu Gly Gly Pro Ser
   515 520 525
   Val Phe Leu Phe Pro Pro Lys Pro Lys Asp Thr Leu Met Ile Ser Arg
   530 535 540
   Thr Pro Glu Val Thr Cys Val Val Val Asp Val Ser His Glu Asp Pro
   545 550 555 560
   Glu Val Lys Phe Asn Trp Tyr Val Asp Gly Val Glu Val His Asn Ala
   565 570 575
   Lys Thr Lys Pro Cys Glu Glu Gln Tyr Gly Ser Thr Tyr Arg Cys Val
   580 585 590
   Ser Val Leu Thr Val Leu His Gln Asp Trp Leu Asn Gly Lys Glu Tyr
   595 600 605
   Lys Cys Lys Val Ser Asn Lys Ala Leu Pro Ala Pro Ile Glu Lys Thr
   610 615 620
   Ile Ser Lys Ala Lys Gly Gln Pro Arg Glu Pro Gln Val Tyr Thr Leu
   625 630 635 640
   Pro Pro Ser Arg Glu Glu Met Thr Lys Asn Gln Val Ser Leu Thr Cys
   645 650 655
   Leu Val Lys Gly Phe Tyr Pro Ser Asp Ile Ala Val Glu Trp Glu Ser
   660 665 670
   Asn Gly Gln Pro Glu Asn Asn Tyr Lys Thr Thr Pro Pro Val Leu Asp
   675 680 685
   Ser Asp Gly Ser Phe Phe Leu Tyr Ser Lys Leu Thr Val Asp Lys Ser
   690 695 700
   Arg Trp Gln Gln Gly Asn Val Phe Ser Cys Ser Val Met His Glu Ala
   705 710 715 720
   Leu His Asn His Tyr Thr Gln Lys Ser Leu Ser Leu Ser Pro Gly Gly
   725 730 735
   Gly Gly Ser Gly Gly Gly Gly Ser Gly Gly Gly Gly Ser Gly Gly Gly
               740                 745                 750
   Gly Ser Gly Gly Gly Gly Ser Gly Gly Gly Gly Ser Asp Lys Thr His
           755                 760                 765
   Thr Cys Pro Pro Cys Pro Ala Pro Glu Leu Leu Gly Gly Pro Ser Val
       770                 775                 780
   Phe Leu Phe Pro Pro Lys Pro Lys Asp Thr Leu Met Ile Ser Arg Thr
   785                 790                 795                 800
   Pro Glu Val Thr Cys Val Val Val Asp Val Ser His Glu Asp Pro Glu
                   805                 810                 815
   Val Lys Phe Asn Trp Tyr Val Asp Gly Val Glu Val His Asn Ala Lys
               820                 825                 830
   Thr Lys Pro Cys Glu Glu Gln Tyr Gly Ser Thr Tyr Arg Cys Val Ser
           835                 840                 845
   Val Leu Thr Val Leu His Gln Asp Trp Leu Asn Gly Lys Glu Tyr Lys
       850                 855                 860
   Cys Lys Val Ser Asn Lys Ala Leu Pro Ala Pro Ile Glu Lys Thr Ile
   865                 870                 875                 880
   Ser Lys Ala Lys Gly Gln Pro Arg Glu Pro Gln Val Tyr Thr Leu Pro
                   885                 890                 895
   Pro Ser Arg Glu Glu Met Thr Lys Asn Gln Val Ser Leu Thr Cys Leu
               900                 905                 910
   Val Lys Gly Phe Tyr Pro Ser Asp Ile Ala Val Glu Trp Glu Ser Asn
           915                 920                 925
   Gly Gln Pro Glu Asn Asn Tyr Lys Thr Thr Pro Pro Val Leu Asp Ser
       930                 935                 940
   Asp Gly Ser Phe Phe Leu Tyr Ser Lys Leu Thr Val Asp Lys Ser Arg
   945                 950                 955                 960
   Trp Gln Gln Gly Asn Val Phe Ser Cys Ser Val Met His Glu Ala Leu
                   965                 970                 975
   His Asn His Tyr Thr Gln Lys Ser Leu Ser Leu Ser Pro Gly Lys
               980                 985                 990
<210> 21
   <211> 498
   <212> PRT
   <213> artificial
<220>
   <223> CD19xCD3 bispecific antibody construct
<400> 21
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ser Gln Val
           115                 120                 125
   Gln Leu Gln Gln Ser Gly Ala Glu Leu Val Arg Pro Gly Ser Ser Val
       130                 135                 140
   Lys Ile Ser Cys Lys Ala Ser Gly Tyr Ala Phe Ser Ser Tyr Trp Met
   145                 150                 155                 160
   Asn Trp Val Lys Gln Arg Pro Gly Gln Gly Leu Glu Trp Ile Gly Gln
                   165                 170                 175
   Ile Trp Pro Gly Asp Gly Asp Thr Asn Tyr Asn Gly Lys Phe Lys Gly
               180                 185                 190
   Lys Ala Thr Leu Thr Ala Asp Glu Ser Ser Ser Thr Ala Tyr Met Gln
           195                 200                 205
   Leu Ser Ser Leu Ala Ser Glu Asp Ser Ala Val Tyr Phe Cys Ala Arg
       210                 215                 220
   Arg Glu Thr Thr Thr Val Gly Arg Tyr Tyr Tyr Ala Met Asp Tyr Trp
   225                 230                 235                 240
   Gly Gln Gly Thr Thr Val Thr Val Ser Ser Gly Gly Gly Gly Ser Asp
                   245                 250                 255
   Ile Lys Leu Gln Gln Ser Gly Ala Glu Leu Ala Arg Pro Gly Ala Ser
               260                 265                 270
   Val Lys Met Ser Cys Lys Thr Ser Gly Tyr Thr Phe Thr Arg Tyr Thr
           275                 280                 285
   Met His Trp Val Lys Gln Arg Pro Gly Gln Gly Leu Glu Trp Ile Gly
       290                 295                 300
   Tyr Ile Asn Pro Ser Arg Gly Tyr Thr Asn Tyr Asn Gln Lys Phe Lys
   305                 310                 315                 320
   Asp Lys Ala Thr Leu Thr Thr Asp Lys Ser Ser Ser Thr Ala Tyr Met
                   325                 330                 335
   Gln Leu Ser Ser Leu Thr Ser Glu Asp Ser Ala Val Tyr Tyr Cys Ala
               340                 345                 350
   Arg Tyr Tyr Asp Asp His Tyr Cys Leu Asp Tyr Trp Gly Gln Gly Thr
           355                 360                 365
   Thr Leu Thr Val Ser Ser Val Glu Gly Gly Ser Gly Gly Ser Gly Gly
       370                 375                 380
   Ser Gly Gly Ser Gly Gly Val Asp Asp Ile Gln Leu Thr Gln Ser Pro
   385                 390                 395                 400
   Ala Ile Met Ser Ala Ser Pro Gly Glu Lys Val Thr Met Thr Cys Arg
                   405                 410                 415
   Ala Ser Ser Ser Val Ser Tyr Met Asn Trp Tyr Gln Gln Lys Ser Gly
               420                 425                 430
   Thr Ser Pro Lys Arg Trp Ile Tyr Asp Thr Ser Lys Val Ala Ser Gly
           435                 440                 445
   Val Pro Tyr Arg Phe Ser Gly Ser Gly Ser Gly Thr Ser Tyr Ser Leu
       450                 455                 460
   Thr Ile Ser Ser Met Glu Ala Glu Asp Ala Ala Thr Tyr Tyr Cys Gln
   465 470 475 480
   Gln Trp Ser Ser Asn Pro Leu Thr Phe Gly Ala Gly Thr Lys Leu Glu
   485 490 495
   Leu Lys
<210> 22
   <211> 989
   <212> PRT
   <213> artificial
<220>
   <223> FL_16xCD3-scFc
<400> 22
   Gln Val Thr Leu Lys Glu Ser Gly Pro Val Leu Val Lys Pro Thr Glu
   1               5                   10                  15
   Thr Leu Thr Leu Thr Cys Thr Val Ser Gly Phe Ser Leu Arg Asn Ala
               20                  25                  30
   Arg Met Ala Val Ser Trp Ile Arg Gln Pro Pro Gly Lys Thr Leu Glu
           35                  40                  45
   Trp Leu Ala His Ile Phe Ser Asn Asp Glu Lys Ser Tyr Ser Thr Ser
       50                  55                  60
   Leu Lys Ser Arg Leu Thr Ile Ser Lys Asp Thr Ser Lys Ser Gln Val
   65                  70                  75                  80
   Val Leu Thr Met Thr Asn Met Asp Pro Val Asp Thr Ala Thr Tyr Tyr
                   85                  90                  95
   Cys Ala Arg Ile Val Gly Tyr Gly Ser Gly Trp Tyr Gly Tyr Phe Asp
               100                 105                 110
   Tyr Trp Gly Gln Gly Thr Leu Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Val Ser Ala Ser Val Gly Asp Arg Val Thr Ile
   145                 150                 155                 160
   Thr Cys Arg Ala Ser Gln Asp Ile Arg Tyr Asp Leu Ala Trp Tyr Gln
                   165                 170                 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Phe Tyr Pro Leu Thr Phe Gly Gly Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
                   485                 490                 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
           595                 600                 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Lys Gly Gly Gly Gly
                   725                 730                 735
   Ser Gly Gly Gly Gly Ser Gly Gly Gly Gly Ser Gly Gly Gly Gly Ser
               740                 745                 750
   Gly Gly Gly Gly Ser Gly Gly Gly Gly Ser Asp Lys Thr His Thr Cys
           755                 760                 765
   Pro Pro Cys Pro Ala Pro Glu Leu Leu Gly Gly Pro Ser Val Phe Leu
       770                 775                 780
   Phe Pro Pro Lys Pro Lys Asp Thr Leu Met Ile Ser Arg Thr Pro Glu
   785                 790                 795                 800
   Val Thr Cys Val Val Val Asp Val Ser His Glu Asp Pro Glu Val Lys
                   805                 810                 815
   Phe Asn Trp Tyr Val Asp Gly Val Glu Val His Asn Ala Lys Thr Lys
               820                 825                 830
   Pro Cys Glu Glu Gln Tyr Gly Ser Thr Tyr Arg Cys Val Ser Val Leu
           835                 840                 845
   Thr Val Leu His Gln Asp Trp Leu Asn Gly Lys Glu Tyr Lys Cys Lys
       850                 855                 860
   Val Ser Asn Lys Ala Leu Pro Ala Pro Ile Glu Lys Thr Ile Ser Lys
   865                 870                 875                 880
   Ala Lys Gly Gln Pro Arg Glu Pro Gln Val Tyr Thr Leu Pro Pro Ser
                   885                 890                 895
   Arg Glu Glu Met Thr Lys Asn Gln Val Ser Leu Thr Cys Leu Val Lys
               900                 905                 910
   Gly Phe Tyr Pro Ser Asp Ile Ala Val Glu Trp Glu Ser Asn Gly Gln
           915                 920                 925
   Pro Glu Asn Asn Tyr Lys Thr Thr Pro Pro Val Leu Asp Ser Asp Gly
   930 935 940
   Ser Phe Phe Leu Tyr Ser Lys Leu Thr Val Asp Lys Ser Arg Trp Gln
   945 950 955 960
   Gln Gly Asn Val Phe Ser Cys Ser Val Met His Glu Ala Leu His Asn
   965 970 975
   His Tyr Thr Gln Lys Ser Leu Ser Leu Ser Pro Gly Lys
   980 985
<210> 23
   <211> 987
   <212> PRT
   <213> artificial
<220>
   <223> FL_16xCD3-scFc_delGK
<400> 23
   Gln Val Thr Leu Lys Glu Ser Gly Pro Val Leu Val Lys Pro Thr Glu
   1 5 10 15
   Thr Leu Thr Leu Thr Cys Thr Val Ser Gly Phe Ser Leu Arg Asn Ala
   20 25 30
   Arg Met Ala Val Ser Trp Ile Arg Gln Pro Pro Gly Lys Thr Leu Glu
   35 40 45
   Trp Leu Ala His Ile Phe Ser Asn Asp Glu Lys Ser Tyr Ser Thr Ser
   50 55 60
   Leu Lys Ser Arg Leu Thr Ile Ser Lys Asp Thr Ser Lys Ser Gln Val
   65 70 75 80
   Val Leu Thr Met Thr Asn Met Asp Pro Val Asp Thr Ala Thr Tyr Tyr
   85 90 95
   Cys Ala Arg Ile Val Gly Tyr Gly Ser Gly Trp Tyr Gly Tyr Phe Asp
   100 105 110
   Tyr Trp Gly Gln Gly Thr Leu Val Thr Val Ser Ser Gly Gly Gly Gly
   115 120 125
   Ser Gly Gly Gly Gly Ser Gly Gly Gly Gly Ser Asp Ile Gln Met Thr
   130 135 140
   Gln Ser Pro Ser Ser Val Ser Ala Ser Val Gly Asp Arg Val Thr Ile
   145                 150                 155                 160
   Thr Cys Arg Ala Ser Gln Asp Ile Arg Tyr Asp Leu Ala Trp Tyr Gln
                   165                 170                 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Phe Tyr Pro Leu Thr Phe Gly Gly Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
                   485                 490                 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
           595                 600                 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Gly Gly Gly Ser Gly
                   725                 730                 735
   Gly Gly Gly Ser Gly Gly Gly Gly Ser Gly Gly Gly Gly Ser Gly Gly
               740                 745                 750
   Gly Gly Ser Gly Gly Gly Gly Ser Asp Lys Thr His Thr Cys Pro Pro
           755                 760                 765
   Cys Pro Ala Pro Glu Leu Leu Gly Gly Pro Ser Val Phe Leu Phe Pro
       770                 775                 780
   Pro Lys Pro Lys Asp Thr Leu Met Ile Ser Arg Thr Pro Glu Val Thr
   785                 790                 795                 800
   Cys Val Val Val Asp Val Ser His Glu Asp Pro Glu Val Lys Phe Asn
                   805                 810                 815
   Trp Tyr Val Asp Gly Val Glu Val His Asn Ala Lys Thr Lys Pro Cys
               820                 825                 830
   Glu Glu Gln Tyr Gly Ser Thr Tyr Arg Cys Val Ser Val Leu Thr Val
           835                 840                 845
   Leu His Gln Asp Trp Leu Asn Gly Lys Glu Tyr Lys Cys Lys Val Ser
       850                 855                 860
   Asn Lys Ala Leu Pro Ala Pro Ile Glu Lys Thr Ile Ser Lys Ala Lys
   865                 870                 875                 880
   Gly Gln Pro Arg Glu Pro Gln Val Tyr Thr Leu Pro Pro Ser Arg Glu
                   885                 890                 895
   Glu Met Thr Lys Asn Gln Val Ser Leu Thr Cys Leu Val Lys Gly Phe
   900 905 910
   Tyr Pro Ser Asp Ile Ala Val Glu Trp Glu Ser Asn Gly Gln Pro Glu
   915 920 925
   Asn Asn Tyr Lys Thr Thr Pro Pro Val Leu Asp Ser Asp Gly Ser Phe
   930 935 940
   Phe Leu Tyr Ser Lys Leu Thr Val Asp Lys Ser Arg Trp Gln Gln Gly
   945 950 955 960
   Asn Val Phe Ser Cys Ser Val Met His Glu Ala Leu His Asn His Tyr
   965 970 975
   Thr Gln Lys Ser Leu Ser Leu Ser Pro Gly Lys
   980 985
<210> 24
   <211> 989
   <212> PRT
   <213> artificial
<220>
   <223> FL_23xCD3-scFc
<400> 24
   Gln Val Thr Leu Lys Glu Ser Gly Pro Ala Leu Val Lys Pro Thr Glu
   1               5                   10                  15
   Thr Leu Thr Leu Thr Cys Thr Val Ser Gly Phe Ser Phe Arg Asn Ala
               20                  25                  30
   Arg Met Gly Val Ser Trp Ile Arg Gln Pro Pro Gly Lys Ala Leu Glu
           35                  40                  45
   Trp Leu Ala His Ile Phe Ser Asn Asp Glu Lys Ser Tyr Ser Thr Ser
       50                  55                  60
   Leu Lys Ser Arg Leu Thr Ile Ser Lys Asp Thr Ser Lys Ser Gln Val
   65                  70                  75                  80
   Val Leu Thr Leu Thr Asn Met Asp Pro Val Asp Thr Ala Thr Tyr Phe
                   85                  90                  95
   Cys Ala Arg Met Pro Glu Tyr Ser Ser Gly Trp Ser Gly Ala Phe Asp
               100                 105                 110
   Ile Trp Gly Gln Gly Thr Met Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Asp Ile Gly Tyr Asp Leu Gly Trp Tyr Gln
                   165                 170                 175
   Gln Lys Pro Gly Lys Ala Pro Lys Arg Leu Ile Tyr Ala Ala Ser Thr
               180                 185                 190
   Leu Gln Ser Gly Val Pro Ser Arg Phe Ser Gly Ser Gly Ser Gly Thr
           195                 200                 205
   Glu Phe Thr Leu Ile Ile Ser Ser Leu Gln Pro Glu Asp Phe Ala Thr
       210                 215                 220
   Tyr Tyr Cys Leu Gln His Asn Ser Phe Pro Trp Thr Phe Gly Gln Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
                   485                 490                 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
           595                 600                 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Lys Gly Gly Gly Gly
                   725                 730                 735
   Ser Gly Gly Gly Gly Ser Gly Gly Gly Gly Ser Gly Gly Gly Gly Ser
               740                 745                 750
   Gly Gly Gly Gly Ser Gly Gly Gly Gly Ser Asp Lys Thr His Thr Cys
           755                 760                 765
   Pro Pro Cys Pro Ala Pro Glu Leu Leu Gly Gly Pro Ser Val Phe Leu
       770                 775                 780
   Phe Pro Pro Lys Pro Lys Asp Thr Leu Met Ile Ser Arg Thr Pro Glu
   785                 790                 795                 800
   Val Thr Cys Val Val Val Asp Val Ser His Glu Asp Pro Glu Val Lys
                   805                 810                 815
   Phe Asn Trp Tyr Val Asp Gly Val Glu Val His Asn Ala Lys Thr Lys
               820                 825                 830
   Pro Cys Glu Glu Gln Tyr Gly Ser Thr Tyr Arg Cys Val Ser Val Leu
           835                 840                 845
   Thr Val Leu His Gln Asp Trp Leu Asn Gly Lys Glu Tyr Lys Cys Lys
       850                 855                 860
   Val Ser Asn Lys Ala Leu Pro Ala Pro Ile Glu Lys Thr Ile Ser Lys
   865                 870                 875                 880
   Ala Lys Gly Gln Pro Arg Glu Pro Gln Val Tyr Thr Leu Pro Pro Ser
   885 890 895
   Arg Glu Glu Met Thr Lys Asn Gln Val Ser Leu Thr Cys Leu Val Lys
   900 905 910
   Gly Phe Tyr Pro Ser Asp Ile Ala Val Glu Trp Glu Ser Asn Gly Gln
   915 920 925
   Pro Glu Asn Asn Tyr Lys Thr Thr Pro Pro Val Leu Asp Ser Asp Gly
   930 935 940
   Ser Phe Phe Leu Tyr Ser Lys Leu Thr Val Asp Lys Ser Arg Trp Gln
   945 950 955 960
   Gln Gly Asn Val Phe Ser Cys Ser Val Met His Glu Ala Leu His Asn
   965 970 975
   His Tyr Thr Gln Lys Ser Leu Ser Leu Ser Pro Gly Lys
   980 985
<210> 25
   <211> 987
   <212> PRT
   <213> artificial
<220>
   <223> FL_23xCD3-scFc_delGK
<400> 25
   Gln Val Thr Leu Lys Glu Ser Gly Pro Ala Leu Val Lys Pro Thr Glu
   1 5 10 15
   Thr Leu Thr Leu Thr Cys Thr Val Ser Gly Phe Ser Phe Arg Asn Ala
   20 25 30
   Arg Met Gly Val Ser Trp Ile Arg Gln Pro Pro Gly Lys Ala Leu Glu
   35 40 45
   Trp Leu Ala His Ile Phe Ser Asn Asp Glu Lys Ser Tyr Ser Thr Ser
   50 55 60
   Leu Lys Ser Arg Leu Thr Ile Ser Lys Asp Thr Ser Lys Ser Gln Val
   65 70 75 80
   Val Leu Thr Leu Thr Asn Met Asp Pro Val Asp Thr Ala Thr Tyr Phe
   85 90 95
   Cys Ala Arg Met Pro Glu Tyr Ser Ser Gly Trp Ser Gly Ala Phe Asp
   100 105 110
   Ile Trp Gly Gln Gly Thr Met Val Thr Val Ser Ser Gly Gly Gly Gly
   115 120 125
   Ser Gly Gly Gly Gly Ser Gly Gly Gly Gly Ser Asp Ile Gln Met Thr
   130 135 140
   Gln Ser Pro Ser Ser Leu Ser Ala Ser Val Gly Asp Arg Val Thr Ile
   145 150 155 160
   Thr Cys Arg Ala Ser Gln Asp Ile Gly Tyr Asp Leu Gly Trp Tyr Gln
   165 170 175
   Gln Lys Pro Gly Lys Ala Pro Lys Arg Leu Ile Tyr Ala Ala Ser Thr
   180 185 190
   Leu Gln Ser Gly Val Pro Ser Arg Phe Ser Gly Ser Gly Ser Gly Thr
   195 200 205
   Glu Phe Thr Leu Ile Ile Ser Ser Leu Gln Pro Glu Asp Phe Ala Thr
   210 215 220
   Tyr Tyr Cys Leu Gln His Asn Ser Phe Pro Trp Thr Phe Gly Gln Gly
   225 230 235 240
   Thr Lys Val Glu Ile Lys Ser Gly Gly Gly Gly Ser Glu Val Gln Leu
   245 250 255
   Val Glu Ser Gly Gly Gly Leu Val Gln Pro Gly Gly Ser Leu Lys Leu
   260 265 270
   Ser Cys Ala Ala Ser Gly Phe Thr Phe Asn Lys Tyr Ala Met Asn Trp
   275 280 285
   Val Arg Gln Ala Pro Gly Lys Gly Leu Glu Trp Val Ala Arg Ile Arg
   290 295 300
   Ser Lys Tyr Asn Asn Tyr Ala Thr Tyr Tyr Ala Asp Ser Val Lys Asp
   305 310 315 320
   Arg Phe Thr Ile Ser Arg Asp Asp Ser Lys Asn Thr Ala Tyr Leu Gln
   325 330 335
   Met Asn Asn Leu Lys Thr Glu Asp Thr Ala Val Tyr Tyr Cys Val Arg
   340 345 350
   His Gly Asn Phe Gly Asn Ser Tyr Ile Ser Tyr Trp Ala Tyr Trp Gly
               355                 360                 365
   Gln Gly Thr Leu Val Thr Val Ser Ser Gly Gly Gly Gly Ser Gly Gly
           370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
                   485                 490                 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
   595 600 605
   Ser Asn Lys Ala Leu Pro Ala Pro Ile Glu Lys Thr Ile Ser Lys Ala
   610 615 620
   Lys Gly Gln Pro Arg Glu Pro Gln Val Tyr Thr Leu Pro Pro Ser Arg
   625 630 635 640
   Glu Glu Met Thr Lys Asn Gln Val Ser Leu Thr Cys Leu Val Lys Gly
   645 650 655
   Phe Tyr Pro Ser Asp Ile Ala Val Glu Trp Glu Ser Asn Gly Gln Pro
   660 665 670
   Glu Asn Asn Tyr Lys Thr Thr Pro Pro Val Leu Asp Ser Asp Gly Ser
   675 680 685
   Phe Phe Leu Tyr Ser Lys Leu Thr Val Asp Lys Ser Arg Trp Gln Gln
   690 695 700
   Gly Asn Val Phe Ser Cys Ser Val Met His Glu Ala Leu His Asn His
   705 710 715 720
   Tyr Thr Gln Lys Ser Leu Ser Leu Ser Pro Gly Gly Gly Gly Ser Gly
   725 730 735
   Gly Gly Gly Ser Gly Gly Gly Gly Ser Gly Gly Gly Gly Ser Gly Gly
   740 745 750
   Gly Gly Ser Gly Gly Gly Gly Ser Asp Lys Thr His Thr Cys Pro Pro
   755 760 765
   Cys Pro Ala Pro Glu Leu Leu Gly Gly Pro Ser Val Phe Leu Phe Pro
   770 775 780
   Pro Lys Pro Lys Asp Thr Leu Met Ile Ser Arg Thr Pro Glu Val Thr
   785 790 795 800
   Cys Val Val Val Asp Val Ser His Glu Asp Pro Glu Val Lys Phe Asn
   805 810 815
   Trp Tyr Val Asp Gly Val Glu Val His Asn Ala Lys Thr Lys Pro Cys
   820 825 830
   Glu Glu Gln Tyr Gly Ser Thr Tyr Arg Cys Val Ser Val Leu Thr Val
   835 840 845
   Leu His Gln Asp Trp Leu Asn Gly Lys Glu Tyr Lys Cys Lys Val Ser
       850                 855                 860
   Asn Lys Ala Leu Pro Ala Pro Ile Glu Lys Thr Ile Ser Lys Ala Lys
   865                 870                 875                 880
   Gly Gln Pro Arg Glu Pro Gln Val Tyr Thr Leu Pro Pro Ser Arg Glu
                   885                 890                 895
   Glu Met Thr Lys Asn Gln Val Ser Leu Thr Cys Leu Val Lys Gly Phe
               900                 905                 910
   Tyr Pro Ser Asp Ile Ala Val Glu Trp Glu Ser Asn Gly Gln Pro Glu
           915                 920                 925
   Asn Asn Tyr Lys Thr Thr Pro Pro Val Leu Asp Ser Asp Gly Ser Phe
       930                 935                 940
   Phe Leu Tyr Ser Lys Leu Thr Val Asp Lys Ser Arg Trp Gln Gln Gly
   945                 950                 955                 960
   Asn Val Phe Ser Cys Ser Val Met His Glu Ala Leu His Asn His Tyr
                   965                 970                 975
   Thr Gln Lys Ser Leu Ser Leu Ser Pro Gly Lys
               980                 985
<210> 26
   <211> 989
   <212> PRT
   <213> artificial
<220>
   <223> FL_36xCD3-scFc
<400> 26
   Gln Val Thr Leu Lys Glu Ser Gly Pro Thr Leu Val Lys Pro Thr Glu
   1               5                   10                  15
   Thr Leu Thr Leu Thr Cys Thr Phe Ser Gly Phe Ser Leu Arg Tyr Ala
               20                  25                  30
   Arg Met Gly Val Ser Trp Ile Arg Gln Pro Pro Gly Lys Ala Leu Glu
           35                  40                  45
   Trp Leu Ala His Ile Phe Ser Asn Asp Glu Lys Ser Tyr Ser Thr Ser
       50                  55                  60
   Leu Lys Ser Arg Leu Thr Ile Ser Lys Asp Thr Ser Lys Ser Gln Val
   65 70 75 80
   Val Leu Thr Leu Thr Asn Met Asp Pro Val Asp Thr Ala Thr Tyr Phe
   85 90 95
   Cys Ala Arg Met Pro Glu Tyr Ser Ser Gly Trp Ser Gly Ala Phe Asp
   100 105 110
   Ile Trp Gly Gln Gly Thr Met Val Thr Val Ser Ser Gly Gly Gly Gly
   115 120 125
   Ser Gly Gly Gly Gly Ser Gly Gly Gly Gly Ser Asp Ile Gln Met Thr
   130 135 140
   Gln Ser Pro Ser Ser Leu Ser Ala Ser Val Gly Asp Arg Val Thr Ile
   145 150 155 160
   Thr Cys Arg Ala Ser Gln Asp Ile Arg Asn Asp Leu Ala Trp Tyr Gln
   165 170 175
   Gln Lys Pro Gly Lys Ala Pro Lys Arg Leu Ile Tyr Ala Ala Ser Ser
   180 185 190
   Leu Gln Ser Gly Val Pro Ser Arg Phe Ser Gly Ser Gly Ser Gly Thr
   195 200 205
   Glu Phe Thr Leu Thr Ile Ser Ser Leu Gln Pro Glu Asp Phe Ala Thr
   210 215 220
   Tyr Tyr Cys Leu Gln His Asn Ser Tyr Pro Leu Thr Phe Gly Gly Gly
   225 230 235 240
   Thr Lys Leu Glu Ile Lys Ser Gly Gly Gly Gly Ser Glu Val Gln Leu
   245 250 255
   Val Glu Ser Gly Gly Gly Leu Val Gln Pro Gly Gly Ser Leu Lys Leu
   260 265 270
   Ser Cys Ala Ala Ser Gly Phe Thr Phe Asn Lys Tyr Ala Met Asn Trp
   275 280 285
   Val Arg Gln Ala Pro Gly Lys Gly Leu Glu Trp Val Ala Arg Ile Arg
   290 295 300
   Ser Lys Tyr Asn Asn Tyr Ala Thr Tyr Tyr Ala Asp Ser Val Lys Asp
   305 310 315 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
                   485                 490                 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
           595                 600                 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Lys Gly Gly Gly Gly
                   725                 730                 735
   Ser Gly Gly Gly Gly Ser Gly Gly Gly Gly Ser Gly Gly Gly Gly Ser
               740                 745                 750
   Gly Gly Gly Gly Ser Gly Gly Gly Gly Ser Asp Lys Thr His Thr Cys
           755                 760                 765
   Pro Pro Cys Pro Ala Pro Glu Leu Leu Gly Gly Pro Ser Val Phe Leu
       770                 775                 780
   Phe Pro Pro Lys Pro Lys Asp Thr Leu Met Ile Ser Arg Thr Pro Glu
   785                 790                 795                 800
   Val Thr Cys Val Val Val Asp Val Ser His Glu Asp Pro Glu Val Lys
                   805                 810                 815
   Phe Asn Trp Tyr Val Asp Gly Val Glu Val His Asn Ala Lys Thr Lys
               820                 825                 830
   Pro Cys Glu Glu Gln Tyr Gly Ser Thr Tyr Arg Cys Val Ser Val Leu
           835                 840                 845
   Thr Val Leu His Gln Asp Trp Leu Asn Gly Lys Glu Tyr Lys Cys Lys
       850                 855                 860
   Val Ser Asn Lys Ala Leu Pro Ala Pro Ile Glu Lys Thr Ile Ser Lys
   865                 870                 875                 880
   Ala Lys Gly Gln Pro Arg Glu Pro Gln Val Tyr Thr Leu Pro Pro Ser
                   885                 890                 895
   Arg Glu Glu Met Thr Lys Asn Gln Val Ser Leu Thr Cys Leu Val Lys
               900                 905                 910
   Gly Phe Tyr Pro Ser Asp Ile Ala Val Glu Trp Glu Ser Asn Gly Gln
           915                 920                 925
   Pro Glu Asn Asn Tyr Lys Thr Thr Pro Pro Val Leu Asp Ser Asp Gly
       930                 935                 940
   Ser Phe Phe Leu Tyr Ser Lys Leu Thr Val Asp Lys Ser Arg Trp Gln
   945                 950                 955                 960
   Gln Gly Asn Val Phe Ser Cys Ser Val Met His Glu Ala Leu His Asn
                   965                 970                 975
   His Tyr Thr Gln Lys Ser Leu Ser Leu Ser Pro Gly Lys
               980                 985
<210> 27
   <211> 987
   <212> PRT
   <213> artificial
<220>
   <223> FL_36xCD3-scFc_delGK
<400> 27
   Gln Val Thr Leu Lys Glu Ser Gly Pro Thr Leu Val Lys Pro Thr Glu
   1               5                   10                  15
   Thr Leu Thr Leu Thr Cys Thr Phe Ser Gly Phe Ser Leu Arg Tyr Ala
               20                  25                  30
   Arg Met Gly Val Ser Trp Ile Arg Gln Pro Pro Gly Lys Ala Leu Glu
           35                  40                  45
   Trp Leu Ala His Ile Phe Ser Asn Asp Glu Lys Ser Tyr Ser Thr Ser
       50                  55                  60
   Leu Lys Ser Arg Leu Thr Ile Ser Lys Asp Thr Ser Lys Ser Gln Val
   65                  70                  75                  80
   Val Leu Thr Leu Thr Asn Met Asp Pro Val Asp Thr Ala Thr Tyr Phe
                   85                  90                  95
   Cys Ala Arg Met Pro Glu Tyr Ser Ser Gly Trp Ser Gly Ala Phe Asp
               100                 105                 110
   Ile Trp Gly Gln Gly Thr Met Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Asp Ile Arg Asn Asp Leu Ala Trp Tyr Gln
                   165                 170                 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Gly Gly
   225                 230                 235                 240
   Thr Lys Leu Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305 310 315 320
   Arg Phe Thr Ile Ser Arg Asp Asp Ser Lys Asn Thr Ala Tyr Leu Gln
   325 330 335
   Met Asn Asn Leu Lys Thr Glu Asp Thr Ala Val Tyr Tyr Cys Val Arg
   340 345 350
   His Gly Asn Phe Gly Asn Ser Tyr Ile Ser Tyr Trp Ala Tyr Trp Gly
   355 360 365
   Gln Gly Thr Leu Val Thr Val Ser Ser Gly Gly Gly Gly Ser Gly Gly
   370 375 380
   Gly Gly Ser Gly Gly Gly Gly Ser Gln Thr Val Val Thr Gln Glu Pro
   385 390 395 400
   Ser Leu Thr Val Ser Pro Gly Gly Thr Val Thr Leu Thr Cys Gly Ser
   405 410 415
   Ser Thr Gly Ala Val Thr Ser Gly Asn Tyr Pro Asn Trp Val Gln Gln
   420 425 430
   Lys Pro Gly Gln Ala Pro Arg Gly Leu Ile Gly Gly Thr Lys Phe Leu
   435 440 445
   Ala Pro Gly Thr Pro Ala Arg Phe Ser Gly Ser Leu Leu Gly Gly Lys
   450 455 460
   Ala Ala Leu Thr Leu Ser Gly Val Gln Pro Glu Asp Glu Ala Glu Tyr
   465 470 475 480
   Tyr Cys Val Leu Trp Tyr Ser Asn Arg Trp Val Phe Gly Gly Gly Thr
   485 490 495
   Lys Leu Thr Val Leu Gly Gly Gly Gly Asp Lys Thr His Thr Cys Pro
   500 505 510
   Pro Cys Pro Ala Pro Glu Leu Leu Gly Gly Pro Ser Val Phe Leu Phe
   515 520 525
   Pro Pro Lys Pro Lys Asp Thr Leu Met Ile Ser Arg Thr Pro Glu Val
   530 535 540
   Thr Cys Val Val Val Asp Val Ser His Glu Asp Pro Glu Val Lys Phe
   545 550 555 560
   Asn Trp Tyr Val Asp Gly Val Glu Val His Asn Ala Lys Thr Lys Pro
   565 570 575
   Cys Glu Glu Gln Tyr Gly Ser Thr Tyr Arg Cys Val Ser Val Leu Thr
   580 585 590
   Val Leu His Gln Asp Trp Leu Asn Gly Lys Glu Tyr Lys Cys Lys Val
   595 600 605
   Ser Asn Lys Ala Leu Pro Ala Pro Ile Glu Lys Thr Ile Ser Lys Ala
   610 615 620
   Lys Gly Gln Pro Arg Glu Pro Gln Val Tyr Thr Leu Pro Pro Ser Arg
   625 630 635 640
   Glu Glu Met Thr Lys Asn Gln Val Ser Leu Thr Cys Leu Val Lys Gly
   645 650 655
   Phe Tyr Pro Ser Asp Ile Ala Val Glu Trp Glu Ser Asn Gly Gln Pro
   660 665 670
   Glu Asn Asn Tyr Lys Thr Thr Pro Pro Val Leu Asp Ser Asp Gly Ser
   675 680 685
   Phe Phe Leu Tyr Ser Lys Leu Thr Val Asp Lys Ser Arg Trp Gln Gln
   690 695 700
   Gly Asn Val Phe Ser Cys Ser Val Met His Glu Ala Leu His Asn His
   705 710 715 720
   Tyr Thr Gln Lys Ser Leu Ser Leu Ser Pro Gly Gly Gly Gly Ser Gly
   725 730 735
   Gly Gly Gly Ser Gly Gly Gly Gly Ser Gly Gly Gly Gly Ser Gly Gly
   740 745 750
   Gly Gly Ser Gly Gly Gly Gly Ser Asp Lys Thr His Thr Cys Pro Pro
   755 760 765
   Cys Pro Ala Pro Glu Leu Leu Gly Gly Pro Ser Val Phe Leu Phe Pro
   770 775 780
   Pro Lys Pro Lys Asp Thr Leu Met Ile Ser Arg Thr Pro Glu Val Thr
   785 790 795 800
   Cys Val Val Val Asp Val Ser His Glu Asp Pro Glu Val Lys Phe Asn
   805 810 815
   Trp Tyr Val Asp Gly Val Glu Val His Asn Ala Lys Thr Lys Pro Cys
   820 825 830
   Glu Glu Gln Tyr Gly Ser Thr Tyr Arg Cys Val Ser Val Leu Thr Val
   835 840 845
   Leu His Gln Asp Trp Leu Asn Gly Lys Glu Tyr Lys Cys Lys Val Ser
   850 855 860
   Asn Lys Ala Leu Pro Ala Pro Ile Glu Lys Thr Ile Ser Lys Ala Lys
   865 870 875 880
   Gly Gln Pro Arg Glu Pro Gln Val Tyr Thr Leu Pro Pro Ser Arg Glu
   885 890 895
   Glu Met Thr Lys Asn Gln Val Ser Leu Thr Cys Leu Val Lys Gly Phe
   900 905 910
   Tyr Pro Ser Asp Ile Ala Val Glu Trp Glu Ser Asn Gly Gln Pro Glu
   915 920 925
   Asn Asn Tyr Lys Thr Thr Pro Pro Val Leu Asp Ser Asp Gly Ser Phe
   930 935 940
   Phe Leu Tyr Ser Lys Leu Thr Val Asp Lys Ser Arg Trp Gln Gln Gly
   945 950 955 960
   Asn Val Phe Ser Cys Ser Val Met His Glu Ala Leu His Asn His Tyr
   965 970 975
   Thr Gln Lys Ser Leu Ser Leu Ser Pro Gly Lys
   980 985
<210> 28
   <211> 989
   <212> PRT
   <213> artificial
<220>
   <223> FL_39_xCD3-scFc
<400> 28
   Gln Val Thr Leu Lys Glu Ser Gly Pro Thr Leu Val Lys Pro Thr Glu
   1               5                   10                  15
   Thr Leu Thr Leu Thr Cys Thr Leu Ser Gly Phe Ser Leu Asn Asn Ala
               20                  25                  30
   Arg Met Gly Val Ser Trp Ile Arg Gln Pro Pro Gly Lys Cys Leu Glu
           35                  40                  45
   Trp Leu Ala His Ile Phe Ser Asn Asp Glu Lys Ser Tyr Ser Thr Ser
       50                  55                  60
   Leu Lys Asn Arg Leu Thr Ile Ser Lys Asp Ser Ser Lys Thr Gln Val
   65                  70                  75                  80
   Val Leu Thr Met Thr Asn Val Asp Pro Val Asp Thr Ala Thr Tyr Tyr
                   85                  90                  95
   Cys Ala Arg Ile Val Gly Tyr Gly Ser Gly Trp Tyr Gly Phe Phe Asp
               100                 105                 110
   Tyr Trp Gly Gln Gly Thr Leu Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Gly Ile Arg Asn Asp Leu Gly Trp Tyr Gln
                   165                 170                 175
   Gln Lys Pro Gly Lys Ala Pro Lys Arg Leu Ile Tyr Ala Ala Ser Thr
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Cys Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
                   485                 490                 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
           595                 600                 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Lys Gly Gly Gly Gly
                   725                 730                 735
   Ser Gly Gly Gly Gly Ser Gly Gly Gly Gly Ser Gly Gly Gly Gly Ser
               740                 745                 750
   Gly Gly Gly Gly Ser Gly Gly Gly Gly Ser Asp Lys Thr His Thr Cys
           755                 760                 765
   Pro Pro Cys Pro Ala Pro Glu Leu Leu Gly Gly Pro Ser Val Phe Leu
       770                 775                 780
   Phe Pro Pro Lys Pro Lys Asp Thr Leu Met Ile Ser Arg Thr Pro Glu
   785                 790                 795                 800
   Val Thr Cys Val Val Val Asp Val Ser His Glu Asp Pro Glu Val Lys
                   805                 810                 815
   Phe Asn Trp Tyr Val Asp Gly Val Glu Val His Asn Ala Lys Thr Lys
               820                 825                 830
   Pro Cys Glu Glu Gln Tyr Gly Ser Thr Tyr Arg Cys Val Ser Val Leu
           835                 840                 845
   Thr Val Leu His Gln Asp Trp Leu Asn Gly Lys Glu Tyr Lys Cys Lys
       850                 855                 860
   Val Ser Asn Lys Ala Leu Pro Ala Pro Ile Glu Lys Thr Ile Ser Lys
   865                 870                 875                 880
   Ala Lys Gly Gln Pro Arg Glu Pro Gln Val Tyr Thr Leu Pro Pro Ser
                   885                 890                 895
   Arg Glu Glu Met Thr Lys Asn Gln Val Ser Leu Thr Cys Leu Val Lys
               900                 905                 910
   Gly Phe Tyr Pro Ser Asp Ile Ala Val Glu Trp Glu Ser Asn Gly Gln
           915                 920                 925
   Pro Glu Asn Asn Tyr Lys Thr Thr Pro Pro Val Leu Asp Ser Asp Gly
       930                 935                 940
   Ser Phe Phe Leu Tyr Ser Lys Leu Thr Val Asp Lys Ser Arg Trp Gln
   945                 950                 955                 960
   Gln Gly Asn Val Phe Ser Cys Ser Val Met His Glu Ala Leu His Asn
                   965                 970                 975
   His Tyr Thr Gln Lys Ser Leu Ser Leu Ser Pro Gly Lys
               980                 985
<210> 29
   <211> 987
   <212> PRT
   <213> artificial
<220>
   <223> FL_39_xCD3-scFc_delGK
<400> 29
   Gln Val Thr Leu Lys Glu Ser Gly Pro Thr Leu Val Lys Pro Thr Glu
   1               5                   10                  15
   Thr Leu Thr Leu Thr Cys Thr Leu Ser Gly Phe Ser Leu Asn Asn Ala
               20                  25                  30
   Arg Met Gly Val Ser Trp Ile Arg Gln Pro Pro Gly Lys Cys Leu Glu
           35                  40                  45
   Trp Leu Ala His Ile Phe Ser Asn Asp Glu Lys Ser Tyr Ser Thr Ser
       50                  55                  60
   Leu Lys Asn Arg Leu Thr Ile Ser Lys Asp Ser Ser Lys Thr Gln Val
   65                  70                  75                  80
   Val Leu Thr Met Thr Asn Val Asp Pro Val Asp Thr Ala Thr Tyr Tyr
                   85                  90                  95
   Cys Ala Arg Ile Val Gly Tyr Gly Ser Gly Trp Tyr Gly Phe Phe Asp
               100                 105                 110
   Tyr Trp Gly Gln Gly Thr Leu Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Gly Ile Arg Asn Asp Leu Gly Trp Tyr Gln
                   165                 170                 175
   Gln Lys Pro Gly Lys Ala Pro Lys Arg Leu Ile Tyr Ala Ala Ser Thr
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Cys Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
   260 265 270
   Ser Cys Ala Ala Ser Gly Phe Thr Phe Asn Lys Tyr Ala Met Asn Trp
   275 280 285
   Val Arg Gln Ala Pro Gly Lys Gly Leu Glu Trp Val Ala Arg Ile Arg
   290 295 300
   Ser Lys Tyr Asn Asn Tyr Ala Thr Tyr Tyr Ala Asp Ser Val Lys Asp
   305 310 315 320
   Arg Phe Thr Ile Ser Arg Asp Asp Ser Lys Asn Thr Ala Tyr Leu Gln
   325 330 335
   Met Asn Asn Leu Lys Thr Glu Asp Thr Ala Val Tyr Tyr Cys Val Arg
   340 345 350
   His Gly Asn Phe Gly Asn Ser Tyr Ile Ser Tyr Trp Ala Tyr Trp Gly
   355 360 365
   Gln Gly Thr Leu Val Thr Val Ser Ser Gly Gly Gly Gly Ser Gly Gly
   370 375 380
   Gly Gly Ser Gly Gly Gly Gly Ser Gln Thr Val Val Thr Gln Glu Pro
   385 390 395 400
   Ser Leu Thr Val Ser Pro Gly Gly Thr Val Thr Leu Thr Cys Gly Ser
   405 410 415
   Ser Thr Gly Ala Val Thr Ser Gly Asn Tyr Pro Asn Trp Val Gln Gln
   420 425 430
   Lys Pro Gly Gln Ala Pro Arg Gly Leu Ile Gly Gly Thr Lys Phe Leu
   435 440 445
   Ala Pro Gly Thr Pro Ala Arg Phe Ser Gly Ser Leu Leu Gly Gly Lys
   450 455 460
   Ala Ala Leu Thr Leu Ser Gly Val Gln Pro Glu Asp Glu Ala Glu Tyr
   465 470 475 480
   Tyr Cys Val Leu Trp Tyr Ser Asn Arg Trp Val Phe Gly Gly Gly Thr
   485 490 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
           595                 600                 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Gly Gly Gly Ser Gly
                   725                 730                 735
   Gly Gly Gly Ser Gly Gly Gly Gly Ser Gly Gly Gly Gly Ser Gly Gly
               740                 745                 750
   Gly Gly Ser Gly Gly Gly Gly Ser Asp Lys Thr His Thr Cys Pro Pro
           755                 760                 765
   Cys Pro Ala Pro Glu Leu Leu Gly Gly Pro Ser Val Phe Leu Phe Pro
       770                 775                 780
   Pro Lys Pro Lys Asp Thr Leu Met Ile Ser Arg Thr Pro Glu Val Thr
   785                 790                 795                 800
   Cys Val Val Val Asp Val Ser His Glu Asp Pro Glu Val Lys Phe Asn
                   805                 810                 815
   Trp Tyr Val Asp Gly Val Glu Val His Asn Ala Lys Thr Lys Pro Cys
               820                 825                 830
   Glu Glu Gln Tyr Gly Ser Thr Tyr Arg Cys Val Ser Val Leu Thr Val
           835                 840                 845
   Leu His Gln Asp Trp Leu Asn Gly Lys Glu Tyr Lys Cys Lys Val Ser
       850                 855                 860
   Asn Lys Ala Leu Pro Ala Pro Ile Glu Lys Thr Ile Ser Lys Ala Lys
   865                 870                 875                 880
   Gly Gln Pro Arg Glu Pro Gln Val Tyr Thr Leu Pro Pro Ser Arg Glu
                   885                 890                 895
   Glu Met Thr Lys Asn Gln Val Ser Leu Thr Cys Leu Val Lys Gly Phe
               900                 905                 910
   Tyr Pro Ser Asp Ile Ala Val Glu Trp Glu Ser Asn Gly Gln Pro Glu
           915                 920                 925
   Asn Asn Tyr Lys Thr Thr Pro Pro Val Leu Asp Ser Asp Gly Ser Phe
       930                 935                 940
   Phe Leu Tyr Ser Lys Leu Thr Val Asp Lys Ser Arg Trp Gln Gln Gly
   945                 950                 955                 960
   Asn Val Phe Ser Cys Ser Val Met His Glu Ala Leu His Asn His Tyr
                   965                 970                 975
   Thr Gln Lys Ser Leu Ser Leu Ser Pro Gly Lys
               980                 985
<210> 30
   <211> 989
   <212> PRT
   <213> artificial
<220>
   <223> FL_42_xCD3-scFc
<400> 30
   Gln Val Thr Leu Lys Glu Ser Gly Pro Ala Leu Val Lys Pro Thr Glu
   1               5                   10                  15
   Thr Leu Thr Leu Thr Cys Thr Leu Ser Gly Phe Ser Leu Asn Asn Ala
               20                  25                  30
   Arg Met Ala Val Ser Trp Ile Arg Gln Pro Pro Gly Lys Cys Leu Glu
           35                  40                  45
   Trp Leu Ala His Ile Phe Ser Asn Asp Glu Lys Ser Tyr Ser Thr Ser
       50                  55                  60
   Leu Lys Ser Arg Leu Thr Ile Ser Lys Asp Thr Ser Lys Ser Gln Val
   65                  70                  75                  80
   Val Leu Thr Met Thr Asn Met Asp Pro Glu Asp Thr Ala Thr Tyr Tyr
                   85                  90                  95
   Cys Ala Arg Ile Val Gly Tyr Gly Thr Gly Trp Tyr Gly Phe Phe Asp
               100                 105                 110
   Tyr Trp Gly Gln Gly Ile Leu Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Gly Ile Arg Asn Asp Leu Ala Trp Tyr Gln
                   165                 170                 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Cys Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
   485 490 495
   Lys Leu Thr Val Leu Gly Gly Gly Gly Asp Lys Thr His Thr Cys Pro
   500 505 510
   Pro Cys Pro Ala Pro Glu Leu Leu Gly Gly Pro Ser Val Phe Leu Phe
   515 520 525
   Pro Pro Lys Pro Lys Asp Thr Leu Met Ile Ser Arg Thr Pro Glu Val
   530 535 540
   Thr Cys Val Val Val Asp Val Ser His Glu Asp Pro Glu Val Lys Phe
   545 550 555 560
   Asn Trp Tyr Val Asp Gly Val Glu Val His Asn Ala Lys Thr Lys Pro
   565 570 575
   Cys Glu Glu Gln Tyr Gly Ser Thr Tyr Arg Cys Val Ser Val Leu Thr
   580 585 590
   Val Leu His Gln Asp Trp Leu Asn Gly Lys Glu Tyr Lys Cys Lys Val
   595 600 605
   Ser Asn Lys Ala Leu Pro Ala Pro Ile Glu Lys Thr Ile Ser Lys Ala
   610 615 620
   Lys Gly Gln Pro Arg Glu Pro Gln Val Tyr Thr Leu Pro Pro Ser Arg
   625 630 635 640
   Glu Glu Met Thr Lys Asn Gln Val Ser Leu Thr Cys Leu Val Lys Gly
   645 650 655
   Phe Tyr Pro Ser Asp Ile Ala Val Glu Trp Glu Ser Asn Gly Gln Pro
   660 665 670
   Glu Asn Asn Tyr Lys Thr Thr Pro Pro Val Leu Asp Ser Asp Gly Ser
   675 680 685
   Phe Phe Leu Tyr Ser Lys Leu Thr Val Asp Lys Ser Arg Trp Gln Gln
   690 695 700
   Gly Asn Val Phe Ser Cys Ser Val Met His Glu Ala Leu His Asn His
   705 710 715 720
   Tyr Thr Gln Lys Ser Leu Ser Leu Ser Pro Gly Lys Gly Gly Gly Gly
   725 730 735
   Ser Gly Gly Gly Gly Ser Gly Gly Gly Gly Ser Gly Gly Gly Gly Ser
   740 745 750
   Gly Gly Gly Gly Ser Gly Gly Gly Gly Ser Asp Lys Thr His Thr Cys
   755 760 765
   Pro Pro Cys Pro Ala Pro Glu Leu Leu Gly Gly Pro Ser Val Phe Leu
   770 775 780
   Phe Pro Pro Lys Pro Lys Asp Thr Leu Met Ile Ser Arg Thr Pro Glu
   785 790 795 800
   Val Thr Cys Val Val Val Asp Val Ser His Glu Asp Pro Glu Val Lys
   805 810 815
   Phe Asn Trp Tyr Val Asp Gly Val Glu Val His Asn Ala Lys Thr Lys
   820 825 830
   Pro Cys Glu Glu Gln Tyr Gly Ser Thr Tyr Arg Cys Val Ser Val Leu
   835 840 845
   Thr Val Leu His Gln Asp Trp Leu Asn Gly Lys Glu Tyr Lys Cys Lys
   850 855 860
   Val Ser Asn Lys Ala Leu Pro Ala Pro Ile Glu Lys Thr Ile Ser Lys
   865 870 875 880
   Ala Lys Gly Gln Pro Arg Glu Pro Gln Val Tyr Thr Leu Pro Pro Ser
   885 890 895
   Arg Glu Glu Met Thr Lys Asn Gln Val Ser Leu Thr Cys Leu Val Lys
   900 905 910
   Gly Phe Tyr Pro Ser Asp Ile Ala Val Glu Trp Glu Ser Asn Gly Gln
   915 920 925
   Pro Glu Asn Asn Tyr Lys Thr Thr Pro Pro Val Leu Asp Ser Asp Gly
   930 935 940
   Ser Phe Phe Leu Tyr Ser Lys Leu Thr Val Asp Lys Ser Arg Trp Gln
   945 950 955 960
   Gln Gly Asn Val Phe Ser Cys Ser Val Met His Glu Ala Leu His Asn
   965 970 975
   His Tyr Thr Gln Lys Ser Leu Ser Leu Ser Pro Gly Lys
   980 985
<210> 31
   <211> 987
   <212> PRT
   <213> artificial
<220>
   <223> FL_42_xCD3-scFc_delGK
<400> 31
   Gln Val Thr Leu Lys Glu Ser Gly Pro Ala Leu Val Lys Pro Thr Glu
   1               5                   10                  15
   Thr Leu Thr Leu Thr Cys Thr Leu Ser Gly Phe Ser Leu Asn Asn Ala
               20                  25                  30
   Arg Met Ala Val Ser Trp Ile Arg Gln Pro Pro Gly Lys Cys Leu Glu
           35                  40                  45
   Trp Leu Ala His Ile Phe Ser Asn Asp Glu Lys Ser Tyr Ser Thr Ser
       50                  55                  60
   Leu Lys Ser Arg Leu Thr Ile Ser Lys Asp Thr Ser Lys Ser Gln Val
   65                  70                  75                  80
   Val Leu Thr Met Thr Asn Met Asp Pro Glu Asp Thr Ala Thr Tyr Tyr
                   85                  90                  95
   Cys Ala Arg Ile Val Gly Tyr Gly Thr Gly Trp Tyr Gly Phe Phe Asp
               100                 105                 110
   Tyr Trp Gly Gln Gly Ile Leu Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Gly Ile Arg Asn Asp Leu Ala Trp Tyr Gln
                   165                 170                 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Cys Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
   450 455 460
   Ala Ala Leu Thr Leu Ser Gly Val Gln Pro Glu Asp Glu Ala Glu Tyr
   465 470 475 480
   Tyr Cys Val Leu Trp Tyr Ser Asn Arg Trp Val Phe Gly Gly Gly Thr
   485 490 495
   Lys Leu Thr Val Leu Gly Gly Gly Gly Asp Lys Thr His Thr Cys Pro
   500 505 510
   Pro Cys Pro Ala Pro Glu Leu Leu Gly Gly Pro Ser Val Phe Leu Phe
   515 520 525
   Pro Pro Lys Pro Lys Asp Thr Leu Met Ile Ser Arg Thr Pro Glu Val
   530 535 540
   Thr Cys Val Val Val Asp Val Ser His Glu Asp Pro Glu Val Lys Phe
   545 550 555 560
   Asn Trp Tyr Val Asp Gly Val Glu Val His Asn Ala Lys Thr Lys Pro
   565 570 575
   Cys Glu Glu Gln Tyr Gly Ser Thr Tyr Arg Cys Val Ser Val Leu Thr
   580 585 590
   Val Leu His Gln Asp Trp Leu Asn Gly Lys Glu Tyr Lys Cys Lys Val
   595 600 605
   Ser Asn Lys Ala Leu Pro Ala Pro Ile Glu Lys Thr Ile Ser Lys Ala
   610 615 620
   Lys Gly Gln Pro Arg Glu Pro Gln Val Tyr Thr Leu Pro Pro Ser Arg
   625 630 635 640
   Glu Glu Met Thr Lys Asn Gln Val Ser Leu Thr Cys Leu Val Lys Gly
   645 650 655
   Phe Tyr Pro Ser Asp Ile Ala Val Glu Trp Glu Ser Asn Gly Gln Pro
   660 665 670
   Glu Asn Asn Tyr Lys Thr Thr Pro Pro Val Leu Asp Ser Asp Gly Ser
   675 680 685
   Phe Phe Leu Tyr Ser Lys Leu Thr Val Asp Lys Ser Arg Trp Gln Gln
   690 695 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Gly Gly Gly Ser Gly
                   725                 730                 735
   Gly Gly Gly Ser Gly Gly Gly Gly Ser Gly Gly Gly Gly Ser Gly Gly
               740                 745                 750
   Gly Gly Ser Gly Gly Gly Gly Ser Asp Lys Thr His Thr Cys Pro Pro
           755                 760                 765
   Cys Pro Ala Pro Glu Leu Leu Gly Gly Pro Ser Val Phe Leu Phe Pro
       770                 775                 780
   Pro Lys Pro Lys Asp Thr Leu Met Ile Ser Arg Thr Pro Glu Val Thr
   785                 790                 795                 800
   Cys Val Val Val Asp Val Ser His Glu Asp Pro Glu Val Lys Phe Asn
                   805                 810                 815
   Trp Tyr Val Asp Gly Val Glu Val His Asn Ala Lys Thr Lys Pro Cys
               820                 825                 830
   Glu Glu Gln Tyr Gly Ser Thr Tyr Arg Cys Val Ser Val Leu Thr Val
           835                 840                 845
   Leu His Gln Asp Trp Leu Asn Gly Lys Glu Tyr Lys Cys Lys Val Ser
       850                 855                 860
   Asn Lys Ala Leu Pro Ala Pro Ile Glu Lys Thr Ile Ser Lys Ala Lys
   865                 870                 875                 880
   Gly Gln Pro Arg Glu Pro Gln Val Tyr Thr Leu Pro Pro Ser Arg Glu
                   885                 890                 895
   Glu Met Thr Lys Asn Gln Val Ser Leu Thr Cys Leu Val Lys Gly Phe
               900                 905                 910
   Tyr Pro Ser Asp Ile Ala Val Glu Trp Glu Ser Asn Gly Gln Pro Glu
           915                 920                 925
   Asn Asn Tyr Lys Thr Thr Pro Pro Val Leu Asp Ser Asp Gly Ser Phe
       930                 935                 940
   Phe Leu Tyr Ser Lys Leu Thr Val Asp Lys Ser Arg Trp Gln Gln Gly
   945                 950                 955                 960
   Asn Val Phe Ser Cys Ser Val Met His Glu Ala Leu His Asn His Tyr
   965 970 975
   Thr Gln Lys Ser Leu Ser Leu Ser Pro Gly Lys
   980 985
<210> 32
   <211> 989
   <212> PRT
   <213> artificial
<220>
   <223> FL_46_CCxCD3-scFc
<400> 32
   Gln Val Thr Leu Lys Glu Ser Gly Pro Val Leu Val Lys Pro Thr Gln
   1 5 10 15
   Thr Leu Thr Leu Thr Cys Thr Phe Ser Gly Phe Ser Leu Ser Asn Ala
   20 25 30
   Arg Met Gly Val Ser Trp Ile Arg Gln Pro Pro Gly Lys Cys Leu Glu
   35 40 45
   Trp Leu Ala Leu Ile Tyr Trp Asn Asp Asp Lys Arg Tyr Ser Pro Ser
   50 55 60
   Leu Lys Ser Arg Leu Thr Ile Thr Lys Asp Thr Ser Lys Asn Gln Val
   65 70 75 80
   Val Leu Thr Met Thr Asn Met Asp Pro Val Asp Thr Ala Thr Tyr Tyr
   85 90 95
   Cys Ala Arg Met Val Gly Tyr Gly Ser Gly Trp Tyr Ala Tyr Phe Asp
   100 105 110
   Tyr Trp Gly Gln Gly Thr Leu Val Thr Val Ser Ser Gly Gly Gly Gly
   115 120 125
   Ser Gly Gly Gly Gly Ser Gly Gly Gly Gly Ser Asp Ile Gln Met Thr
   130 135 140
   Gln Ser Pro Ser Ser Leu Ser Ala Ser Val Gly Asp Arg Val Thr Ile
   145 150 155 160
   Thr Cys Arg Ala Ser Gln Gly Ile Arg Asn Asp Leu Gly Trp Tyr Gln
   165 170 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Cys Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
                   405                 410                 415
   Ser Thr Gly Ala Val Thr Ser Gly Asn Tyr Pro Asn Trp Val Gln Gln
               420                 425                 430
   Lys Pro Gly Gln Ala Pro Arg Gly Leu Ile Gly Gly Thr Lys Phe Leu
           435                 440                 445
   Ala Pro Gly Thr Pro Ala Arg Phe Ser Gly Ser Leu Leu Gly Gly Lys
       450                 455                 460
   Ala Ala Leu Thr Leu Ser Gly Val Gln Pro Glu Asp Glu Ala Glu Tyr
   465                 470                 475                 480
   Tyr Cys Val Leu Trp Tyr Ser Asn Arg Trp Val Phe Gly Gly Gly Thr
                   485                 490                 495
   Lys Leu Thr Val Leu Gly Gly Gly Gly Asp Lys Thr His Thr Cys Pro
               500                 505                 510
   Pro Cys Pro Ala Pro Glu Leu Leu Gly Gly Pro Ser Val Phe Leu Phe
           515                 520                 525
   Pro Pro Lys Pro Lys Asp Thr Leu Met Ile Ser Arg Thr Pro Glu Val
       530                 535                 540
   Thr Cys Val Val Val Asp Val Ser His Glu Asp Pro Glu Val Lys Phe
   545                 550                 555                 560
   Asn Trp Tyr Val Asp Gly Val Glu Val His Asn Ala Lys Thr Lys Pro
                   565                 570                 575
   Cys Glu Glu Gln Tyr Gly Ser Thr Tyr Arg Cys Val Ser Val Leu Thr
               580                 585                 590
   Val Leu His Gln Asp Trp Leu Asn Gly Lys Glu Tyr Lys Cys Lys Val
           595                 600                 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Lys Gly Gly Gly Gly
                   725                 730                 735
   Ser Gly Gly Gly Gly Ser Gly Gly Gly Gly Ser Gly Gly Gly Gly Ser
               740                 745                 750
   Gly Gly Gly Gly Ser Gly Gly Gly Gly Ser Asp Lys Thr His Thr Cys
           755                 760                 765
   Pro Pro Cys Pro Ala Pro Glu Leu Leu Gly Gly Pro Ser Val Phe Leu
       770                 775                 780
   Phe Pro Pro Lys Pro Lys Asp Thr Leu Met Ile Ser Arg Thr Pro Glu
   785                 790                 795                 800
   Val Thr Cys Val Val Val Asp Val Ser His Glu Asp Pro Glu Val Lys
                   805                 810                 815
   Phe Asn Trp Tyr Val Asp Gly Val Glu Val His Asn Ala Lys Thr Lys
               820                 825                 830
   Pro Cys Glu Glu Gln Tyr Gly Ser Thr Tyr Arg Cys Val Ser Val Leu
           835                 840                 845
   Thr Val Leu His Gln Asp Trp Leu Asn Gly Lys Glu Tyr Lys Cys Lys
       850                 855                 860
   Val Ser Asn Lys Ala Leu Pro Ala Pro Ile Glu Lys Thr Ile Ser Lys
   865                 870                 875                 880
   Ala Lys Gly Gln Pro Arg Glu Pro Gln Val Tyr Thr Leu Pro Pro Ser
                   885                 890                 895
   Arg Glu Glu Met Thr Lys Asn Gln Val Ser Leu Thr Cys Leu Val Lys
               900                 905                 910
   Gly Phe Tyr Pro Ser Asp Ile Ala Val Glu Trp Glu Ser Asn Gly Gln
           915                 920                 925
   Pro Glu Asn Asn Tyr Lys Thr Thr Pro Pro Val Leu Asp Ser Asp Gly
   930 935 940
   Ser Phe Phe Leu Tyr Ser Lys Leu Thr Val Asp Lys Ser Arg Trp Gln
   945 950 955 960
   Gln Gly Asn Val Phe Ser Cys Ser Val Met His Glu Ala Leu His Asn
   965 970 975
   His Tyr Thr Gln Lys Ser Leu Ser Leu Ser Pro Gly Lys
   980 985
<210> 33
   <211> 987
   <212> PRT
   <213> artificial
<220>
   <223> FL_46_CCxCD3-scFc_delGK
<400> 33
   Gln Val Thr Leu Lys Glu Ser Gly Pro Val Leu Val Lys Pro Thr Gln
   1               5                   10                  15
   Thr Leu Thr Leu Thr Cys Thr Phe Ser Gly Phe Ser Leu Ser Asn Ala
               20                  25                  30
   Arg Met Gly Val Ser Trp Ile Arg Gln Pro Pro Gly Lys Cys Leu Glu
           35                  40                  45
   Trp Leu Ala Leu Ile Tyr Trp Asn Asp Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Met Val Gly Tyr Gly Ser Gly Trp Tyr Ala Tyr Phe Asp
               100                 105                 110
   Tyr Trp Gly Gln Gly Thr Leu Val Thr Val Ser Ser Gly Gly Gly Gly
           115                 120                 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Gly Ile Arg Asn Asp Leu Gly Trp Tyr Gln
                   165                 170                 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Cys Gly
   225                 230                 235                 240
   Thr Lys Val Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385                 390                 395                 400
   Ser Leu Thr Val Ser Pro Gly Gly Thr Val Thr Leu Thr Cys Gly Ser
   405 410 415
   Ser Thr Gly Ala Val Thr Ser Gly Asn Tyr Pro Asn Trp Val Gln Gln
   420 425 430
   Lys Pro Gly Gln Ala Pro Arg Gly Leu Ile Gly Gly Thr Lys Phe Leu
   435 440 445
   Ala Pro Gly Thr Pro Ala Arg Phe Ser Gly Ser Leu Leu Gly Gly Lys
   450 455 460
   Ala Ala Leu Thr Leu Ser Gly Val Gln Pro Glu Asp Glu Ala Glu Tyr
   465 470 475 480
   Tyr Cys Val Leu Trp Tyr Ser Asn Arg Trp Val Phe Gly Gly Gly Thr
   485 490 495
   Lys Leu Thr Val Leu Gly Gly Gly Gly Asp Lys Thr His Thr Cys Pro
   500 505 510
   Pro Cys Pro Ala Pro Glu Leu Leu Gly Gly Pro Ser Val Phe Leu Phe
   515 520 525
   Pro Pro Lys Pro Lys Asp Thr Leu Met Ile Ser Arg Thr Pro Glu Val
   530 535 540
   Thr Cys Val Val Val Asp Val Ser His Glu Asp Pro Glu Val Lys Phe
   545 550 555 560
   Asn Trp Tyr Val Asp Gly Val Glu Val His Asn Ala Lys Thr Lys Pro
   565 570 575
   Cys Glu Glu Gln Tyr Gly Ser Thr Tyr Arg Cys Val Ser Val Leu Thr
   580 585 590
   Val Leu His Gln Asp Trp Leu Asn Gly Lys Glu Tyr Lys Cys Lys Val
   595 600 605
   Ser Asn Lys Ala Leu Pro Ala Pro Ile Glu Lys Thr Ile Ser Lys Ala
   610 615 620
   Lys Gly Gln Pro Arg Glu Pro Gln Val Tyr Thr Leu Pro Pro Ser Arg
   625 630 635 640
   Glu Glu Met Thr Lys Asn Gln Val Ser Leu Thr Cys Leu Val Lys Gly
   645 650 655
   Phe Tyr Pro Ser Asp Ile Ala Val Glu Trp Glu Ser Asn Gly Gln Pro
   660 665 670
   Glu Asn Asn Tyr Lys Thr Thr Pro Pro Val Leu Asp Ser Asp Gly Ser
   675 680 685
   Phe Phe Leu Tyr Ser Lys Leu Thr Val Asp Lys Ser Arg Trp Gln Gln
   690 695 700
   Gly Asn Val Phe Ser Cys Ser Val Met His Glu Ala Leu His Asn His
   705 710 715 720
   Tyr Thr Gln Lys Ser Leu Ser Leu Ser Pro Gly Gly Gly Gly Ser Gly
   725 730 735
   Gly Gly Gly Ser Gly Gly Gly Gly Ser Gly Gly Gly Gly Ser Gly Gly
   740 745 750
   Gly Gly Ser Gly Gly Gly Gly Ser Asp Lys Thr His Thr Cys Pro Pro
   755 760 765
   Cys Pro Ala Pro Glu Leu Leu Gly Gly Pro Ser Val Phe Leu Phe Pro
   770 775 780
   Pro Lys Pro Lys Asp Thr Leu Met Ile Ser Arg Thr Pro Glu Val Thr
   785 790 795 800
   Cys Val Val Val Asp Val Ser His Glu Asp Pro Glu Val Lys Phe Asn
   805 810 815
   Trp Tyr Val Asp Gly Val Glu Val His Asn Ala Lys Thr Lys Pro Cys
   820 825 830
   Glu Glu Gln Tyr Gly Ser Thr Tyr Arg Cys Val Ser Val Leu Thr Val
   835 840 845
   Leu His Gln Asp Trp Leu Asn Gly Lys Glu Tyr Lys Cys Lys Val Ser
   850 855 860
   Asn Lys Ala Leu Pro Ala Pro Ile Glu Lys Thr Ile Ser Lys Ala Lys
   865 870 875 880
   Gly Gln Pro Arg Glu Pro Gln Val Tyr Thr Leu Pro Pro Ser Arg Glu
   885 890 895
   Glu Met Thr Lys Asn Gln Val Ser Leu Thr Cys Leu Val Lys Gly Phe
   900 905 910
   Tyr Pro Ser Asp Ile Ala Val Glu Trp Glu Ser Asn Gly Gln Pro Glu
   915 920 925
   Asn Asn Tyr Lys Thr Thr Pro Pro Val Leu Asp Ser Asp Gly Ser Phe
   930 935 940
   Phe Leu Tyr Ser Lys Leu Thr Val Asp Lys Ser Arg Trp Gln Gln Gly
   945 950 955 960
   Asn Val Phe Ser Cys Ser Val Met His Glu Ala Leu His Asn His Tyr
   965 970 975
   Thr Gln Lys Ser Leu Ser Leu Ser Pro Gly Lys
   980 985
<210> 34
   <211> 989
   <212> PRT
   <213> artificial
<220>
   <223> FL_52_CCxCD3-scFc
<400> 34
   Gln Val Thr Leu Lys Glu Ser Gly Pro Thr Leu Val Lys Pro Thr Glu
   1 5 10 15
   Thr Leu Thr Leu Thr Cys Thr Phe Ser Gly Phe Ser Leu Arg Tyr Ala
   20 25 30
   Arg Met Gly Val Ser Trp Ile Arg Gln Pro Pro Gly Lys Cys Leu Glu
   35 40 45
   Trp Leu Ala His Ile Phe Ser Asn Asp Glu Lys Ser Tyr Ser Thr Ser
   50 55 60
   Leu Lys Ser Arg Leu Thr Ile Ser Lys Asp Thr Ser Lys Ser Gln Val
   65 70 75 80
   Val Leu Thr Leu Thr Asn Met Asp Pro Val Asp Thr Ala Thr Tyr Phe
   85 90 95
   Cys Ala Arg Met Pro Glu Tyr Ser Ser Gly Trp Ser Gly Ala Phe Asp
   100 105 110
   Ile Trp Gly Gln Gly Thr Met Val Thr Val Ser Ser Gly Gly Gly Gly
   115 120 125
   Ser Gly Gly Gly Gly Ser Gly Gly Gly Gly Ser Asp Ile Gln Met Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Asp Ile Arg Asn Asp Leu Ala Trp Tyr Gln
                   165                 170                 175
   Gln Lys Pro Gly Lys Ala Pro Lys Arg Leu Ile Tyr Ala Ala Ser Ser
               180                 185                 190
   Leu Gln Ser Gly Val Pro Ser Arg Phe Ser Gly Ser Gly Ser Gly Thr
           195                 200                 205
   Glu Phe Thr Leu Thr Ile Ser Ser Leu Gln Pro Glu Asp Phe Ala Thr
       210                 215                 220
   Tyr Tyr Cys Leu Gln His Asn Ser Tyr Pro Leu Thr Phe Gly Cys Gly
   225                 230                 235                 240
   Thr Lys Leu Glu Ile Lys Ser Gly Gly Gly Gly Ser Glu Val Gln Leu
                   245                 250                 255
   Val Glu Ser Gly Gly Gly Leu Val Gln Pro Gly Gly Ser Leu Lys Leu
               260                 265                 270
   Ser Cys Ala Ala Ser Gly Phe Thr Phe Asn Lys Tyr Ala Met Asn Trp
           275                 280                 285
   Val Arg Gln Ala Pro Gly Lys Gly Leu Glu Trp Val Ala Arg Ile Arg
       290                 295                 300
   Ser Lys Tyr Asn Asn Tyr Ala Thr Tyr Tyr Ala Asp Ser Val Lys Asp
   305                 310                 315                 320
   Arg Phe Thr Ile Ser Arg Asp Asp Ser Lys Asn Thr Ala Tyr Leu Gln
                   325                 330                 335
   Met Asn Asn Leu Lys Thr Glu Asp Thr Ala Val Tyr Tyr Cys Val Arg
               340                 345                 350
   His Gly Asn Phe Gly Asn Ser Tyr Ile Ser Tyr Trp Ala Tyr Trp Gly
           355                 360                 365
   Gln Gly Thr Leu Val Thr Val Ser Ser Gly Gly Gly Gly Ser Gly Gly
       370                 375                 380
   Gly Gly Ser Gly Gly Gly Gly Ser Gln Thr Val Val Thr Gln Glu Pro
   385 390 395 400
   Ser Leu Thr Val Ser Pro Gly Gly Thr Val Thr Leu Thr Cys Gly Ser
   405 410 415
   Ser Thr Gly Ala Val Thr Ser Gly Asn Tyr Pro Asn Trp Val Gln Gln
   420 425 430
   Lys Pro Gly Gln Ala Pro Arg Gly Leu Ile Gly Gly Thr Lys Phe Leu
   435 440 445
   Ala Pro Gly Thr Pro Ala Arg Phe Ser Gly Ser Leu Leu Gly Gly Lys
   450 455 460
   Ala Ala Leu Thr Leu Ser Gly Val Gln Pro Glu Asp Glu Ala Glu Tyr
   465 470 475 480
   Tyr Cys Val Leu Trp Tyr Ser Asn Arg Trp Val Phe Gly Gly Gly Thr
   485 490 495
   Lys Leu Thr Val Leu Gly Gly Gly Gly Asp Lys Thr His Thr Cys Pro
   500 505 510
   Pro Cys Pro Ala Pro Glu Leu Leu Gly Gly Pro Ser Val Phe Leu Phe
   515 520 525
   Pro Pro Lys Pro Lys Asp Thr Leu Met Ile Ser Arg Thr Pro Glu Val
   530 535 540
   Thr Cys Val Val Val Asp Val Ser His Glu Asp Pro Glu Val Lys Phe
   545 550 555 560
   Asn Trp Tyr Val Asp Gly Val Glu Val His Asn Ala Lys Thr Lys Pro
   565 570 575
   Cys Glu Glu Gln Tyr Gly Ser Thr Tyr Arg Cys Val Ser Val Leu Thr
   580 585 590
   Val Leu His Gln Asp Trp Leu Asn Gly Lys Glu Tyr Lys Cys Lys Val
   595 600 605
   Ser Asn Lys Ala Leu Pro Ala Pro Ile Glu Lys Thr Ile Ser Lys Ala
   610 615 620
   Lys Gly Gln Pro Arg Glu Pro Gln Val Tyr Thr Leu Pro Pro Ser Arg
   625 630 635 640
   Glu Glu Met Thr Lys Asn Gln Val Ser Leu Thr Cys Leu Val Lys Gly
   645 650 655
   Phe Tyr Pro Ser Asp Ile Ala Val Glu Trp Glu Ser Asn Gly Gln Pro
   660 665 670
   Glu Asn Asn Tyr Lys Thr Thr Pro Pro Val Leu Asp Ser Asp Gly Ser
   675 680 685
   Phe Phe Leu Tyr Ser Lys Leu Thr Val Asp Lys Ser Arg Trp Gln Gln
   690 695 700
   Gly Asn Val Phe Ser Cys Ser Val Met His Glu Ala Leu His Asn His
   705 710 715 720
   Tyr Thr Gln Lys Ser Leu Ser Leu Ser Pro Gly Lys Gly Gly Gly Gly
   725 730 735
   Ser Gly Gly Gly Gly Ser Gly Gly Gly Gly Ser Gly Gly Gly Gly Ser
   740 745 750
   Gly Gly Gly Gly Ser Gly Gly Gly Gly Ser Asp Lys Thr His Thr Cys
   755 760 765
   Pro Pro Cys Pro Ala Pro Glu Leu Leu Gly Gly Pro Ser Val Phe Leu
   770 775 780
   Phe Pro Pro Lys Pro Lys Asp Thr Leu Met Ile Ser Arg Thr Pro Glu
   785 790 795 800
   Val Thr Cys Val Val Val Asp Val Ser His Glu Asp Pro Glu Val Lys
   805 810 815
   Phe Asn Trp Tyr Val Asp Gly Val Glu Val His Asn Ala Lys Thr Lys
   820 825 830
   Pro Cys Glu Glu Gln Tyr Gly Ser Thr Tyr Arg Cys Val Ser Val Leu
   835 840 845
   Thr Val Leu His Gln Asp Trp Leu Asn Gly Lys Glu Tyr Lys Cys Lys
   850 855 860
   Val Ser Asn Lys Ala Leu Pro Ala Pro Ile Glu Lys Thr Ile Ser Lys
   865 870 875 880
   Ala Lys Gly Gln Pro Arg Glu Pro Gln Val Tyr Thr Leu Pro Pro Ser
   885 890 895
   Arg Glu Glu Met Thr Lys Asn Gln Val Ser Leu Thr Cys Leu Val Lys
   900 905 910
   Gly Phe Tyr Pro Ser Asp Ile Ala Val Glu Trp Glu Ser Asn Gly Gln
   915 920 925
   Pro Glu Asn Asn Tyr Lys Thr Thr Pro Pro Val Leu Asp Ser Asp Gly
   930 935 940
   Ser Phe Phe Leu Tyr Ser Lys Leu Thr Val Asp Lys Ser Arg Trp Gln
   945 950 955 960
   Gln Gly Asn Val Phe Ser Cys Ser Val Met His Glu Ala Leu His Asn
   965 970 975
   His Tyr Thr Gln Lys Ser Leu Ser Leu Ser Pro Gly Lys
   980 985
<210> 35
   <211> 987
   <212> PRT
   <213> artificial
<220>
   <223> FL_52_CCxCD3-scFc_delGK
<400> 35
   Gln Val Thr Leu Lys Glu Ser Gly Pro Thr Leu Val Lys Pro Thr Glu
   1               5                   10                  15
   Thr Leu Thr Leu Thr Cys Thr Phe Ser Gly Phe Ser Leu Arg Tyr Ala
               20                  25                  30
   Arg Met Gly Val Ser Trp Ile Arg Gln Pro Pro Gly Lys Cys Leu Glu
           35                  40                  45
   Trp Leu Ala His Ile Phe Ser Asn Asp Glu Lys Ser Tyr Ser Thr Ser
       50                  55                  60
   Leu Lys Ser Arg Leu Thr Ile Ser Lys Asp Thr Ser Lys Ser Gln Val
   65                  70                  75                  80
   Val Leu Thr Leu Thr Asn Met Asp Pro Val Asp Thr Ala Thr Tyr Phe
                   85                  90                  95
   Cys Ala Arg Met Pro Glu Tyr Ser Ser Gly Trp Ser Gly Ala Phe Asp
               100                 105                 110
   Ile Trp Gly Gln Gly Thr Met Val Thr Val Ser Ser Gly Gly Gly Gly
   115 120 125
   Ser Gly Gly Gly Gly Ser Gly Gly Gly Gly Ser Asp Ile Gln Met Thr
   130 135 140
   Gln Ser Pro Ser Ser Leu Ser Ala Ser Val Gly Asp Arg Val Thr Ile
   145 150 155 160
   Thr Cys Arg Ala Ser Gln Asp Ile Arg Asn Asp Leu Ala Trp Tyr Gln
   165 170 175
   Gln Lys Pro Gly Lys Ala Pro Lys Arg Leu Ile Tyr Ala Ala Ser Ser
   180 185 190
   Leu Gln Ser Gly Val Pro Ser Arg Phe Ser Gly Ser Gly Ser Gly Thr
   195 200 205
   Glu Phe Thr Leu Thr Ile Ser Ser Leu Gln Pro Glu Asp Phe Ala Thr
   210 215 220
   Tyr Tyr Cys Leu Gln His Asn Ser Tyr Pro Leu Thr Phe Gly Cys Gly
   225 230 235 240
   Thr Lys Leu Glu Ile Lys Ser Gly Gly Gly Gly Ser Glu Val Gln Leu
   245 250 255
   Val Glu Ser Gly Gly Gly Leu Val Gln Pro Gly Gly Ser Leu Lys Leu
   260 265 270
   Ser Cys Ala Ala Ser Gly Phe Thr Phe Asn Lys Tyr Ala Met Asn Trp
   275 280 285
   Val Arg Gln Ala Pro Gly Lys Gly Leu Glu Trp Val Ala Arg Ile Arg
   290 295 300
   Ser Lys Tyr Asn Asn Tyr Ala Thr Tyr Tyr Ala Asp Ser Val Lys Asp
   305 310 315 320
   Arg Phe Thr Ile Ser Arg Asp Asp Ser Lys Asn Thr Ala Tyr Leu Gln
   325 330 335
   Met Asn Asn Leu Lys Thr Glu Asp Thr Ala Val Tyr Tyr Cys Val Arg
   340 345 350
   His Gly Asn Phe Gly Asn Ser Tyr Ile Ser Tyr Trp Ala Tyr Trp Gly
   355 360 365
   Gln Gly Thr Leu Val Thr Val Ser Ser Gly Gly Gly Gly Ser Gly Gly
   370 375 380
   Gly Gly Ser Gly Gly Gly Gly Ser Gln Thr Val Val Thr Gln Glu Pro
   385 390 395 400
   Ser Leu Thr Val Ser Pro Gly Gly Thr Val Thr Leu Thr Cys Gly Ser
   405 410 415
   Ser Thr Gly Ala Val Thr Ser Gly Asn Tyr Pro Asn Trp Val Gln Gln
   420 425 430
   Lys Pro Gly Gln Ala Pro Arg Gly Leu Ile Gly Gly Thr Lys Phe Leu
   435 440 445
   Ala Pro Gly Thr Pro Ala Arg Phe Ser Gly Ser Leu Leu Gly Gly Lys
   450 455 460
   Ala Ala Leu Thr Leu Ser Gly Val Gln Pro Glu Asp Glu Ala Glu Tyr
   465 470 475 480
   Tyr Cys Val Leu Trp Tyr Ser Asn Arg Trp Val Phe Gly Gly Gly Thr
   485 490 495
   Lys Leu Thr Val Leu Gly Gly Gly Gly Asp Lys Thr His Thr Cys Pro
   500 505 510
   Pro Cys Pro Ala Pro Glu Leu Leu Gly Gly Pro Ser Val Phe Leu Phe
   515 520 525
   Pro Pro Lys Pro Lys Asp Thr Leu Met Ile Ser Arg Thr Pro Glu Val
   530 535 540
   Thr Cys Val Val Val Asp Val Ser His Glu Asp Pro Glu Val Lys Phe
   545 550 555 560
   Asn Trp Tyr Val Asp Gly Val Glu Val His Asn Ala Lys Thr Lys Pro
   565 570 575
   Cys Glu Glu Gln Tyr Gly Ser Thr Tyr Arg Cys Val Ser Val Leu Thr
   580 585 590
   Val Leu His Gln Asp Trp Leu Asn Gly Lys Glu Tyr Lys Cys Lys Val
   595 600 605
   Ser Asn Lys Ala Leu Pro Ala Pro Ile Glu Lys Thr Ile Ser Lys Ala
       610                 615                 620
   Lys Gly Gln Pro Arg Glu Pro Gln Val Tyr Thr Leu Pro Pro Ser Arg
   625                 630                 635                 640
   Glu Glu Met Thr Lys Asn Gln Val Ser Leu Thr Cys Leu Val Lys Gly
                   645                 650                 655
   Phe Tyr Pro Ser Asp Ile Ala Val Glu Trp Glu Ser Asn Gly Gln Pro
               660                 665                 670
   Glu Asn Asn Tyr Lys Thr Thr Pro Pro Val Leu Asp Ser Asp Gly Ser
           675                 680                 685
   Phe Phe Leu Tyr Ser Lys Leu Thr Val Asp Lys Ser Arg Trp Gln Gln
       690                 695                 700
   Gly Asn Val Phe Ser Cys Ser Val Met His Glu Ala Leu His Asn His
   705                 710                 715                 720
   Tyr Thr Gln Lys Ser Leu Ser Leu Ser Pro Gly Gly Gly Gly Ser Gly
                   725                 730                 735
   Gly Gly Gly Ser Gly Gly Gly Gly Ser Gly Gly Gly Gly Ser Gly Gly
               740                 745                 750
   Gly Gly Ser Gly Gly Gly Gly Ser Asp Lys Thr His Thr Cys Pro Pro
           755                 760                 765
   Cys Pro Ala Pro Glu Leu Leu Gly Gly Pro Ser Val Phe Leu Phe Pro
       770                 775                 780
   Pro Lys Pro Lys Asp Thr Leu Met Ile Ser Arg Thr Pro Glu Val Thr
   785                 790                 795                 800
   Cys Val Val Val Asp Val Ser His Glu Asp Pro Glu Val Lys Phe Asn
                   805                 810                 815
   Trp Tyr Val Asp Gly Val Glu Val His Asn Ala Lys Thr Lys Pro Cys
               820                 825                 830
   Glu Glu Gln Tyr Gly Ser Thr Tyr Arg Cys Val Ser Val Leu Thr Val
           835                 840                 845
   Leu His Gln Asp Trp Leu Asn Gly Lys Glu Tyr Lys Cys Lys Val Ser
       850                 855                 860
   Asn Lys Ala Leu Pro Ala Pro Ile Glu Lys Thr Ile Ser Lys Ala Lys
   865 870 875 880
   Gly Gln Pro Arg Glu Pro Gln Val Tyr Thr Leu Pro Pro Ser Arg Glu
   885 890 895
   Glu Met Thr Lys Asn Gln Val Ser Leu Thr Cys Leu Val Lys Gly Phe
   900 905 910
   Tyr Pro Ser Asp Ile Ala Val Glu Trp Glu Ser Asn Gly Gln Pro Glu
   915 920 925
   Asn Asn Tyr Lys Thr Thr Pro Pro Val Leu Asp Ser Asp Gly Ser Phe
   930 935 940
   Phe Leu Tyr Ser Lys Leu Thr Val Asp Lys Ser Arg Trp Gln Gln Gly
   945 950 955 960
   Asn Val Phe Ser Cys Ser Val Met His Glu Ala Leu His Asn His Tyr
   965 970 975
   Thr Gln Lys Ser Leu Ser Leu Ser Pro Gly Lys
   980 985
<210> 36
   <211> 994
   <212> PRT
   <213> artificial
<220>
   <223> FL_61xCD3-scFc
<400> 36
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Ser Tyr Glu Gly Ser Asn Glu Phe Tyr Ala Glu Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Gly Glu Ile Thr Met Val Arg Gly Val Ile Gly Tyr Tyr
   100 105 110
   Tyr Tyr Gly Met Asp Val Trp Gly Gln Gly Thr Thr Val Thr Val Ser
   115 120 125
   Ser Gly Gly Gly Gly Ser Gly Gly Gly Gly Ser Gly Gly Gly Gly Ser
   130 135 140
   Asp Ile Gln Met Thr Gln Ser Pro Ser Ser Leu Ser Ala Ser Val Gly
   145 150 155 160
   Asp Arg Val Thr Ile Thr Cys Arg Ala Ser Gln Ser Ile Ser Ser Tyr
   165 170 175
   Leu Asn Trp Tyr Gln Gln Lys Pro Gly Lys Ala Pro Lys Leu Leu Ile
   180 185 190
   Tyr Ala Ala Ser Ser Leu Gln Ser Gly Val Pro Ser Arg Phe Ser Gly
   195 200 205
   Ser Gly Ser Gly Thr Glu Phe Thr Leu Thr Ile Ser Ser Leu Gln Pro
   210 215 220
   Glu Asp Phe Ala Thr Tyr Tyr Cys Leu Gln His Asn Ser Tyr Pro Leu
   225 230 235 240
   Thr Phe Gly Gly Gly Thr Lys Val Glu Ile Lys Ser Gly Gly Gly Gly
   245 250 255
   Ser Glu Val Gln Leu Val Glu Ser Gly Gly Gly Leu Val Gln Pro Gly
   260 265 270
   Gly Ser Leu Lys Leu Ser Cys Ala Ala Ser Gly Phe Thr Phe Asn Lys
   275 280 285
   Tyr Ala Met Asn Trp Val Arg Gln Ala Pro Gly Lys Gly Leu Glu Trp
   290 295 300
   Val Ala Arg Ile Arg Ser Lys Tyr Asn Asn Tyr Ala Thr Tyr Tyr Ala
   305 310 315 320
   Asp Ser Val Lys Asp Arg Phe Thr Ile Ser Arg Asp Asp Ser Lys Asn
   325 330 335
   Thr Ala Tyr Leu Gln Met Asn Asn Leu Lys Thr Glu Asp Thr Ala Val
               340                 345                 350
   Tyr Tyr Cys Val Arg His Gly Asn Phe Gly Asn Ser Tyr Ile Ser Tyr
           355                 360                 365
   Trp Ala Tyr Trp Gly Gln Gly Thr Leu Val Thr Val Ser Ser Gly Gly
       370                 375                 380
   Gly Gly Ser Gly Gly Gly Gly Ser Gly Gly Gly Gly Ser Gln Thr Val
   385                 390                 395                 400
   Val Thr Gln Glu Pro Ser Leu Thr Val Ser Pro Gly Gly Thr Val Thr
                   405                 410                 415
   Leu Thr Cys Gly Ser Ser Thr Gly Ala Val Thr Ser Gly Asn Tyr Pro
               420                 425                 430
   Asn Trp Val Gln Gln Lys Pro Gly Gln Ala Pro Arg Gly Leu Ile Gly
           435                 440                 445
   Gly Thr Lys Phe Leu Ala Pro Gly Thr Pro Ala Arg Phe Ser Gly Ser
       450                 455                 460
   Leu Leu Gly Gly Lys Ala Ala Leu Thr Leu Ser Gly Val Gln Pro Glu
   465                 470                 475                 480
   Asp Glu Ala Glu Tyr Tyr Cys Val Leu Trp Tyr Ser Asn Arg Trp Val
                   485                 490                 495
   Phe Gly Gly Gly Thr Lys Leu Thr Val Leu Gly Gly Gly Gly Asp Lys
               500                 505                 510
   Thr His Thr Cys Pro Pro Cys Pro Ala Pro Glu Leu Leu Gly Gly Pro
           515                 520                 525
   Ser Val Phe Leu Phe Pro Pro Lys Pro Lys Asp Thr Leu Met Ile Ser
       530                 535                 540
   Arg Thr Pro Glu Val Thr Cys Val Val Val Asp Val Ser His Glu Asp
   545                 550                 555                 560
   Pro Glu Val Lys Phe Asn Trp Tyr Val Asp Gly Val Glu Val His Asn
                   565                 570                 575
   Ala Lys Thr Lys Pro Cys Glu Glu Gln Tyr Gly Ser Thr Tyr Arg Cys
               580                 585                 590
   Val Ser Val Leu Thr Val Leu His Gln Asp Trp Leu Asn Gly Lys Glu
   595 600 605
   Tyr Lys Cys Lys Val Ser Asn Lys Ala Leu Pro Ala Pro Ile Glu Lys
   610 615 620
   Thr Ile Ser Lys Ala Lys Gly Gln Pro Arg Glu Pro Gln Val Tyr Thr
   625 630 635 640
   Leu Pro Pro Ser Arg Glu Glu Met Thr Lys Asn Gln Val Ser Leu Thr
   645 650 655
   Cys Leu Val Lys Gly Phe Tyr Pro Ser Asp Ile Ala Val Glu Trp Glu
   660 665 670
   Ser Asn Gly Gln Pro Glu Asn Asn Tyr Lys Thr Thr Pro Pro Val Leu
   675 680 685
   Asp Ser Asp Gly Ser Phe Phe Leu Tyr Ser Lys Leu Thr Val Asp Lys
   690 695 700
   Ser Arg Trp Gln Gln Gly Asn Val Phe Ser Cys Ser Val Met His Glu
   705 710 715 720
   Ala Leu His Asn His Tyr Thr Gln Lys Ser Leu Ser Leu Ser Pro Gly
   725 730 735
   Lys Gly Gly Gly Gly Ser Gly Gly Gly Gly Ser Gly Gly Gly Gly Ser
   740 745 750
   Gly Gly Gly Gly Ser Gly Gly Gly Gly Ser Gly Gly Gly Gly Ser Asp
   755 760 765
   Lys Thr His Thr Cys Pro Pro Cys Pro Ala Pro Glu Leu Leu Gly Gly
   770 775 780
   Pro Ser Val Phe Leu Phe Pro Pro Lys Pro Lys Asp Thr Leu Met Ile
   785 790 795 800
   Ser Arg Thr Pro Glu Val Thr Cys Val Val Val Asp Val Ser His Glu
   805 810 815
   Asp Pro Glu Val Lys Phe Asn Trp Tyr Val Asp Gly Val Glu Val His
   820 825 830
   Asn Ala Lys Thr Lys Pro Cys Glu Glu Gln Tyr Gly Ser Thr Tyr Arg
   835 840 845
   Cys Val Ser Val Leu Thr Val Leu His Gln Asp Trp Leu Asn Gly Lys
   850 855 860
   Glu Tyr Lys Cys Lys Val Ser Asn Lys Ala Leu Pro Ala Pro Ile Glu
   865 870 875 880
   Lys Thr Ile Ser Lys Ala Lys Gly Gln Pro Arg Glu Pro Gln Val Tyr
   885 890 895
   Thr Leu Pro Pro Ser Arg Glu Glu Met Thr Lys Asn Gln Val Ser Leu
   900 905 910
   Thr Cys Leu Val Lys Gly Phe Tyr Pro Ser Asp Ile Ala Val Glu Trp
   915 920 925
   Glu Ser Asn Gly Gln Pro Glu Asn Asn Tyr Lys Thr Thr Pro Pro Val
   930 935 940
   Leu Asp Ser Asp Gly Ser Phe Phe Leu Tyr Ser Lys Leu Thr Val Asp
   945 950 955 960
   Lys Ser Arg Trp Gln Gln Gly Asn Val Phe Ser Cys Ser Val Met His
   965 970 975
   Glu Ala Leu His Asn His Tyr Thr Gln Lys Ser Leu Ser Leu Ser Pro
   980 985 990
   Gly Lys
<210> 37
   <211> 992
   <212> PRT
   <213> artificial
<220>
   <223> FL_61xCD3-scFc_delGK
<400> 37
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Glu Gly Ser Asn Glu Phe Tyr Ala Glu Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Gly Glu Ile Thr Met Val Arg Gly Val Ile Gly Tyr Tyr
   100 105 110
   Tyr Tyr Gly Met Asp Val Trp Gly Gln Gly Thr Thr Val Thr Val Ser
   115 120 125
   Ser Gly Gly Gly Gly Ser Gly Gly Gly Gly Ser Gly Gly Gly Gly Ser
   130 135 140
   Asp Ile Gln Met Thr Gln Ser Pro Ser Ser Leu Ser Ala Ser Val Gly
   145 150 155 160
   Asp Arg Val Thr Ile Thr Cys Arg Ala Ser Gln Ser Ile Ser Ser Tyr
   165 170 175
   Leu Asn Trp Tyr Gln Gln Lys Pro Gly Lys Ala Pro Lys Leu Leu Ile
   180 185 190
   Tyr Ala Ala Ser Ser Leu Gln Ser Gly Val Pro Ser Arg Phe Ser Gly
   195 200 205
   Ser Gly Ser Gly Thr Glu Phe Thr Leu Thr Ile Ser Ser Leu Gln Pro
   210 215 220
   Glu Asp Phe Ala Thr Tyr Tyr Cys Leu Gln His Asn Ser Tyr Pro Leu
   225 230 235 240
   Thr Phe Gly Gly Gly Thr Lys Val Glu Ile Lys Ser Gly Gly Gly Gly
   245 250 255
   Ser Glu Val Gln Leu Val Glu Ser Gly Gly Gly Leu Val Gln Pro Gly
   260 265 270
   Gly Ser Leu Lys Leu Ser Cys Ala Ala Ser Gly Phe Thr Phe Asn Lys
   275 280 285
   Tyr Ala Met Asn Trp Val Arg Gln Ala Pro Gly Lys Gly Leu Glu Trp
       290                 295                 300
   Val Ala Arg Ile Arg Ser Lys Tyr Asn Asn Tyr Ala Thr Tyr Tyr Ala
   305                 310                 315                 320
   Asp Ser Val Lys Asp Arg Phe Thr Ile Ser Arg Asp Asp Ser Lys Asn
                   325                 330                 335
   Thr Ala Tyr Leu Gln Met Asn Asn Leu Lys Thr Glu Asp Thr Ala Val
               340                 345                 350
   Tyr Tyr Cys Val Arg His Gly Asn Phe Gly Asn Ser Tyr Ile Ser Tyr
           355                 360                 365
   Trp Ala Tyr Trp Gly Gln Gly Thr Leu Val Thr Val Ser Ser Gly Gly
       370                 375                 380
   Gly Gly Ser Gly Gly Gly Gly Ser Gly Gly Gly Gly Ser Gln Thr Val
   385                 390                 395                 400
   Val Thr Gln Glu Pro Ser Leu Thr Val Ser Pro Gly Gly Thr Val Thr
                   405                 410                 415
   Leu Thr Cys Gly Ser Ser Thr Gly Ala Val Thr Ser Gly Asn Tyr Pro
               420                 425                 430
   Asn Trp Val Gln Gln Lys Pro Gly Gln Ala Pro Arg Gly Leu Ile Gly
           435                 440                 445
   Gly Thr Lys Phe Leu Ala Pro Gly Thr Pro Ala Arg Phe Ser Gly Ser
       450                 455                 460
   Leu Leu Gly Gly Lys Ala Ala Leu Thr Leu Ser Gly Val Gln Pro Glu
   465                 470                 475                 480
   Asp Glu Ala Glu Tyr Tyr Cys Val Leu Trp Tyr Ser Asn Arg Trp Val
                   485                 490                 495
   Phe Gly Gly Gly Thr Lys Leu Thr Val Leu Gly Gly Gly Gly Asp Lys
               500                 505                 510
   Thr His Thr Cys Pro Pro Cys Pro Ala Pro Glu Leu Leu Gly Gly Pro
           515                 520                 525
   Ser Val Phe Leu Phe Pro Pro Lys Pro Lys Asp Thr Leu Met Ile Ser
       530                 535                 540
   Arg Thr Pro Glu Val Thr Cys Val Val Val Asp Val Ser His Glu Asp
   545                 550                 555                 560
   Pro Glu Val Lys Phe Asn Trp Tyr Val Asp Gly Val Glu Val His Asn
                   565                 570                 575
   Ala Lys Thr Lys Pro Cys Glu Glu Gln Tyr Gly Ser Thr Tyr Arg Cys
               580                 585                 590
   Val Ser Val Leu Thr Val Leu His Gln Asp Trp Leu Asn Gly Lys Glu
           595                 600                 605
   Tyr Lys Cys Lys Val Ser Asn Lys Ala Leu Pro Ala Pro Ile Glu Lys
       610                 615                 620
   Thr Ile Ser Lys Ala Lys Gly Gln Pro Arg Glu Pro Gln Val Tyr Thr
   625                 630                 635                 640
   Leu Pro Pro Ser Arg Glu Glu Met Thr Lys Asn Gln Val Ser Leu Thr
                   645                 650                 655
   Cys Leu Val Lys Gly Phe Tyr Pro Ser Asp Ile Ala Val Glu Trp Glu
               660                 665                 670
   Ser Asn Gly Gln Pro Glu Asn Asn Tyr Lys Thr Thr Pro Pro Val Leu
           675                 680                 685
   Asp Ser Asp Gly Ser Phe Phe Leu Tyr Ser Lys Leu Thr Val Asp Lys
       690                 695                 700
   Ser Arg Trp Gln Gln Gly Asn Val Phe Ser Cys Ser Val Met His Glu
   705                 710                 715                 720
   Ala Leu His Asn His Tyr Thr Gln Lys Ser Leu Ser Leu Ser Pro Gly
                   725                 730                 735
   Gly Gly Gly Ser Gly Gly Gly Gly Ser Gly Gly Gly Gly Ser Gly Gly
               740                 745                 750
   Gly Gly Ser Gly Gly Gly Gly Ser Gly Gly Gly Gly Ser Asp Lys Thr
           755                 760                 765
   His Thr Cys Pro Pro Cys Pro Ala Pro Glu Leu Leu Gly Gly Pro Ser
       770                 775                 780
   Val Phe Leu Phe Pro Pro Lys Pro Lys Asp Thr Leu Met Ile Ser Arg
   785                 790                 795                 800
   Thr Pro Glu Val Thr Cys Val Val Val Asp Val Ser His Glu Asp Pro
                   805                 810                 815
   Glu Val Lys Phe Asn Trp Tyr Val Asp Gly Val Glu Val His Asn Ala
               820                 825                 830
   Lys Thr Lys Pro Cys Glu Glu Gln Tyr Gly Ser Thr Tyr Arg Cys Val
           835                 840                 845
   Ser Val Leu Thr Val Leu His Gln Asp Trp Leu Asn Gly Lys Glu Tyr
       850                 855                 860
   Lys Cys Lys Val Ser Asn Lys Ala Leu Pro Ala Pro Ile Glu Lys Thr
   865                 870                 875                 880
   Ile Ser Lys Ala Lys Gly Gln Pro Arg Glu Pro Gln Val Tyr Thr Leu
                   885                 890                 895
   Pro Pro Ser Arg Glu Glu Met Thr Lys Asn Gln Val Ser Leu Thr Cys
               900                 905                 910
   Leu Val Lys Gly Phe Tyr Pro Ser Asp Ile Ala Val Glu Trp Glu Ser
           915                 920                 925
   Asn Gly Gln Pro Glu Asn Asn Tyr Lys Thr Thr Pro Pro Val Leu Asp
       930                 935                 940
   Ser Asp Gly Ser Phe Phe Leu Tyr Ser Lys Leu Thr Val Asp Lys Ser
   945                 950                 955                 960
   Arg Trp Gln Gln Gly Asn Val Phe Ser Cys Ser Val Met His Glu Ala
                   965                 970                 975
   Leu His Asn His Tyr Thr Gln Lys Ser Leu Ser Leu Ser Pro Gly Lys
               980                 985                 990

## Description

### FIELD OF THE INVENTION

The present invention provides immune-checkpoint inhibitors for use in the treatment of blood-borne cancers, in particular AML. The invention further relates to pharmaceutical compositions comprising said immune-checkpoint inhibitors and antibody constructs capable of engaging T cells.

### BACKGROUND OF THE INVENTION

Among the most promising approaches to activating therapeutic anti-tumor immunity is the blockade of immune checkpoints. Immune-checkpoints refer to a plethora of inhibitory pathways hardwired into the immune system that are crucial for maintaining self-tolerance and modulating the duration and amplitude of physiological immune responses in peripheral tissues in order to minimize collateral tissue damage.

In general, T cells do not respond to these ligand-receptor interactions unless they first recognize their cognate antigen through the TCR. Many of the ligands bind to multiple receptors, some of which deliver co-stimulatory signals and others deliver inhibitory signals. In general, pairs of co-stimulatory-inhibitory receptors that bind the same ligand or ligands - such as CD28 and cytotoxic T-lymphocyte-associated antigen 4 (CTLA4) - display distinct kinetics of expression with the co-stimulatory receptor expressed on naive and resting T cells, but the inhibitory receptor is commonly upregulated after T cell activation. One important family of membrane-bound ligands that bind both co-stimulatory and inhibitory receptors is the B7 family. All of the B7 family members and their known ligands belong to the immunoglobulin superfamily. Many of the receptors for more recently identified B7 family members have not yet been identified. TNF family members that bind to cognate TNF receptor family molecules represent a second family of regulatory ligand-receptor pairs. These receptors predominantly deliver co-stimulatory signals when engaged by their cognate ligands.

Another major category of signals that regulate the activation of T cells comes from soluble cytokines in the microenvironment. Communication between T cells and APCs is bidirectional. In some cases, this occurs when ligands themselves signal to the APC. In other cases, activated T cells upregulate ligands, such as CD40L, that engage cognate receptors on APCs.

However, tumors co-opt certain immune-checkpoint pathways as a major mechanism of immune resistance, particularly against T cells that are specific for tumor antigens. Because many of the immune-checkpoints are initiated by ligand-receptor interactions, they can be blocked by antibodies or modulated by recombinant forms of ligands or receptors. CTLA4 antibodies were the first of this class of immunotherapeutics to achieve FDA approval. Preliminary clinical findings with blockers of additional immune-checkpoint proteins, such as PD-1, indicate broad and diverse opportunities to enhance anti-tumor immunity with the potential to produce durable clinical responses. For example, Krupka et al. ("Blockade of the PD-1/PD-L1 axis augments lysis of AML cells by the CD33/CD3 BiTE® antibody construct AMG 330: reversing a T-cell-induced immune escape mechanism.", LEUKEMIA FEB 2016, vol. 30, no. 2, pages 484-491) discloses the use of anti-PD-1 antibody in combination with anti-CD33/CD3 BiTE® antibody construct to kill AML cells in vitro.

Cancer immunotherapy thus also focuses on the development of agents that can render neoplastic and cancer cells more amenable to killing by the immune system, particularly by T cells. This can, for example, be achieved by interfering with immune checkpoint proteins, either with ligands or receptors or both. Although the knowledge for immune-checkpoint molecules as well as the knowledge which immune-checkpoint molecules are used by which cancer cells increases, it is not known which of them may be used by which neoplastic and then cancer cells to evade the immune system, thereby gaining the capability of uncontrolled growth. For example, blood-borne cancers, in particular acute myeloid leukemia (AML) are meanwhile known to evade the immune system. Though it is speculated that immune-checkpoint proteins may be involved, there is not yet proof for this. Sanchez-Correa Beatriz et al. ("Natural killer cell immunosenescence in acute myeloid leukaemia patients: new targets for immunotherapeutic strategies?", CANCER IMMUNOLOGY , IMMUNOTHERAPY : CII APR 2016, vol. 65, no. 4, pages 453-463) reviews the mechanism of NK cell anergy in AML, discusses regulation of NK cells via TIGIT and DNAM-1 ligands (i.e. CD112, CD155) and discloses CD112 expression on AML cells. Hou Shengke et al. ("Recombinant soluble CD226 protein directly inhibits cancer cell proliferation in vitro.", INTERNATIONAL IMMUNOPHARMACOLOGY MAR 2014, vol. 19, no. 1, March 2014, pages 119-126) discloses the use of sCD266 (soluble CD226, i.e. DNAM-1) for inhibiting leukemic cell growth (Item 3.3). CD226 is a receptor for both CD112 and CD155. sCD226 is thus at least an inhibitor of CD112/CD226 and CD155/ CD226 interaction. There is thus an unmet need to provide means and methods for overriding the influence of immune-checkpoint proteins in order to make blood-borne cancer cells more amenable to access of the killing machinery of the immune system.

Accordingly, the technical problem underlying the present application is to satisfy this unmet need, i.e. to provide means and methods for making blood-borne cancer cells, particularly AML cells more amenable to killing by the body's immune system. The solution is, in general, the provision of inhibitors of the immune-checkpoint ligands CD112, CD155, their receptor TIGIT for use in the treatment of blood-borne cancers, in particular AML. Said solution is also reflected in the claims, embodied in the description, exemplified in the appended Examples and illustrated in the Figures.

### SUMMARY OF THE INVENTION

The subject-matter of the invention is identified in the appended claims.

The present invention relates to compounds that modulate the immune system for use in the treatment of blood-borne cancers, such as lymphoma or leukemia, particularly AML. Moreover, the present invention relates to a pharmaceutical composition comprising said immunomodulating compounds and, as an aspect of the disclosure, chimeric antigen receptor T cells (CAR T cells). Further, the present invention provides a pharmaceutical composition comprising said immunomodulating compounds and an antibody construct capable of engaging T cells.

In this regard the present invention pays attention to the need of providing new immune-checkpoints in blood-borne cancer therapy, particularly AML therapy that can be influenced by diverse inhibitors in order to treat cancer cells that have developed immune escape mechanisms. In particular, the present inventors discovered CD112, CD155, and TIGIT -and as aspects of the disclosure Galectin-9 and TIM-3- as new immune-checkpoints proteins that can be specifically targeted for therapy of blood-borne cancers, particularly AML. Thereby, inhibitors against CD112, CD155, and/or TIGIT - and as aspects of the disclosure Galectin-9 and/or TIM-3 - lead to a significantly increased cell lysis of AML cells. Additionally, the inventors discovered that the impact of said immune-checkpoint inhibitors may be even more effective when combined with -as an aspect of the disclosure chimeric antigen receptors T cells (CAR T cells)- or antibody constructs capable of engaging T cells, such as the bispecific T-cell-engaging (BiTE®) antibody construct AMG 330 having dual specificity for CD3 and CD33. Thus, the compounds and composition of the present invention allow for a novel therapeutic option for patients with blood-borne cancer, in particular AML thereby providing a promising way for making blood-borne cancer cells more amenable to killing by the body's defense. Accordingly, the present invention indicates broad and diverse opportunities to enhance antitumor immunity and provides compounds and composition that seem to have the potential to achieve durable clinical responses.

In a first aspect, the present invention relates to an inhibitor against CD112 (Nectin-2, PVRL2), CD155 (PVR) and/or TIGIT for use in a method of treatment of blood-borne cancers, in particular acute myeloid leukemia (AML). A first group of such an inhibitor inhibits the interaction between immune-check point protein ligands and receptors as described herein. It is thus envisaged that the inhibitor used in the present invention against CD112 inhibits the interaction between CD112 and TIGIT. It is envisaged that the inhibitor used in the present invention against CD155 inhibits the interaction between CD155 and TIGIT. It is envisaged that the inhibitor used in the present invention against TIGIT inhibits the interaction between TIGIT and CD112. It is envisaged that the inhibitor used in the present invention against TIGIT inhibits the interaction between TIGIT and CD155. As an aspect of the disclosure, it is envisaged that the inhibitor against Galectin-9 inhibits the interaction between Galectin-9 and TIM-3. As an aspect of the disclosure, it is envisaged that the inhibitor against TIM-3 inhibits the interaction between TIM-3 and Galectin-9. The inhibitor used in the present invention is an antibody construct.

As an aspect of the disclosure, it is also envisaged that the inhibitor can further comprise a CAR T cell. The inhibitor can also comprise an antibody construct that is capable of engaging T cells. The antibody construct capable of engaging T cells preferably comprises a CD3 binding domain and a further binding domain targeting a surface molecule expressed on AML cells. Said surface molecule may be selected from the group consisting of CD33, CD19, and Flt3. The antibody construct capable of engaging T cells is preferably a binding molecule capable of binding to CD3epsilon. The inhibitor may further comprise an immunostimulant.

A further group of immune-checkpoint inhibitors reduces expression of immune checkpoint proteins as described herein. Accordingly, it is thus envisaged that the inhibitor used in the present invention against CD112 reduces expression of CD112. It is envisaged that the inhibitor used in the present invention against CD155 reduces expression of CD155. It is envisaged that the inhibitor used in the present invention against TIGIT reduces expression of TIGIT. As an aspect of the disclosure, it is envisaged that the inhibitor against Galectin-9 increases expression of Galectin-9. As an aspect of the disclosure, it is envisaged that the inhibitor against TIM3 increases expression of TIM-3. As an aspect of the disclosure, it is envisaged that the inhibitor is an iRNA. It is envisaged that the inhibitor knocks out CD112, CD155, and/or TIGIT. The knock-out may be achieved by CRISPR/cas9 technique.

Another group of immune-checkpoint inhibitors modulates intracellular signaling of the immune-checkpoint proteins as described herein. It is thus envisaged that the inhibitor used in the present invention against CD112 modulates intracellular signaling of CD112. It is envisaged that the inhibitor used in the present invention against CD155 modulates intracellular signaling of CD155. It is envisaged that the inhibitor used in the present invention against TIGIT modulates intracellular signaling of TIGIT. As an aspect of the disclosure, it is envisaged that the inhibitor against Galectin-9 modulates intracellular signaling of Galectin-9. As an aspect of the disclosure, it is envisaged that the inhibitor against TIM-3 modulates intracellular signaling of TIM-3.

In a further aspect the disclosure relates to a pharmaceutical composition comprising an inhibitor against CD112 (Nectin-2, PVRL2), CD155 (PVR), Galectin-9, TIM-3 and/or TIGIT and a CAR T cell. Said inhibitor against CD112 (Nectin-2, PVRL2), CD155 (PVR), and/or TIGIT may be an antibody construct.

In a further aspect, a pharmaceutical composition comprising an inhibitor against CD112 (Nectin-2, PVRL2), CD155 (PVR), and/or TIGIT and an antibody construct that is capable of engaging T cells is provided. Said antibody construct capable of engaging T cells may comprise a CD3 binding domain and a further binding domain targeting a surface molecule expressed on AML cells.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series.
The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.
The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.
In each instance herein any of the terms "comprising", "consisting essentially of" and "consisting of" may be replaced with either of the other two terms.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

### BRIEF DESCRIPTION OF THE DRAWNINGS

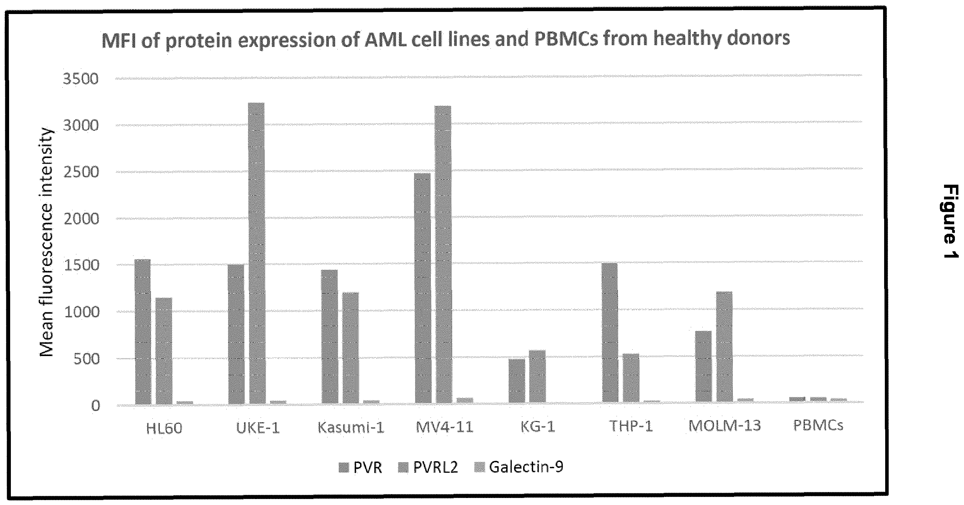

**Figure 1****: PVR, PVRL2 and Galectin-9 protein expression on different AML cell lines compared to PBMCs from healthy donors.** Protein expression was determined by FACS. All examined AML cell lines are about 100% positive for PVR and PVRL2 protein expression. In contrast, only 52% and 40% of PBMCs from healthy donors express PVR and PVRL2, respectively. Protein density (assumed by MFI) for PVR and PVRL2 is very high compared to very low density on PBMCs. Galectin-9 protein expression and density is low and comparable to PBMCs from healthy donors. Left column shows PVR expression, middle column shows PVRL2 expression, right column shows Galectin-9 expression.
**Figure 2****: Protein expression of PVR and PVRL2 by primary blasts of AML patients.** MNCs of de novo AML patients have been isolated and stained simultaneously for CD33 and PVR or PVRL2, respectively. Depicted here is the percentage share of PVR or PVRL2 positive cells inside the CD33 population. Left column shows PVRL2 expression, right column shows PVR expression.
**Figure 3****: *In vitro* assay measuring PBMC derived cytotoxicity using blocking antibodies against PVR and PVRL2 in combination with AMG330.**
**Figure 4****: PVR blocking antibodies enhance killing in cytotoxicity assays (cell line MV441).** PVR blocking antibody D171 significantly enhances killing of cells in dose dependent manner after 24h. Lower graph shows experiment 1, upper graph shows experiment 2.
**Figure 5****: Blocking of PVR leads to significant increase in cell lysis of MV4-11 cells.** PVR-Ab has a comparable effect to AMG330 alone. Additive effects could be observed for the combination of AMG330 and PVR-Ab.
**Figure 6****: PVR blocking antibodies enhance killing in cytotoxicity assays (cell line KG-1).** PVR blocking antibody D171 significantly enhances killing of cells in dose dependent manner after 24h. Lower graph shows experiment 1, upper graph shows experiment 2.
**Figure 7****: Blocking of PVR leads to significant increase in cell lysis of KG-1 cells.** PVR-Ab has comparable effect to AMG330 alone. Additive effects could be observed for the combination of AMG330 and PVR-Ab.
**Figure 8****: PVRL2 blocking antibodies enhance killing in cytotoxicity assays (cell line MV4-11).** PVRL2 antibody L-14 significantly enhances killing of cells after 24h. Lower graph shows experiment 2, upper graph shows experiment 1.
**Figure 9****: Blocking of PVRL2 leads to significant increase in cell lysis of MV4-11 cells.** Additive effects could be observed for the combination of AMG330 and PVRL2-Ab.
**Figure 10****: PVRL2 blocking antibodies enhance killing in cytotoxicity assays (cell line Kasumi-1).** PVRL2 antibody L-14 significantly enhances killing of cells after 24h. Lower graph shows experiment 1, middle graph shows experiment 3, upper graph shows experiment 2.
**Figure 11****: Blocking of PVRL2 leads to significant increase in cell lysis in Kasumi-1 cells.** PVRL2-Ab evokes similar effects on cell lysis compared to treatment with AMG330 only. Additive effects could be observed for the combination of AMG330 and PVRL2-Ab.
**Figure 12****: PVRL2 blocking antibodies enhance killing in cytotoxicity assays (cell line UKE-1).** PVRL2 antibody L-14 significantly enhances killing of the cells after 24h. Lower graph shows experiment 2, upper graph shows experiment 1.
**Figure 13****: A combination of both AMG330 and PVRL2 blocking antibody result in significant increased cytotoxicity in UKE-1.**
**Figure 14****: Blocking of Galectin-9 leads to significant increase in cell lysis of MV4-11 cells.** Additive effects could be observed for the combination of AMG330 and 9M1-3-Ab.
**Figure 15****: Blocking of Galectin-9 leads to significant increase in cell lysis of KG-1 cells.** Additive effects could be observed for the combination of AMG330 and 9M1-3-Ab.

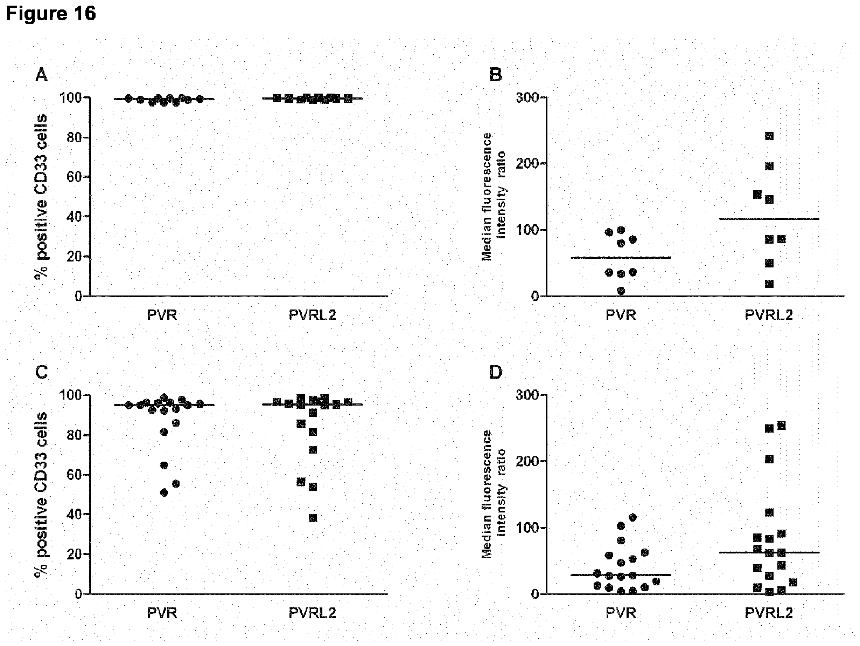

**Figure 16****: PVR and PVRL2 are highly expressed on AML cell lines and primary CD33⁺ AML blasts.** PVR and PVRL2 protein expression as depicted by percentage of positive CD33⁺ cells as well as median fluorescence intensity ratio as measure of expression intensity on AML cell lines (n=8; A,B) and CD33⁺AML blasts from untreated patients (n=17; C,D). Black dashes represent the median.

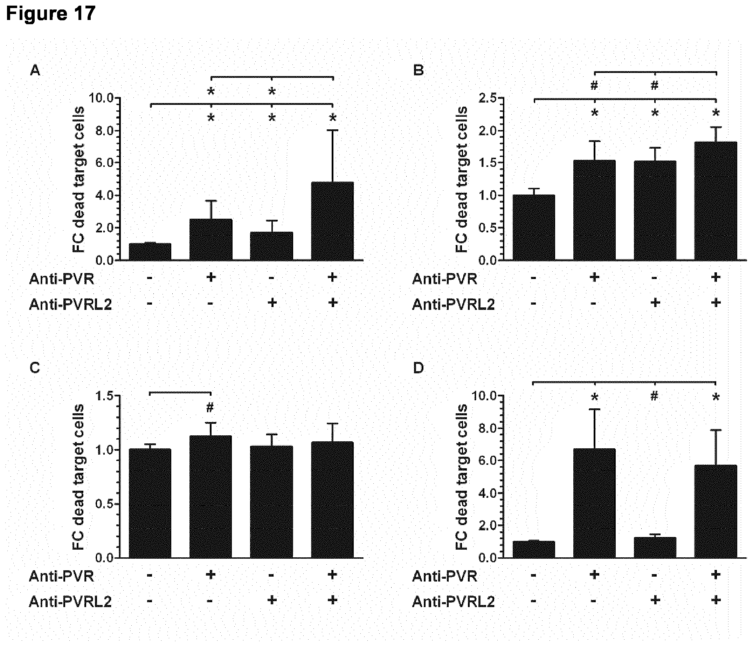

**Figure 17****: Blocking of PVR and PVRL2 increases the lysis of AML cell lines.** HD-PBMC-mediated lysis of AML cell lines MV4-11 (A), TF-1 (B), Molm-13 (C), Kasumi-1 (D) was measured after 24 h. Results are depicted as the mean ± SD of fold changes (FC) of dead target cells normalized to the control without blocking antibodies. For statistical analysis, Mann-Whitney U tests were performed (# p≤0.05; * p≤0.001; n≥3).

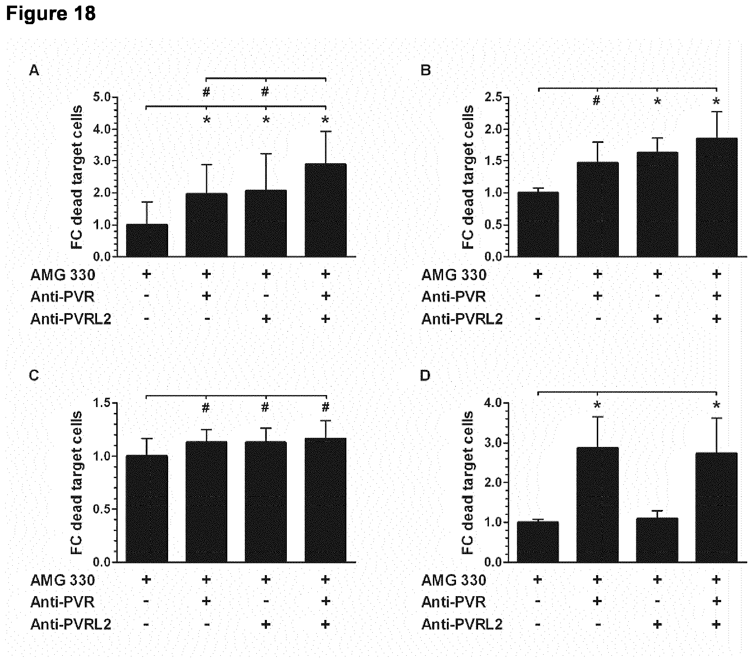

**Figure 18****: T-cell mediated lysis of the BiTE® antibody construct AMG 330 is significantly enhanced by additional administration of PVR and PVRL2 blocking antibodies.** MV4-11 (A), TF-1 (B), Molm-13 (C), Kasumi-1 (D) cells were incubated with HD-PBMCs and AMG 330 in the presence or absence of blocking antibodies against PVR or PVRL2. Results are depicted as the mean ± SD of fold changes (FC) of dead target cells normalized to the control without blocking antibodies. For statistical analysis Mann-Whitney U tests were performed (# p≤0.05; * p≤0.001; n≥3).

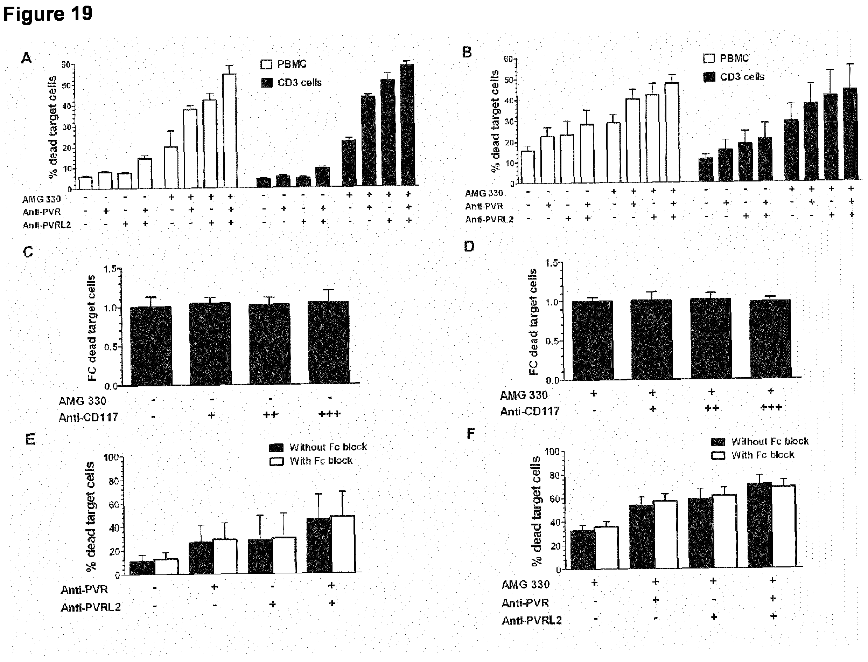

**Figure 19****: The increase of cell lysis by blocking PVR and PVRL2 is specific and not mediated via ADCC. A,** B To rule out a contribution of ADCC, the anti-leukemic effects of CD3+ T cells have been comparatively analyzed to the PBMCs from the same healthy donor with our without additional administration of AMG 330 using the cell lines MV4-11 (A) and TF-1 (B). Results are depicted as the mean ± SD of dead target cells (n=2). C, D Kasumi-1 cells were incubated with escalating doses of an antibody targeting CD117 in the presence or absence of AMG 330 (n=2, + 2µg/mL, ++ 10 µg/mL, +++ 50 µg/mL). Results are depicted as the mean ± SD of fold changes (FC) of dead target cells normalized to the control. E, F Fcγ receptors on HD-PBMCs were saturated with polyclonal human IgGs and compared with unsaturated HD-PBMCs, both used as effector cells against MV4-11 cells. Results are depicted as the mean ± SD of dead target cells (n=3).

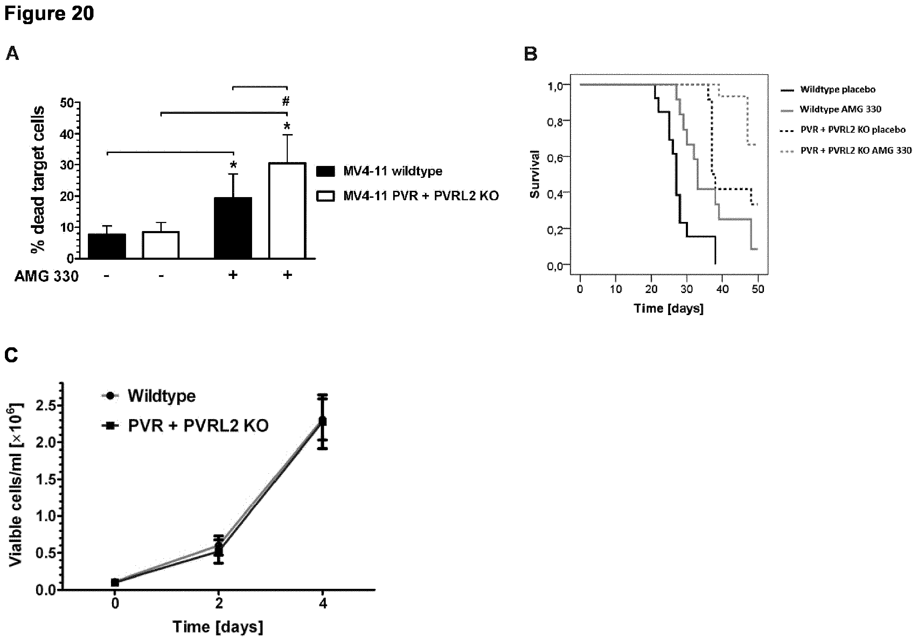

**Figure 20****: PVR and PVRL2 double knockout cells recapitulate antibody effects in vitro and prolong the survival of NSG mice reconstituted with human T cells in vivo.** A By using CRISPR/Cas9, a polyclonal population of MV4-11 harboring a double knockout of PVR and PVRL2 was generated. Either MV4-11 wildtype or double knockout cells were incubated with HD-PBMCs for 24 h without or with AMG 330. For statistical analysis Mann-Whitney U tests were performed (# p≤0.05; * p≤0.001, n=3). B Immunodeficient NSG mice were transplanted with either MV4-11 wildtype (WT) or PVR and PVRL2 double knockout (KO) cells and reconstituted with human T cells. Treatment consisted of daily intraperitoneal application of either placebo (n=13 for WT and n=12 for KO) or 15 µg/kg AMG 330 (n=12 for WT and n=15 for KO). Log-rank tests were performed: WT placebo vs. KO placebo p<0.001; WT AMG 330 vs. KO AMG 330 p<0.001; WT placebo vs. WT AMG 330 p=0.003; KO placebo vs. KO AMG 330 p=0.027. C Proliferation capacity of CRISPR/Cas9-generated knockout cells. The growth rate of MV4-11 PVR and PVRL2 double knockout cells was compared to the proliferation capacity of MV4-11 wildtype cells. Cell counts were measured on day 2 and 4 using the Vi-Cell™XR automatic cell counter (Beckman Coulter); n=3.

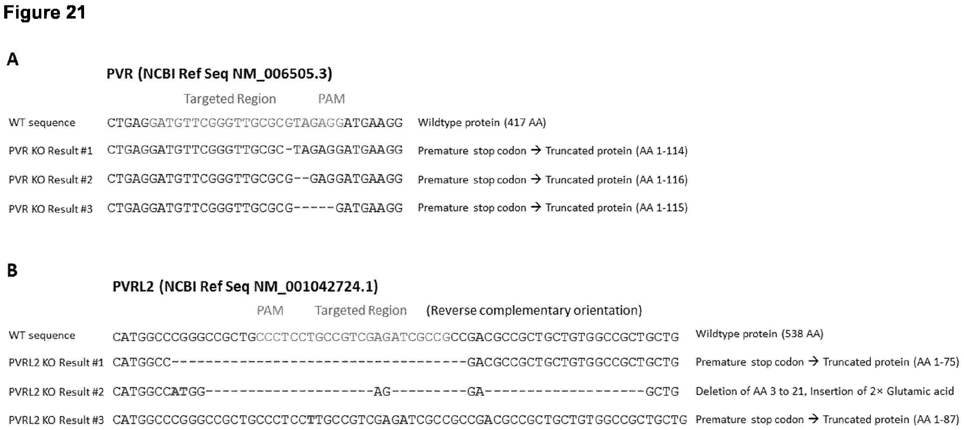

**Figure 21****: Genomic analysis of CRISPR/Cas9-mediated PVR and PVRL2 double knockout cells.** To validate the CRISPR/Cas9-mediated knockout of PVR and PVRL2 in MV4-11 cells on the genomic level, the corresponding gene sections of several single cells were analyzed by subcloning and sequencing. The genomic alterations for three different knockout clones including the impact on the protein sequence are presented for PVR (A) and PVRL2 (B), respectively. The wildtype sequence with the target sites in blue and the PAM sequence in green is shown at the top. For PVRL2, the targeted region and PAM sequence are in reverse complementary orientation as the PVRL2 guide RNA recognized the antiparallel DNA strand. Deletions within the subclones are shown as red dashes and insertions are shown in red. WT = wildtype, KO = double knockout, PAM = protospacer adjacent motif, AA = amino acid.

### DETAILED DESCRIPTION

The following description includes information that may be useful in understanding the presentation. It is not an admission that any of the information provided herein is prior art or relevant to the presently claimed inventions, or that any publication specifically or implicitly referenced is prior art.

The present invention is at least partly based on the surprising finding that immune-checkpoint inhibitors against CD112 (Nectin-2, PVRL2), CD155 (PVR), and/or TIGIT can be efficiently used for the treatment of blood-borne cancers, in particular acute myeloid leukemia (AML) having immune escape mechanisms, thereby providing a new and very efficient immunotherapeutic approach in cancer therapy. In this regard the inventors discovered in *in vitro* studies with various leukemic cell lines that the immune-checkpoint ligands CD112 and CD155 and their receptor TIGIT, the immune-checkpoint ligand Galectin-9 and its receptor TIM-3 are well suited targets for use in the treatment of blood-borne cancers, in particular AML. CD112 are CD155 immune-checkpoint protein ligands, while their receptor is TIGIT. As an aspect of the disclosure, Galectin-9 is also a ligand and TIM-3 its receptor.

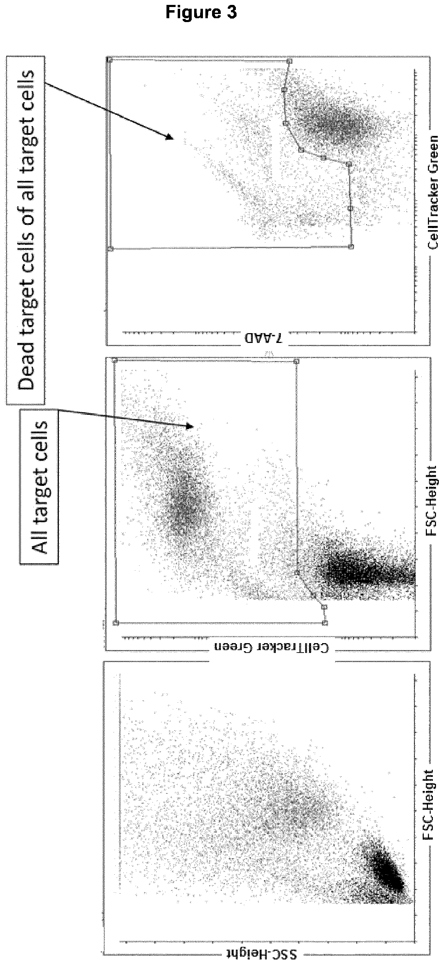

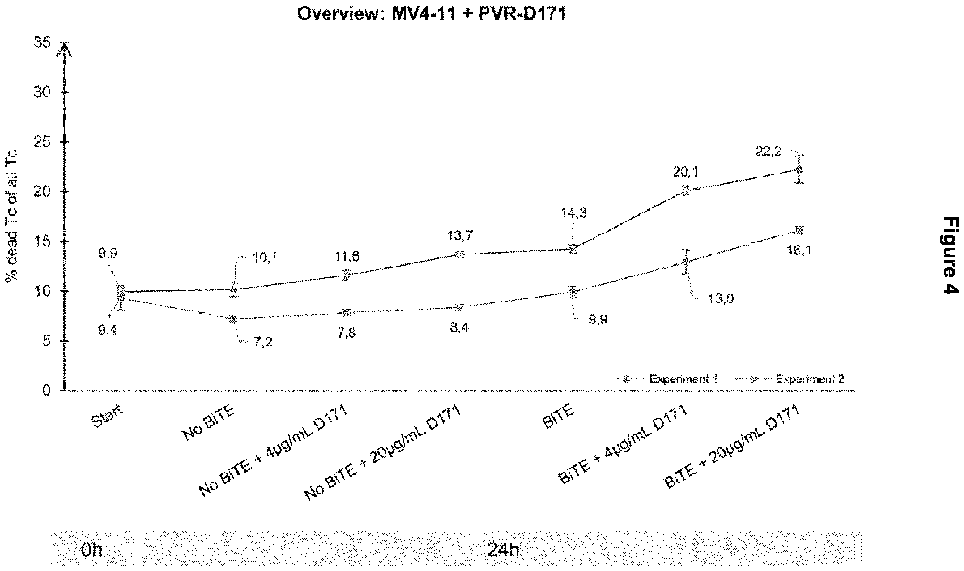

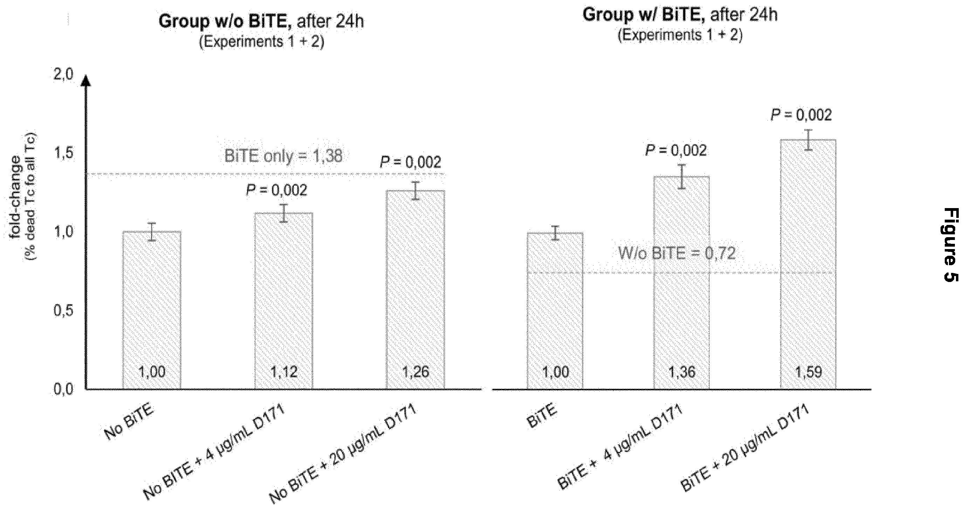

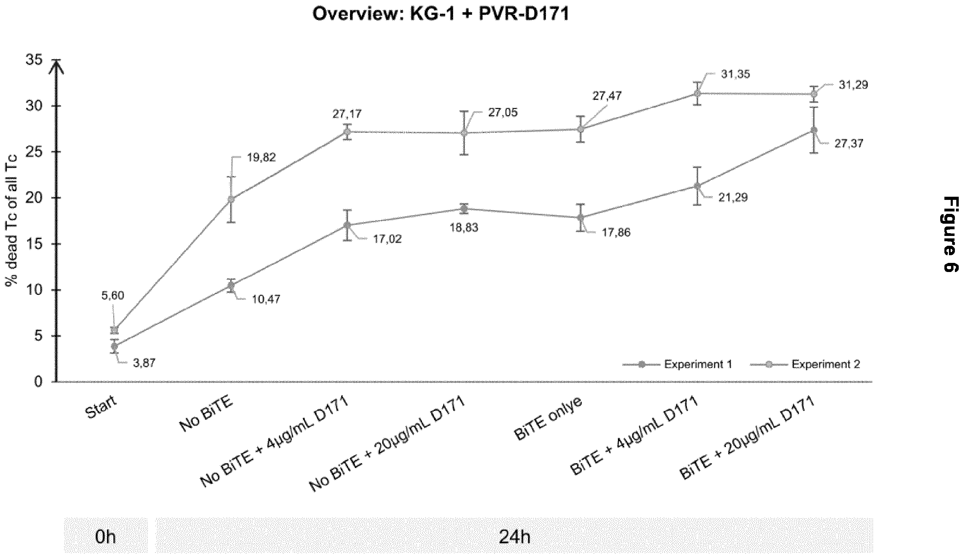

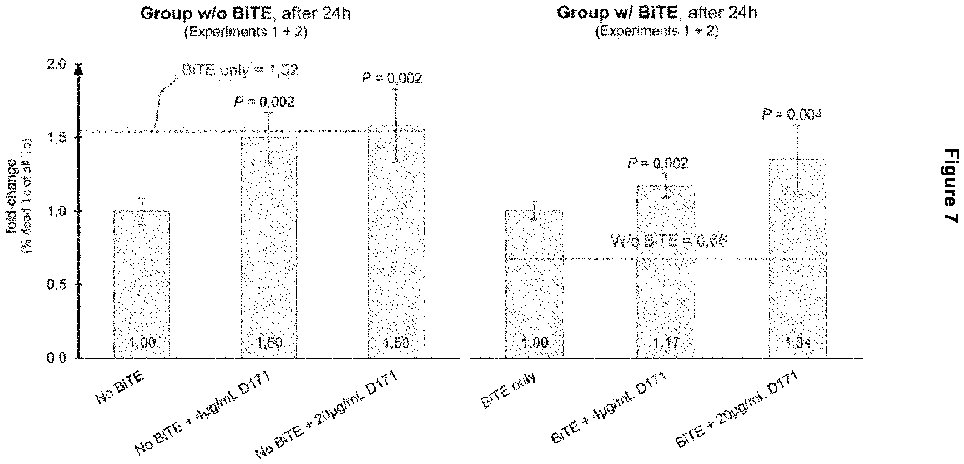

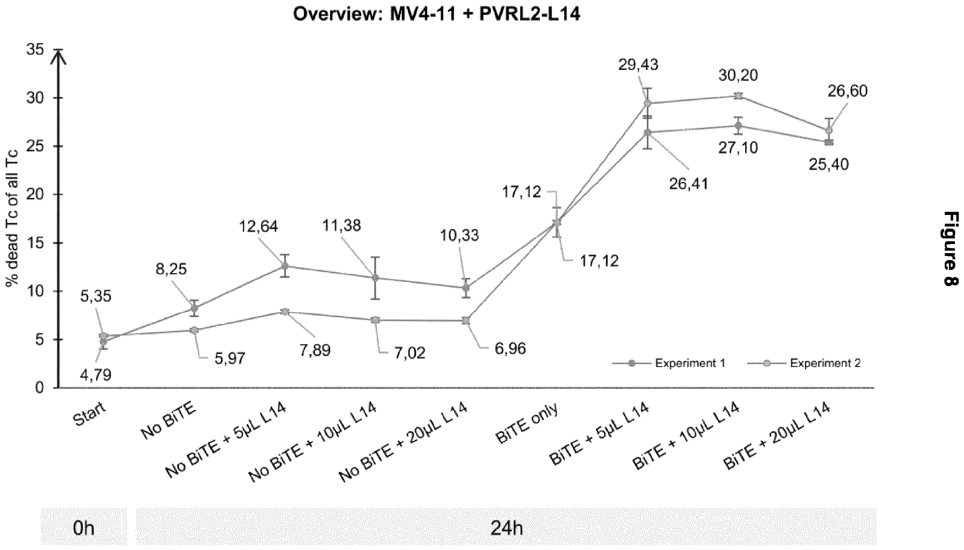

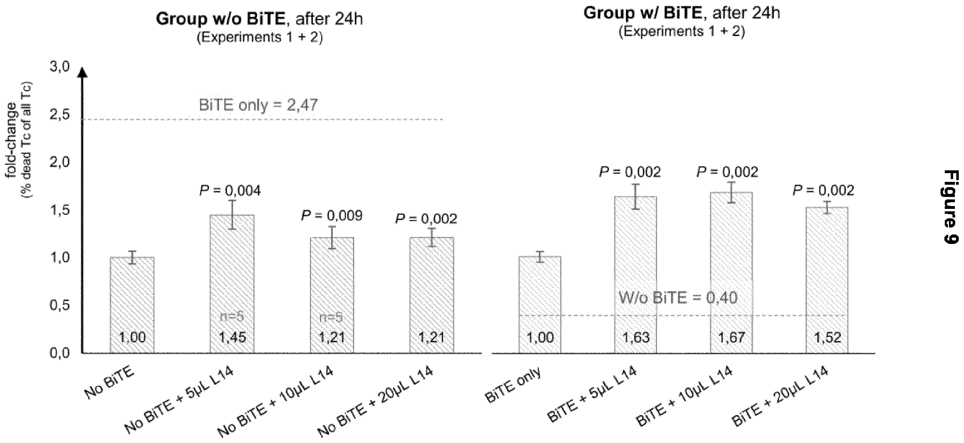

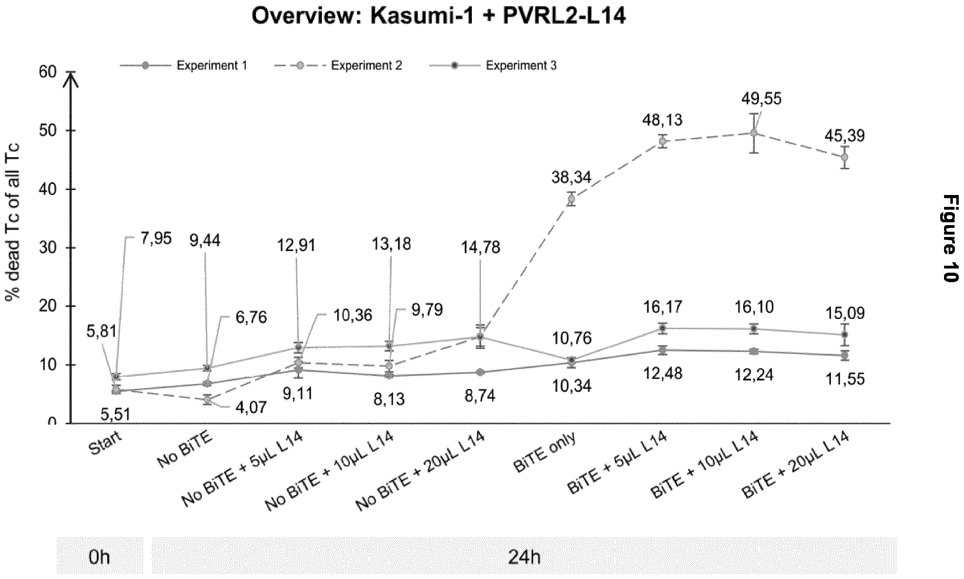

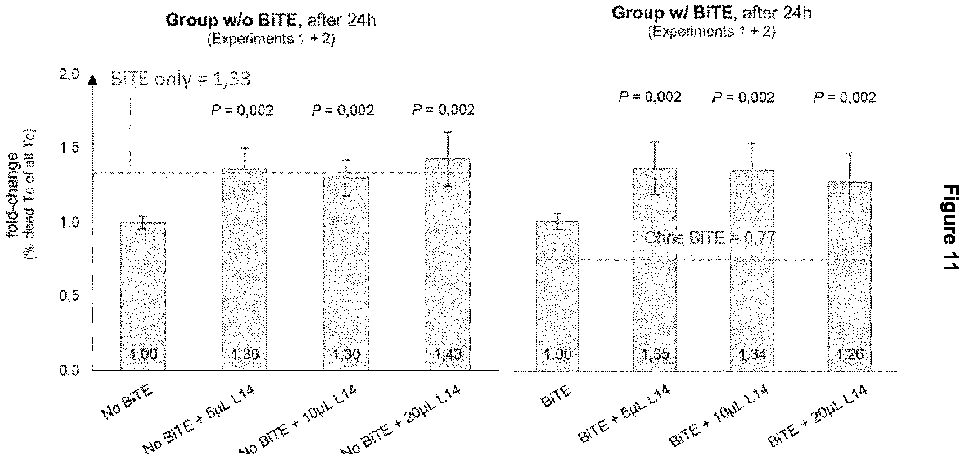

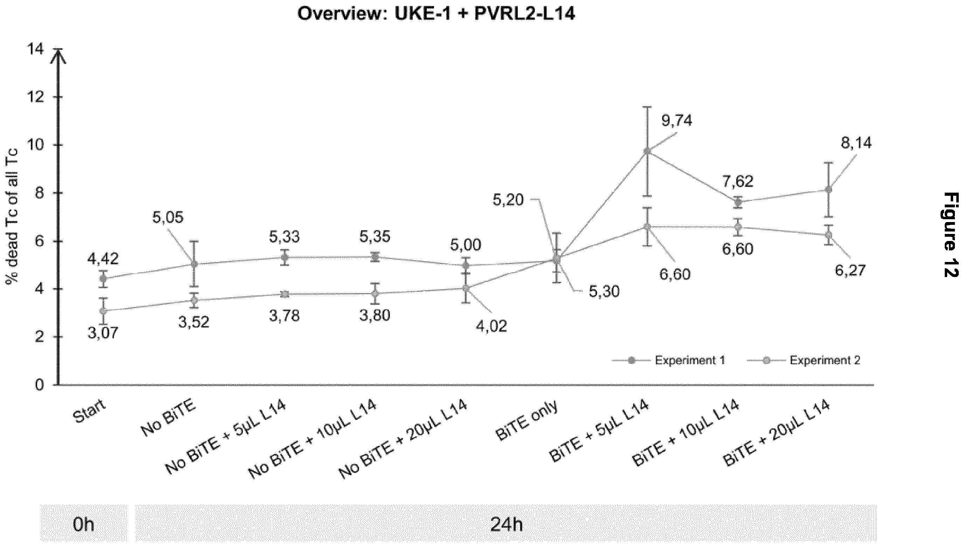

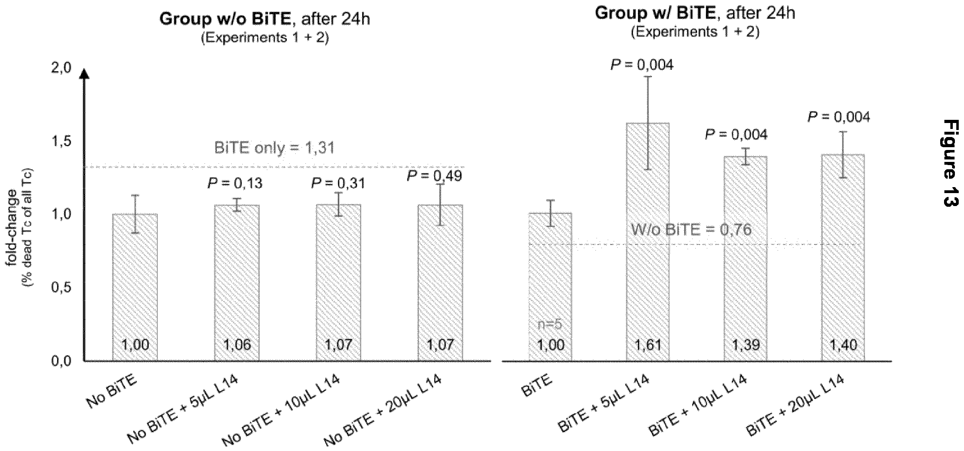

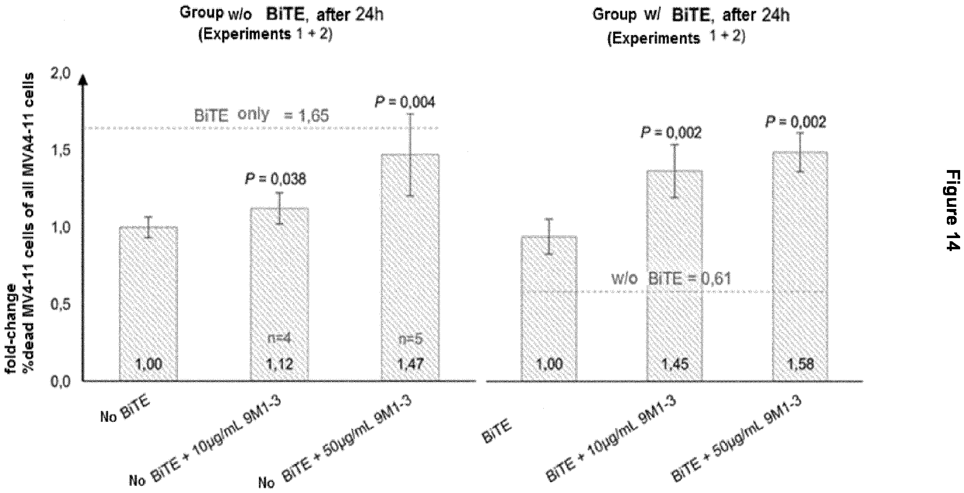

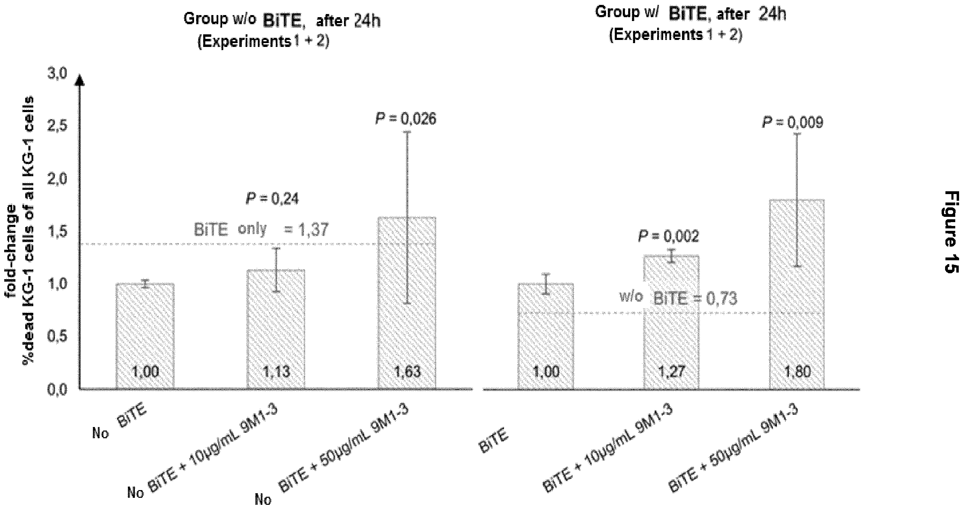

It was found by the present inventors that the immune-checkpoint ligands PVR and PVRL2 and its receptor TIGIT seem to have negative prognostic impact on the overall survival in AML patients (data not shown). Moreover, the present inventors revealed that blockage of PVL, PVRL2 or TIGIT leads to a significant killing of AML cells **(****Figures 3-13****).** Accordingly, the present inventors paid attention to PVR, PVRL2 and TIGIT as a new targets for use in blood-born cancer treatment, particularly AML treatment and provide substances that efficiently inhibit the immunoinhibitory signal of PVR, PVRL2 and/or TIGIT, thereby inhibiting cancer proliferation through the mechanism of the recovery and activation of immune function of T cells. Further, there are contradictory data in the art describing positive as well as negative prognostic impact of the immune-checkpoint ligand Galectin-9 and its receptor TIM-3 on T cell activity and tumor development. Although expression studies seem to underline that Galectin-9 and TIM-3 have a rather positive prognostic impact on cancer patients (data not shown), as an aspect of the disclosure it is revealed that blockage of Galectin-9 and TIM-3 leads to a significant killing of AML cells (**see** **Figures 14** **and** **15**)**.**

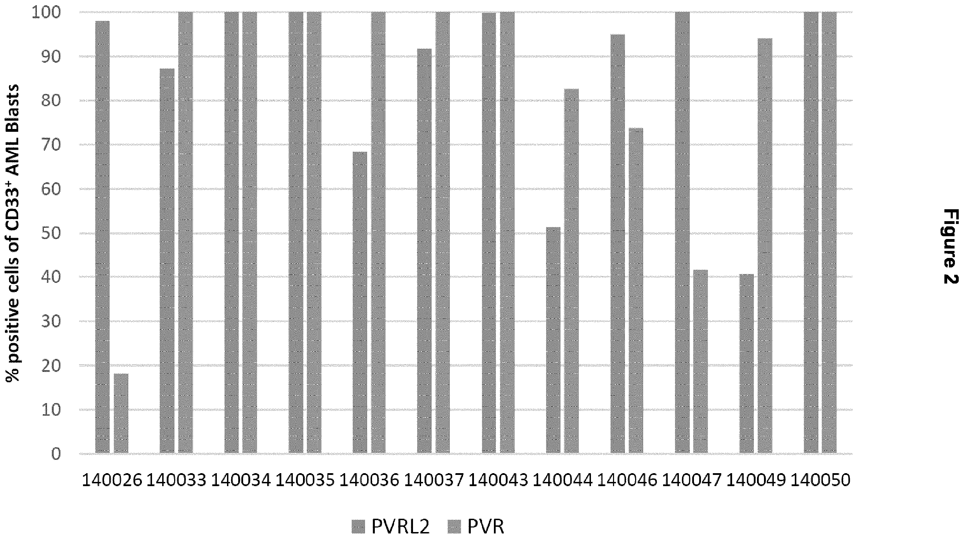

As shown by the present inventors, all examined AML cell lines are about 100% positive for PVR and PVRL2 protein expression. In contrast, only 52% and 40% of PBMCs from healthy donors express PVR and PVRL2, respectively (**Figure 1**)**.** Moreover, protein density for PVR and PVRL2 is very high compared to very low density on PBMCs. Further, Galectin-9 protein expression and density is low and comparable to PBMCs from healthy donors. Also primary blasts of AML patients show protein expression of PVR and PVRL2. Thereby, MNCs of de novo AML patients have been isolated and stained simultaneously for CD33 and PVR or PVRL2, respectively. Here a percentage share of PVR or PVRL2 positive cells inside the CD33 population could be found (**Figure 2**).

As disclosed herein, PVR and PVRL2 interaction with its receptor TIGIT as well as - as an aspect of the disclosure - Galectin-9 interaction with its receptor TIM-3 act as an immunosuppressive or even immunoinhibitory signal and thus functions as an immune escape mechanism in AML. In accordance with the foregoing, it is intended that the immune-checkpoint inhibitors against CD112, CD155, and/or TIGIT, should inhibit or make difficult the interaction between CD112 and TIGIT, CD155 and TIGIT, thereby foster cancer specific immune response. Likewise, it is intended that the immune-checkpoint inhibitors against CD155 inhibit the interaction between CD155 and TIGIT or make the interaction between CD155 and TIGIT more difficult. It is also intended that the immune-checkpoint inhibitors against TIGIT inhibit the interaction between TIGIT and CD112 and/or CD155 or make the interaction between TIGIT and CD112 and/or CD155 more difficult. Moreover, as an aspect of the disclosure it is intended that the immune-checkpoint inhibitors against Galectin-9 inhibit the interaction between Galectin-9 and TIM-3 or make the interaction between Galectin-9 and TIM-3 more difficult. Likewise, it is intended as an aspect of the disclosure that the immune-checkpoint inhibitors against TIM-3 inhibit the interaction between TIM-3 and Galectin-9 or make the interaction between TIM-3 and Galectin-9 more difficult.

As used herein, the term "inhibit" or "inhibiting" refers to the ability of the inhibitors to block, partially block, interfere, decrease, suppress, reduce or deactivate a target protein, i.e. the immune-checkpoint ligands CD112 and CD155 and/or its receptor TIGIT, the immune-checkpoint ligand Galectin-9 and/or its receptor TIM-3. Thus, one of skill in the art understands that the term "inhibit" may encompass a complete and/or partial loss of activity of said ligand or receptor. The activity of said ligand or receptor may be suppressed or inhibited by a compound binding to the active site of the ligand/receptor protein, or by other means, such as disabling a second protein that activates the inhibited first protein. For example, a complete and/or partial inhibition of the interaction between CD112 and TIGIT, CD155 and TIGIT as well as Galectin-9 and TIM-3 may be indicated by a significantly increase cell lysis, i.e. a significantly increased dead cell rate of blood-borne cancer target cells, in particular AML target cells.

The term "blood-borne cancer" as used herein includes particularly leukemia and lymphoma, e.g. Hodgkin lymphoma or Non-Hodgkin lymphoma. It also includes Myelodysplastic Syndrome (MDS) and Multiple Myeloma (MM).

Immune-checkpoint inhibitors of the present application can, apart from being for use in the treatment of blood-borne cancer, also be for use in the treatment of solid tumors, such as oral cancer or pancreatic cancer (Thijssen et al. (2015), Biochim Biophys 1855, 235-247).

"Acute myeloid leukemia", also called acute myelocytic leukemia, acute myelogenous leukemia, acute granulocytic leukemia, acute non-lymphocytic leukemia, or just "AML" generally refers to an acute form of leukemia which is typically characterized by the overproduction and/or accumulation of cancerous, immature myeloblasts, red blood cells, or platelets in the bone marrow. "Acute" means that this leukemia can progress quickly if not treated, and would probably be fatal in a few months. "Myeloid" refers to the type of cell this leukemia starts from. Most cases of AML develop from cells that would turn into white blood cells (other than lymphocytes), however there are different subtypes of AML. AML starts in the bone marrow, but in most cases it quickly moves into the blood. As used herein, the term "AML" includes acute, refractory and relapsed AML. The term "refractory AML" as used herein means resistance of the AML to conventional or standard AML therapy, such as chemotherapy and/or hematopoietic stem cell transplantation (HSCT), i.e. the conventional or standard AML therapy is not able to ultimately cure all AML patients. The term "relapsed AML" as used herein denotes the return of signs and symptoms of the AML disease after a patient has enjoyed a remission. For example, after conventional AML treatment using chemotherapy and/or HSCT, a AML patient may go into remission with no sign or symptom of the AML, remains in remission for a couple of years , but the suffers a relapse and has to be treated once again for AML. The term "AML" as used herein also includes minimal residual disease (MRD) in a patient with AML, i.e. the presence of small numbers of cancerous myeloid cells remaining in the patient during treatment, or after treatment when the patient is in remission.

The term "immune-checkpoint inhibitor" when used herein refers to any binding agent or compound suitable to act against the immune-checkpoint proteins CD112, CD155, TIGIT, Galectin-9 and/or TIM-3, thereby inhibiting or suppressing the immunoinhibitory signal between CD112 and TIGIT, CD155 and TIGIT and/or Galectin-9 and TIM-3 interaction. The term "immunoinhibitory signal" when used herein refers to the interaction between the immune-checkpoint ligands and receptors, thereby reducing the immunoactivity of the involved T cell against the involved tumor cell and allowing the tumor cell to escape from the immune-defense mechanisms of the organism. The inhibitors used in the present invention directed against CD112, CD155, and/or TIGIT therefore inhibit this immunoinhibitory signal between the immune-checkpoint ligands and receptors, thereby allowing the T cells to attack and eliminate the involved tumor cell. Accordingly, the inhibitors used in the present invention are capable of decreasing or inhibiting the immunoinhibitory signal between the immune-checkpoint ligand and its receptor. Accordingly, the inhibitors used in the present invention exhibit "immune-potentiating" activity by activating T cell response towards cancer cells. In particular, the inhibitor having immune-potentiating activity is capable of decreasing or inhibiting the intensity of the immunoinhibitory signal between CD112 and TIGIT. It is further envisaged that the inhibitor having immune-potentiating activity is capable of decreasing or inhibiting the intensity of the immunoinhibitory signal between CD155 and TIGIT. It is further envisaged as an aspect of the disclosure that the inhibitor having immune-potentiating activity is capable of decreasing or inhibiting the intensity of the immunoinhibitory signal between Galectin-9 and TIM-3.

According to the present invention, the binding of the immune-checkpoint inhibitor against the respective immune-checkpoint target protein modulates the intracellular signaling of said target protein. Thus, it is envisaged that the inhibitor against CD112 modulates intracellular signaling of CD112. Likewise, it is envisaged that the inhibitor against CD155 modulates intracellular signaling of CD155. Likewise it is envisaged that the inhibitor against TIGIT modulates intracellular signaling of TIGIT. Likewise it is an aspect of the disclosure that the inhibitor against Galectin-9 modulates intracellular signaling of Galectin-9. Likewise it is an aspect of the disclosure that the inhibitor against TIM-3 modulates intracellular signaling of TIM-3. The term "modulate" or "modulating" when used herein includes increasing, decreasing, or otherwise changing the intracellular signal of the immune-checkpoint proteins, i.e. CD112, CD155, and/or TIGIT. In this regard the intracellular signal pathway coupled to this immune-checkpoint proteins can be enhanced, impeded or completely prevented, thereby changing the level, amount, and/or activity of downstream signaling elements, comprising cell activation, proliferation and malignant growth.

As disclosed herein, the inhibitors against CD112, CD155, and/or TIGIT, can be used to treat blood-born cancer, in particular acute myeloid leukemia (AML). Blood-borne cancers are cancers of the blood cells that start in the bone marrow, typically comprising leukemia and lymphoma. In the case of a blood-borne cancer, the bone marrow begins to make abnormal cells that crowd out the normal blood cells. Likewise, the inhibitors as disclosed herein are particularly useful to treat any kind of blood-borne cancer characterized by an increased expression of CD112, and/or CD155 on the tumor cells. In particular, the inhibitors disclosed herein are especially useful for treating any kind of blood-borne cancer characterized by an Accordingly, it is envisaged that the inhibitors may also be for use in the treatment of other blood-borne cancers characterized by an increased expression of CD112, and/or CD155 on the tumor cells, such as chronic myeloid leukemia (CML), acute lymphoblastic leukemia (ALL), myelodysplastic syndrome (MS or myelodysplasia), and myeloproliferative neoplasms (MPN). It is further envisaged that the inhibitors can be used for treating solid tumors characterized by an increased expression of CD112, CD155, and/or Galectin-9. In this regard, the inhibitors disclosed herein can be used to interact between the immune-checkpoint ligands CD112, and/or CD155 expressed on the tumor cell and their immune-checkpoint receptor TIGIT expressed on the T cell as described elsewhere herein.

As demonstrated in the present invention by FACS analysis and *in vitro* cytotoxicity assay test for various leukemic cell lines, antibodies against PVR and PVRL2 are well suited for blocking these immune-checkpoint ligands, thereby significantly increasing the cell lysis of AML cells (**Figures 3-13**). Accordingly, the inhibitor used in the present invention is an antibody construct. The term "antibody construct" in the sense of the present disclosure indicates antibody-based "binding molecule" or "binding agent" which is capable of (specifically) binding to, interacting with or recognizing the immune-checkpoint molecules of the present invention. The antibody construct can bind/interact with the surface molecule on an AML cancer target cell or a receptor complex on a T cell. A preferred binding molecule is an antibody.

The term "antibody" refers to a molecule in which the structure and/or function is/are based on the structure and/or function of an antibody, e.g. of a full-length or whole immunoglobulin molecule. According to the present invention, the antibody for use in the treatment of a blood-borne cancer, in particular AML is an inhibitory antibody which specifically inhibits the interaction between the immune-checkpoint ligand and the immune checkpoint receptor as defined in the claims. In this regard it is envisaged that the antibody disclosed herein specifically inhibits the interaction between CD112 and TIGIT. It is also envisaged that the antibody disclosed herein specifically inhibits the interaction between CD155 and TIGIT. In particular, when inhibiting the interaction between the immune-checkpoint ligand and the immune checkpoint receptor, the antibody inhibits the immunoinhibitory interaction between CD112/TIGIT, and/or CD55/TIGI , thereby inhibiting the signal of the ligand-receptor interaction. Thus, the antibody disclosed herein binds itself to the immune-checkpoint ligands or receptors and makes it difficult, if not impossible, to obtain an immunoinhibitory signal between said ligands and receptors. In accordance with the foregoing, exemplary antibodies useful in the uses of the present invention included anti-CD112 antibodies, anti-CD112 antibodies, and anti-TIGIT antibodies. The skilled artisan is aware of a huge number of various inhibitory antibodies that can be used according to the present invention, thereby inhibiting the interaction between the immune-checkpoint ligands and receptors as disclosed elsewhere herein.

The definition of the term "antibody" includes embodiments such as monoclonal, chimeric, single chain, humanized and human antibodies, as well as antibody fragments, like, inter alia, Fab fragments. Antibody fragments or derivatives further comprise F(ab')₂, Fv, scFv fragments or single domain antibodies such as domain antibodies or nanobodies, single variable domain antibodies or immunoglobulin single variable domain comprising merely one variable domain, which might be VHH, VH or VL, that specifically bind an antigen or epitope independently of other V regions or domains; see, for example, Harlow and Lane (1988) and (1999), loc. cit.; Kontermann and Dübel, Antibody Engineering, Springer, 2nd ed. 2010 and Little, Recombinant Antibodies for Immunotherapy, Cambridge University Press 2009. Such immunoglobulin single variable domain encompasses not only an isolated antibody single variable domain polypeptide, but also larger polypeptides that comprise one or more monomers of an antibody single variable domain polypeptide sequence. Monovalent antibody fragments in line with the above definition describe an embodiment of a binding domain in connection with this invention. Such monovalent antibody fragments bind to a specific antigen and can be also designated "antigen-binding domain", "antigen-binding fragment" or "antibody binding region".

In line with this definition provided herein, the term antibody can be subsumed under the term "antibody construct". Said term also includes diabodies or Dual-Affinity Re-Targeting (DART) antibodies. Further envisaged are (bispecific) single chain diabodies, tandem diabodies (Tandab's), "minibodies" exemplified by a structure which is as follows: (VH-VL-CH3)₂, (scFv-CH3)₂ or (scFv-CH3-scFv)₂, "Fc DART" antibodies and "IgG DART" antibodies, and multibodies such as triabodies. Immunoglobulin single variable domains encompass not only an isolated antibody single variable domain polypeptide, but also larger polypeptides that comprise one or more monomers of an antibody single variable domain polypeptide sequence.

Various procedures are known in the art and may be used for the production of such antibody constructs (antibodies and/or fragments). Thus, (antibody) derivatives can be produced by peptidomimetics. Further, techniques described for the production of single chain antibodies (see, inter alia, US Patent 4,946,778, Kontermann and Dübel (2010), loc. cit. and Little(2009), loc. cit.) can be adapted to produce single chain antibodies specific for elected polypeptide(s). Also, transgenic animals may be used to express humanized antibodies specific for polypeptides and fusion proteins of this invention. For the preparation of monoclonal antibodies, any technique, providing antibodies produced by continuous cell line cultures can be used. Examples for such techniques include the hybridoma technique (Kohler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a target polypeptide, such as CD3 epsilon (Schier, Human Antibodies Hybridomas 7 (1996), 97-105; Malmborg, J. Immunol. Methods 183 (1995), 7-13). It is also envisaged in the context of this invention that the term "antibody" comprises antibody constructs, which may be expressed in a host as described herein below, e.g. antibody constructs which may be transfected and/or transduced via, inter alia, viruses or plasmid vectors.

Furthermore, the term "antibody" as employed herein also relates to derivatives or variants of the antibodies described herein which display the same specificity as the described antibodies. Examples of "antibody variants" include humanized variants of non-human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function(s) (see, e.g., US Patent 5, 648, 260, Kontermann and Dübel (2010), loc. cit. and Little(2009), loc. cit.).

The terms "antigen-binding domain", "antigen-binding fragment" and "antibody binding region" when used herein refer to a part of an antibody molecule that comprises amino acids responsible for the specific binding between antibody and antigen. The part of the antigen that is specifically recognized and bound by the antibody is referred to as the "epitope" as described herein above. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of antigen-binding fragments of an antibody include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) a Fd fragment having the two VH and CH1 domains; (4) a Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv). Although the two domains of the Fv fragment, VL and VH are coded for by separate genes, they can be joined, using recombinant methods, by a synthetic linker that enables them to be made as a single protein chain in which the VL and VH regions pair to form monovalent molecules (known as single chain Fv (scFv); see e.g., Huston et al. (1988) Proc. Natl. Acad. Sci USA 85:5879-5883). These antibody fragments are obtained using conventional techniques known to those with skill in the art, and the fragments are evaluated for function in the same manner as are intact antibodies.

In the event that a (synthetic) linker is used, this linker is preferably of a length and sequence sufficient to ensure that each of the first and second domains can, independently from one another, retain their differential binding specificities. Most preferably and as documented in the appended examples, the antibody construct of the invention is a "bispecific single chain antibody construct", more preferably a bispecific single chain Fv (scFv). Bispecific single chain molecules are known in the art and are described in WO 99/54440, Mack, J. Immunol. (1997), 158, 3965-3970, Mack, PNAS, (1995), 92, 7021-7025, Kufer, Cancer Immunol. Immunother., (1997), 45, 193-197, Loffler, Blood, (2000), 95, 6, 2098-2103, Brühl, Immunol., (2001), 166, 2420-2426, Kipriyanov, J. Mol. Biol., (1999), 293, 41-56. One example of a CD33 targeting compound in connection with the present invention, which is a bispecific single chain molecule is AMG330, which has also been used in the appended examples. The sequence of AMG330 was initially described in WO 2008/119567 The said variable domains comprised in the herein described antibody constructs may be connected by additional linker sequences. The term "peptide linker" defines in accordance with the present invention an amino acid sequence by which the amino acid sequences of the first domain and the second domain of the antibody construct of the invention are linked with each other. An essential technical feature of such peptide linker is that said peptide linker does not comprise any polymerization activity. Preferred amino acid residues for a peptide linker include Gly, Ser and Thr are characterized by a length between 5 and 25 amino acid residues. Among the suitable peptide linkers are those described in U.S. Patents 4,751,180 and 4,935,233 or WO 88/09344. A preferred embodiment of a peptide linker is characterized by the amino acid sequence Gly-Gly-Gly-Gly-Ser, i.e. Gly₄Ser (SEQ ID No: 9), or polymers thereof, i.e. (Gly₄Ser)x, where x is an integer 1 or greater (e.g. 2 or 3). Also preferred are variations of this linker sequence which includes examples such as (Gly-Gly-Gly-Gly)ₓ (SEQ ID No: 10), (Gly-Gly-Gly-Gly-Gln)ₓ (SEQ ID No: 11), (Pro-Gly-Gly-Gly-Gly-Ser)ₓ (SEQ ID No: 12), (Pro-Gly-Gly-Asp-Gly-Ser)ₓ (SEQ ID No: 13) and (Ser-Gly-Gly-Gly-Gly-Ser)ₓ (SEQ ID No: 14) The characteristics of said peptide linker, which comprise the absence of the promotion of secondary structures are known in the art and described e.g. in Dall'Acqua et al. (Biochem. (1998) 37, 9266-9273), Cheadle et al. (Mol Immunol (1992) 29, 21-30) and Raag and Whitlow (FASEB (1995) 9(1), 73-80). Peptide linkers which also do not promote any secondary structures are preferred. The linkage of said domains to each other can be provided by, e.g. genetic engineering, as described in the examples. Methods for preparing fused and operatively linked bispecific single chain constructs and expressing them in mammalian cells or bacteria are well-known in the art (e.g. WO 99/54440 or Sambrook et al., Molecular Cloning: A Laboratory Manual, Cold Spring Harbor Laboratory Press, Cold Spring Harbor, New York, 2001).

For peptide linkers, which connect the at least two binding domains in the antibody construct of the invention peptide linkers are preferred which comprise only a few number of amino acid residues, e.g. 12 amino acid residues or less. Thus, peptide linker of 12, 11, 10, 9, 8, 7, 6 or 5 amino acid residues are preferred. An envisaged peptide linker with less than 5 amino acids comprises 4, 3, 2 or one amino acid(s) wherein Gly-rich linkers are preferred. A particularly preferred "single" amino acid in context of said "peptide linker" is Gly. Accordingly, said peptide linker may consist of the single amino acid Gly.

The term "monoclonal antibody" as used herein refers to an antibody obtained from a population of substantially homogeneous antibodies, i.e., the individual antibodies comprising the population are identical except for possible naturally occurring mutations and/or post- translation modifications (e.g., isomerizations, amidations) that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, e.g., U. S. Patent No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The term "human antibody" includes antibodies having variable and constant regions corresponding substantially to human germline immunoglobulin sequences known in the art, including, for example, those described by Kabat et al. (See Kabat et al. (1991) loc. cit.). The human antibodies used in the invention may include amino acid residues not encoded by human germline immunoglobulin sequences (e.g., mutations introduced by random or site-specific mutagenesis in vitro or by somatic mutation in vivo), for example in the CDRs, and in particular, CDR3. The human antibody can have at least one, two, three, four, five, or more positions replaced with an amino acid residue that is not encoded by the human germline immunoglobulin sequence. It is emphasized that the definition of human antibodies as used herein also contemplates fully human antibodies, which include only non-artificially and/or genetically altered human sequences of antibodies as those can be derived by using technologies using systems such as the Xenomice.

Examples of "antibody variants" include humanized variants of non- human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function (s) (see, e.g., US Patent 5, 648, 260, Kontermann and Dübel (2010), loc. cit. and Little(2009), loc. cit.). As used herein, "in vitro generated antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell. The pairing of a VH and VL together forms a single antigen-binding site. The CH domain most proximal to VH is designated as CH1. Each L chain is linked to an H chain by one covalent disulfide bond, while the two H chains are linked to each other by one or more disulfide bonds depending on the H chain isotype. The VH and VL domains consist of four regions of relatively conserved sequences called framework regions (FR1, FR2, FR3, and FR4), which form a scaffold for three regions of hypervariable sequences (complementarity determining regions, CDRs). The CDRs contain most of the residues responsible for specific interactions of the antibody with the antigen. CDRs are referred to as CDR 1, CDR2, and CDR3. Accordingly, CDR constituents on the heavy chain are referred to as H1, H2, and H3, while CDR constituents on the light chain are referred to as L1, L2, and L3.

The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in sub-domains of each of the heavy and light chain variable regions. These sub-domains are called "hypervariable" regions or "complementarity determining regions" (CDRs). The more conserved (i.e., non-hypervariable) portions of the variable domains are called the "framework" regions (FRM). The variable domains of naturally occurring heavy and light chains each comprise four FRM regions, largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding site (see Kabat *et al.,* loc. cit.). The constant domains are not directly involved in antigen binding, but exhibit various effector functions, such as, for example, antibody-dependent, cell-mediated cytotoxicity and complement activation.

The terms "CDR", and its plural "CDRs", refer to a complementarity determining region (CDR) of which three make up the binding character of a light chain variable region (CDRL1, CDRL2 and CDRL3) and three make up the binding character of a heavy chain variable region (CDRH1, CDRH2 and CDRH3). CDRs contribute to the functional activity of an antibody molecule and are separated by amino acid sequences that comprise scaffolding or framework regions. The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat, Chothia, and/or MacCallum (Kabat *et al.,* loc. cit.; Chothia et al., J. Mol. Biol, 1987, 196: 901; and MacCallum et al., J. Mol. Biol, 1996, 262: 732). However, the numbering in accordance with the so-called Kabat system is preferred. The CDR3 of the light chain and, particularly, CDR3 of the heavy chain may constitute the most important determinants in antigen binding within the light and heavy chain variable regions. In some antibody constructs, the heavy chain CDR3 appears to constitute the major area of contact between the antigen and the antibody. In vitro selection schemes in which CDR3 alone is varied can be used to vary the binding properties of an antibody or determine which residues contribute to the binding of an antigen.

In some embodiments, the binding molecules described herein are isolated proteins or substantially pure proteins. An "isolated" protein is unaccompanied by at least some of the material with which it is normally associated in its natural state, for example constituting at least about 5%, or at least about 50% by weight of the total protein in a given sample. It is understood that the isolated protein may constitute from 5 to 99.9% by weight of the total protein content depending on the circumstances. For example, the protein may be made at a significantly higher concentration through the use of an inducible promoter or high expression promoter, such that the protein is made at increased concentration levels. The definition includes the production of an antigen binding protein in a wide variety of organisms and/or host cells that are known in the art.

As disclosed herein, the experimental results of the present invention particularly demonstrate the inhibitory effect of antibodies directed against the immune-checkpoints CD112, CD155, and TIGIT, and as aspects of the disclosure Galectin-9 and TIM-3. However any other substance that can inhibit the immunoinhibitory signal between CD112 and TIGIT, CD155 and TIGIT and/or Galectin-9 and TIM-3 will have similar effects. The substance with such effects include for example soluble CD112, soluble CD155, soluble TIGIT, soluble Galectin-9, soluble TIM-3, C112 antagonists, CD155 antagonists, TIGIT antagonists, Galectin-9 antagonists, TIM-3 antagonists, substances that inhibit interaction between CD112 and TIGIT, CD155 and TIGIT and/or Galectin-9 and TIM-3, CD112 production inhibitors, CD155 production inhibitors, TIGIT production inhibitors, TIGIT production inhibitors, TIM-3 production inhibitors, and intracellular inhibitory signal inhibitors by TIGIT or TIM-3.

Accordingly, the CD112, CD155, and/or TIGIT, inhibitors used in the present invention comprise protein or non-protein compounds or agents. In this regard, proteins and polypeptides or derivatives that bind to CD112, CD155, and/or TIGIT include each partial proteins of CD112, CD155, and/or TIGIT, of which the immunoinhibiting signal between CD112 and TIGIT, CD155 and TIGIT and/or Galectin-9 and TIM-3 is not induced. The presence of TIGIT or TIM-3 in the neighborhood of the immune-checkpoint receptors is indispensable for the inducement of the immunoinhibitory signal of TIGIT or TIM-3, for that purpose it is restrained by the interaction with CD112, CD155 (TIGIT) or Galectin-9 (TIM-3) in tumors or carcinoma cells. Therefore, soluble CD112, CD155 or Galectin-9 with a part that is only extracellular domains and interacts with TIGIT or TIM-3 can inhibit the immunoinhibitory signal of CD112, CD155 or Galectin-9. On the other hand, soluble TIGIT or TIM-3 with a part which has a similar structure and can interact with CD122, CD155 or Galectin-9 can inhibit the immunoinhibitory signal. These soluble proteins have only to include extracellular region which is necessary and sufficient to bind to CD122, CD155, TIGIT, Galectin-9 or TIM-3 and can be prepared by a well-known expression and refining techniques.

If an interaction inhibitor of CD112, CD155, TIGIT, Galectin-9 or TIM-3 is a protein or polypeptide and an essential area for the interaction is composed by only a polypeptide and an essential area for the interaction is composed by only consecutive polypeptide, such a polypeptide fragment can become a mutual antagonist. Further, an antagonist with stringer activity can be identified from molecular groups of which this polypeptide fragment is chemically modified, or designed by computer based on the spatial structure of the polypeptide fragment. Also, the best antagonist can be more efficiently selected from molecular groups designed by computer based on protein stereoanalysis data of the interaction area.

It is further envisaged, that the inhibitor for use in the treatment of a blood-borne cancer, particularly AML as disclosed herein is a small molecule inhibitor. The term "small molecule" means a molecule with a low molecular weight, typically smaller than 1000 Da. Such a small molecule as used herein refers to beneficial agents having low molecular weight which are usually synthesized by organic chemistry, but may also be isolated from natural sources such as plants, fungi, and microbes. When for use in the treatment of blood-borne cancers, such small molecules are also termed as small molecule drugs. The common routes for delivering small molecule drugs are oral, injection, pulmonary, and transdermal.

It is further envisaged, that the inhibitor against CD112 reduces expression of CD112. Likewise, the inhibitor against CD155 reduces expression of CD155. Likewise, the inhibitor against TIGIT reduces expression of TIGIT. Likewise, the inhibitor against Galectin-9 reduces expression of Galectin-9. Likewise, the inhibitor against TIM-3 reduces expression of TIM-3. Accordingly, further disclosed herein is the use of a nucleic acid sequence (e.g. a therapeutic nucleic acid molecule, e.g., an antisense oligonucleotide, a DNA encoding same, or a vector producing same) to prepare an antisense molecule suitable for reducing the expression of a target gene, e.g. the genes encoding for CD112, CD155, TIGIT Galectin-9 and TIM-3. The term "reduce expression" when used herein refers to the ability of the inhibitor to decrease or block expression of the target gene, i.e. the genes encoding for CD112, CD155, TIGIT, Galectin-9 and TIM-3 in a specific and post-transcriptional manner. In this regard, the present disclosure relates to nucleotides capable of reducing the expression of CD112, CD155, TIGIT, Galectin-9 and/or TIM-3 in cancer cells. Said nucleotides may characterized by sequence which targets the mRNA and by having at least 50% sequence identity, or at least 70% sequence identity, or at least 80% sequence identity, or at least 90% sequence identity with the target mRNA. Particularly useful for this purpose are RNA sequences which can be used to prepare a nucleotide-based inhibitor. RNA duplexes of 21 - 23 nucleotides, with nucleotides 3' overhangs (called small interfering RNAs or siRNAs), have been shown to mediate sequence-specific inhibition of gene expression in mammalian cells via a post-transcriptional gene silencing (PTGS) mechanism termed RNA interference (RNAi). Accordingly, RNAi is considered as one of the most promising novel therapeutic strategies through the silencing of disease-causing genes *in vivo.* Thus, in one aspect of the disclosure, the nucleotide capable of reducing the expression of CD112, CD155, TIGIT, Galectin-9 and/or TIM-3 is an RNAi (iRNA). The person skilled in the art is aware of several techniques to synthetize RNAi and to deliver these constructs to tumor cells *in vivo,* thereby using e.g. liposomal formulations as described e.g. in Santel et al. 2006, Gene Therapy 13, 1360-1370. In this regard, double-stranded RNA is first synthesized with a sequence complementary to a gene of interest and introduced into a cell or organism, where it is recognized as exogenous genetic material and activates the RNAi pathway. Since RNAi may not totally abolish expression of the gene, this technique is sometimes referred as a *"gene knockdown",* to distinguish it from "http://en.wikipedia.org/wiki/Gene_knockout" procedures in which expression of a gene is entirely eliminated.

Accordingly, it is further envisaged that the inhibitor reducing expression of CD112, CD155, TIGIT, Galectin9 and/or TIM-3 knocks out CD112, CD155, TIGIT, Galectin-9 and/or TIM-3. The term "knock out" when used herein refers to a complete reduction of the expression of at least a portion of a polypeptide encoded by an endogenous gene encoding for CD112, CD155, TIGIT, Galectin-9 and/or TIM-3 of a single cell, selected cells, or all of the cells of a mammal, as compared to a wild type animal. The mammal may be a "heterozygous knockout", wherein one allele of the endogenous gene has been disrupted. Alternatively, the mammal may be a "homozygous knockout", wherein both alleles of the endogenous gene have been disrupted. It is also envisaged that more than one gene encoding for CD112, CD155, TIGIT, Galectin-9 and/or TIM-3, preferably two genes are "knocked out". In this case the mammal is a "double knocked out" mammal. In accordance with the present invention, the present inventors demonstrated that PVR and PVRL2 can be specifically single or double knocked out in AML cell lines when using CRISPR/Cas9 technique. Accordingly, the present disclosure also refers to an inhibitor against CD112, CD155, TIGIT, Galectin-9 and/or TIM-3 inhibitor that knocks out CD112, CD155, TIGIT, Galectin-9 and/or TIM-3, wherein said knock-out is achieved by CRISPR/cas9 technique. However, the skilled artisan is aware various different techniques applicable to knockout immune-checkpoints in blood-borne cancer cell, in particular AML cells. Knockout techniques therefore comprise any of the techniques to alter a gene sequence that result in an inactivated gene, or one in which the expression can be inactivated at a chosen time during development resulting in the loss of function of a gene.

The immune-checkpoint inhibitor can further comprise a chimeric antigen receptor (CAR) T cell. CAR T cells exhibit engineered receptors (chimeric antigen receptors) which graft the specificity of a monoclonal antibody onto a T cell with transfer of their coding sequence facilitated by retroviral vectors. These CARs allow the T cell to recognize a specific protein (antigen) on tumor cells. The CAR T cell preferably exhibits CARs that allow the T cell to recognize specific proteins on blood-borne cancer cells. Preferably, the CAR T cell is capable to recognize CD33 on the surface of blood-borne cancer cells, in particular on AML cells. In this regard, the addition of CAR T cells directed to specific surface molecule expressed on blood-borne cancer cells such as CD33 can enhance the cytotoxic effect of inhibitors against CD112, CD155, TIGIT, Galectin-9 and/or TIM-3. Accordingly, it is envisaged that the inhibitors against CD112, CD155, TIGIT, Galectin-9 and/or TIM-3 comprise a CAR T cell. Said CAR T cell preferably comprises a binding domain targeting a surface molecule expressed on blood-borne cancer cells, in particular AML cells. Said surface molecule is preferably CD33, CD19, or Flt3. Accordingly, the inhibitor may comprise a CAR T cell having a binding domain targeting CD33. The inhibitor may further comprise a CAR T cell having a binding domain targeting CD19. The inhibitor can also comprise a CAR T cell having a binding domain targeting Flt3. The skilled artisan is aware of a variety of techniques to produce CAR T cells of so called first, second and third generations directed said surface molecules.

In some aspects, the immune-checkpoint inhibitor as described elsewhere herein can further comprise an antibody construct engaging T cells. Such antibody constructs engaging T cells are preferably bispecific T cell engagers (BiTE antibody constructs), i.e. bispecific antibodies that bind to a T cell antigen and a tumor antigen. BiTE antibody constructs have been shown to induce directed lysis of target tumor cells and thus also provide great potential therapies for cancers and other disorders. One possible approach is the bispecific T cell engaging antibody construct AMG330 with dual specificity for CD3 and the sialic acid-binding lectin CD33, which is frequently expressed on the surface of AML blasts and leukemic stem cells. AMG330 was developed for the therapy of acute myeloid leukemia (AML) and will be evaluated in phase I studies shortly (Friedrich et al. 2014, American Association for Cancer Research, 1549-1557).

Bispecific T cell engagers may be in the format of different antibody constructs, such formats comprising e.g. di-scFv or bi(s)-scFv, (scFv)₂-Fc, scFv-zipper, (scFab)₂, Fab₂, Fab₃, diabodies, single chain diabodies, tandem diabodies (Tandab's), tandem di-scFv, tandem tri-scFv, "minibodies" exemplified by a structure which is as follows: (VH-VL-CH3)₂, (scFv-CH3)₂ , ((scFv)₂-CH3 + CH3), ((scFv)₂-CH3) or (scFv-CH3-scFv)₂, multibodies such as triabodies or tetrabodies, and bispecific single domain antibodies such as nanobodies or bispecific single variable domain antibodies comprising merely one variable domain in one or both of the binding domains, which might be VHH, VH or VL, that specifically bind an antigen or epitope independently of other V regions or domains. A binding domain within the T cell engaging molecule/bispecific antibody may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Additional examples for the format of antibody fragments, antibody variants or binding domains include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv), the latter being preferred (for example, derived from an scFV-library). Examples for antibody constructs are e.g. described in WO 00/006605, WO 2005/040220, WO 2008/119567, WO 2010/037838, WO 2013/026837, WO 2013/026833, US 2014/0308285, US 2014/0302037, WO2014/144722, WO 2014/151910, and WO 2015/048272.

In this regard, the present inventors studied in *in vitro* killing assays the therapeutic effect of PVR and PVRL2 blockage also in presence of AMG330 (**Figure 3-13**)**.** Thereby it was surprisingly found that AMG330 could significantly enhance cytotoxicity of PVR and/or PVRL2 blocking antibodies. Accordingly, it is envisaged that the inhibitors against CD112, CD155, TIGIT, Galectin-9 and/or TIM-3 may further comprise an antibody construct capable of engaging T cells. Said antibody construct preferably comprises a CD3 binding domain and a further binding domain targeting a surface molecule expressed on blood-borne cancer cell, in particular AML cells. Said surface molecule is selected from the group consisting of CD33, CD19, and Flt3. In accordance with the foregoing, the present disclosure provides for an inhibitor against CD112, CD155, Galectin-9, TIM-3 and/or TIGIT for use in a method of treatment of a blood-borne cancer, particularly AML comprising an antibody construct having a CD3 binding domain and a CD33 binding domain. The present disclosure further provides for an inhibitor against CD112, CD155, Galectin-9, TIM-3 and/or TIGIT for use in a method of treatment of a blood-borne cancer, particularly AML comprising an antibody construct having a CD3 binding domain and a CD19 binding domain. The present disclosure also provides for an inhibitor against CD112, CD155, Galectin-9, TIM-3 and/or TIGIT for use in a method of treatment of a blood-borne cancer, particularly AML comprising an antibody construct having a CD3 binding domain and a Flt3 binding domain. Said antibody construct is preferably a binding molecule capable of binding to CD3epsilon.

It is thus envisaged that the CD33, CD19 or Flt3 targeting antibody construct described herein has, apart from its function to bind to the cell surface molecules CD33, CD19, or Flt3 on a blood-borne cancer target cell and CD3 on the cell surface of a T cell, an additional function. In this format, the compound is a multifunctional compound by targeting cells through binding to CD33, CD19, or Flt3 on the cell surface of a cancer target cell, mediating cytotoxic T cell activity through CD3 binding and providing a further function such as a fully functional Fc constant domain mediating antibody-dependent cellular cytotoxicity through recruitment of effector cells like NK cells, a half-life extending domain such as an albumin binding domain or a modified Fc constant domain lacking antibody-dependent cellular cytotoxicity but extending the molecular weight of the compound, mediation of a label (fluorescent etc.), a therapeutic agent such as, e.g. a toxin or radionuclide, and/or means to enhance serum half-life, etc. Examples for bispecific antibody constructs targeting CD33, CD19 or Flt3 on the surface of blood-borne cancer target cells and CD3 on the cell surface of a T cell are e.g. molecules depicted in SEQ ID NOs: 15 to 37.

The terms "(capable of) binding to", "specifically recognizing", "against" and "reacting with" mean in accordance with this invention that a binding domain is capable of specifically interacting with one or more, preferably at least two, more preferably at least three and most preferably at least four amino acids of an epitope. As used herein, the terms "specifically interacting", "specifically binding" or "specifically bind(s)" mean that a binding domain exhibits appreciable affinity for a particular protein or antigen and, generally, does not exhibit significant reactivity with proteins or antigens other than CD33, CD19, Flt3, or CD3. "Appreciable affinity" includes binding with an affinity of about 10⁻⁶ M (KD) or stronger. Preferably, binding is considered specific when binding affinity is about 10⁻¹² to 10⁻⁸ M, 10⁻¹² to 10⁻⁹ M, 10⁻¹² to 10⁻¹⁰ M, 10⁻¹¹ to 10⁻⁸ M, preferably of about 10⁻¹¹ to 10⁻⁹ M. Whether a binding domain specifically reacts with or binds to a target can be tested readily by, *inter alia,* comparing the reaction of said binding domain with a target protein or antigen with the reaction of said binding domain with proteins or antigens other than CD33, CD19, Flt3, or CD3. Preferably, a binding domain of the invention does not essentially bind or is not capable of binding to proteins or antigens other than CD33, CD19, Flt3, or CD3 (i.e. the first binding domain is not capable of binding to proteins other than CD33, CD19 or Flt3 and the second binding domain is not capable of binding to proteins other than CD3). The term "does not essentially bind", or "is not capable of binding" means that a binding domain of the present invention does not bind another protein or antigen other than CD33, CD19, Flt3, or CD3, i.e., does not show reactivity of more than 30%, preferably not more than 20%, more preferably not more than 10%, particularly preferably not more than 9%, 8%, 7%, 6% or 5% with proteins or antigens other than CD33, CD19, Flt3, or CD3, whereby binding to CD33, CD19, Flt3, or CD3, respectively, is set to be 100%.A CD33, CD19 or FLT3 targeting compound described herein may also comprise additional domains, which e.g. are helpful in the isolation of the molecule or relate to an adapted pharmacokinetic profile of the molecule.

The targeting compound binding CD33, CD19 or FLT3 and CD3 as described herein can be produced in bacteria. After expression, the targeting compound, preferably the antibody construct is isolated from the *E. coli* cell paste in a soluble fraction and can be purified through, e.g., affinity chromatography and/or size exclusion. Final purification can be carried out similar to the process for purifying antibody expressed e. g, in CHO cells. In addition to prokaryotes, eukaryotic microbes such as filamentous fungi or yeast are suitable cloning or expression hosts for the CD33, CD19 or FLT3 targeting compound described herein. Plant cell cultures of cotton, corn, potato, soybean, petunia, tomato, Arabidopsis and tobacco can also be utilized as hosts. Cloning and expression vectors useful in the production of proteins in plant cell culture are known to those of skill in the art. See e.g. Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

A substance that inhibits the interaction of CD112 and TIGIT, CD155 and TIGIT and/or Galectin-9 and TIM-3 as described herein can be screened directly. Such substance can e.g. be identified from libraries of protein, polypeptide peptide, polynucleotide or polynucleoside, non-peptide compound organic synthesis compound or natural products (e.g. fermentation products, cell extracts, plant extracts, and animal tissues extracts). Accordingly, further disclosed herein is a method for screening an inhibitor against CD112, CD155, TIGIT, Galectin-9, and/or TIM-3 for use in a method of treatment of a blood-borne cancer, in particular AML. The screening method described can be executed by a method of measuring the cell function of a blood-borne cancer cell, particularly an AML cell. In this regard cells expressing CD112, CD155, or Galectin-9 on their surface can be used for the screening method. Such cells include leukocytes, monocytes, macrophages or antigen-presenting cells, epithelial cells, tumor cells, carcinoma cells, or those cell lines.

Further disclosed herein is the inhibitor, comprising an immunostimulant. As used herein, the term "immunostimulant" refers to any adjuvant which additionally stimulates the immune-potentiating effect of the inhibitors, thereby enhancing the cytotoxic activity of the involved T cell against the involved tumor cell. There is considerable evidence that cancer patients have T cells that are capable of attacking their tumor cells and the appearance of cancer is a failure of immune surveillance: the ability of one's own immune system to destroy cancer cells as soon as they appear. Immunostimulants are nonspecific agents that tune-up the body's immune defenses. In this regard, some successes has been described with injecting adjuvant-like agents directly into the tumor, oral therapy with levamisole, interleukine-2 (IL-2), a potent growth factor for T cells, interleukin-15 (IL-15), or alpha-interferon (IFN-α), just to name some.

As described herein, inhibitors against CD112, CD155, TIGIT, Galectin-9 and/or TIM-3 may be well suited for use in a method of treatment of a blood-borne cancer, in particular AML.

The term "treat", "treating", or "treatment" as used herein means to reduce, stabilize, or inhibit the progression of a blood-borne cancer, in particular AML. Those in need of a treatment comprise those already suffering from said disease. Preferably, a treatment reduces, or inhibits the proliferation activity of blood-borne cancer cells, thereby leading to an enhanced lysis of said cancer cells. "Treat", "treating", or "treatment" refers to therapeutic treatment, wherein the object is to slow down (lessen) or at least partially alleviate or abrogate the pathologic condition in the organism. Those in need of treatment include those already with the disease as well as those supposed to having the disease. The term "administering" relates to a method of incorporating a compound into cells or tissues of an organism.

The compounds for use in the treatment of a subject from a blood-borne cancer, in particular AML as described in the present invention are generally administered to the subject in a therapeutically effective amount. Said therapeutically effective amount is sufficient to inhibit or alleviate the symptoms of a blood-borne cancer, in particular AML. By "therapeutic effect" or "therapeutically effective" is meant that the compound for use will elicit the biological or medical response of a tissue, system, animal or human that is being sought by the researcher, veterinarian, medical doctor or other clinician. The term "therapeutically effective" further refers to the inhibition of factors causing or contributing to the disease. The term "therapeutically effective amount" includes that the amount of the compound when administered is sufficient to significantly improve the progression of the disease being treated. The therapeutically effective amount will vary depending on the compound, the disease and its severity and on individual factor of the subject such. Therefore, the compound used in the present invention will not in all cases turn out to be therapeutically effective, because the method disclosed herein cannot provide a 100% safe prediction whether or not a subject may be responsive to said compound, since individual factors are involved as well. It is to expect that age, body weight, general health, sex, diet, drug interaction and the like may have a general influence as to whether or not the compound for use in the treatment of a subject suffering from a blood-borne cancer, in particular AML will be therapeutically effective. Preferably, the therapeutically effective amount of the compound used to treat a subject suffering from a blood-borne cancer, in particular AML is between about 0.01 mg per kg body weight and about 1 g per kg body weight, such as about 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, 10, 100, 200, 300, 400, 500, 600, 700, 800, or about 900 mg per kg body weight. Even more preferably, the therapeutically effective amount of the compound used to treat a subject suffering from a blood-borne cancer, in particular AML is between about 0.01 mg per kg body weight and about 100 mg per kg body weight, such as between about 0.1 mg per kg body weight and about 10 mg per kg body weight. The therapeutic effective amount of the compound will vary with regard to the weight of active compound contained therein, depending on the species of subject to be treated.

The term "subject" as used herein, also addressed as an individual, refers to a mammal. The mammal may be any one of mouse, rat, guineas pig, rabbit, cat, dog, monkey, horse, or human. Accordingly, the mammal according to the present invention may be a human or a non-human mammal. Thus, the methods, uses and compositions described in this document are generally applicable to both human and non-human mammals. Where the subject is a human who may receive the compound for use in the treatment for a disease as described herein, it is also addressed as a "patient". What is disclosed for a "patient" herein also applies to a group of patients, mutatis mutandis.

The administration of the inhibitors for use in the treatment of a subject suffering from blood-borne cancer, in particular AML according to the present invention can be carried out by any method known in the art. In some embodiments, the administration is carried out orally, parenterally, subcutaneously, intravenously, intramuscularly, intraperitoneally, by intranasal instillation, by implantation, by intracavitary or intravesical instillation, intraocularly, intraarterially, intralesionally, transdermally, or by application to mucous membranes, or combinations thereof, just to name some.

In the scope of the present invention, it is for example envisaged that the therapeutic effect of the used inhibitors is detected by evaluating the number of blood-born cancer cells in a patient, using techniques available in the art, e.g. using the white blood cell (WBC, or leukocyte) count and differential. White blood cells can be counted manually in hemocytometers (Neubauer chamber) or with automated counters. To determine the differential, a drop of blood can be thinly spread over a glass slide, air dried, and stained with a Romanofsky stain, most commonly the Wright or May-Grunewald-Giemsa technique. Cells are then counted and classified using morphologic examination and/or histochemistry as described in Blumenreich MS. The White Blood Cell and Differential Count. In: Walker HK, Hall WD, Hurst JW, editors. Clinical Methods: The History, Physicals, and Laboratory Examinations. 3rd edition. Boston: Butterworths; 1990. Chapter 153. Alternatively, leukocytes are isolated from a blood sample and stained with fluorescent-labeled antibodies against leucocyte cell surface markers and subsequently analyzed by flow cytometry in order to calculate absolute cell numbers for each leukocyte subpopulation. Additionally or alternatively, it is also possible to evaluate the general appearance of the respective patient, which will also aid the skilled practitioner to evaluate whether the therapy is effective. Those skilled in the art are aware of numerous other ways which will enable a practitioner to observe a therapeutic effect of the compound for use in the treatment of blood-borne cancer, in particular AML as disclosed herein in the context of a method or use of the present invention.

While it is possible to administer the inhibitors according to the present invention directly without any formulation, in another aspect of the present invention the compounds are preferably employed in the form of a pharmaceutical or veterinary formulation composition, comprising a pharmaceutically or veterinarily acceptable carrier, diluent or excipient and a compound as defined in the claims, i.e. the immunoglobulin as defined in the claims. The carrier used in combination with the compound is water-based and forms an aqueous solution. An oil-based carrier solution containing the compound is an alternative to the aqueous carrier solution. Either aqueous or oil-based solutions further contain thickening agents to provide the composition with the viscosity of a liniment, cream, ointment, gel, or the like. Suitable thickening agents are well known to those skilled in the art. Alternative embodiments of the present invention can also use a solid carrier containing the compound for use in the treatment of a S100A12:TLR4/MD2/CD14-mediated inflammatory disorder as disclosed elsewhere herein. This enables the alternative embodiment to be applied via a stick applicator, patch, or suppository. The solid carrier further contains thickening agents to provide the composition with the consistency of wax or paraffin.

It is also conceivable to use further AML treatment in combination with the inhibitors for use in the treatment of a subject suffering from a blood-borne cancer, in particular AML. Further AML treatment can in general be applied antecedently, simultaneously, and/or subsequently to the uses of the invention.

Hematopoietic stem cell transplantation (HSCT) is a common AML treatment. The term generally refers to transplantation of hematopoietic stem cells, usually derived from bone marrow or blood, and comprises autologous (i.e., the stem cells are derived from the patient) and allogeneic (i.e., the stem cells are derived from a donor) HSCT. For AML treatment, allogeneic HSCT is generally preferred. It is also envisaged that the uses of the present invention can be applied before or after HSCT, or both, or in between two HSCT treatments.

Patients (or groups of patients) treated according to the invention may also receive a chemotherapeutic treatment. In the context of the present invention, a "chemotherapeutic treatment" refers to a treatment with an antineoplastic agent or the combination of more than one of these agents into a standardized treatment regimen. In the context of the present invention, the term "chemotherapeutic treatment" comprises any antineoplastic agent including small sized organic molecules, peptides, oligonucleotides and the like. Agents included in the definition of chemotherapy are, without limitation, alkylating agents, e.g. mechlorethamine, cyclophosphamide, melphalan, chlorambucil, ifosfamide, busulfan, N-Nitroso-N-methylurea (MNU), carmustine (BCNU), lomustine (CCNU), semustine (MeCCNU), fotemustine, streptozotocin, dacarbazine, mitozolomide, temozolomide, thiotepa, mytomycin, diaziquone (AZQ), cisplatin, carboplatin, oxaliplatin, procarbazine and hexamethylmelamine; antimetabolites, e.g. methotrexate, pemetrexed, fluorouracil, capecitabine, cytarabine, gemcitabine, decitabine, Vidaza, fludarabine, nelarabine, cladribine, clofarabine, pentostatin, thioguanine, mercaptopurine; anti-microtubule agents e.g. vincristine, vinblastine, vinorelbine, vindesine, vinflunine, paclitaxel, docetaxel, podophyllotoxin; topoisomerase inhibitors, e.g. irinotecan, topotecan, etoposide, doxorubicin, mitoxantrone, teniposide, novobiocin, merbarone, aclarubicin; cytotoxic antibiotics, e.g. actinomycin, bleomycin, plicamycin, mitomycin, doxorubicin, daunorubicin, epirubicin, idarubicin, pirarubicin, aclarubicin, and mitoxantrone, just to name some. However, one of ordinary skill in the art will appreciate that the invention is not limited to these chemotherapeutic agents and may involve the use of other DNA damaging agents as well. Said combination according to the present invention can be administered as a combined formulation or separate from each other.

Further AML treatment also includes radiation therapy. CNS treatment or prophylaxis is also envisaged in order to prevent malignant cells from spreading in the CNS, e.g. by intrathecal chemotherapy and/or radiation therapy of the brain and spinal cord.
Since the present inventors speculate that therapeutic success of the inhibitors disclosed herein could be based (in part) on an immunopotentiating activity of said inhibitors by inhibiting the immune-checkpoints described herein, thereby resulting in enhanced T-cell anti-cancer activity, inducers and enhancers of T cell activation and/or proliferation, CAR T cells, donor T cells, anti-cytotoxic T-lymphocyte-associated antigen-4 (CTLA-4) antibodies and others are also envisaged.

According to another aspect, the present disclosure provides a pharmaceutical composition comprising an immune-checkpoint inhibitor against CD112 (Nectin-2, PVRL2), CD155 (PVR), Galectin-9, TIM-3 and/or TIGIT as described elsewhere herein and a CAR T cell as described elsewhere herein. In another aspect, the present disclosure provides a pharmaceutical composition comprising an immune-checkpoint inhibitor against CD112 (Nectin-2, PVRL2), CD155 (PVR), Galectin-9, TIM-3 and/or TIGIT as described elsewhere herein and an antibody construct capable of engaging T cells as described elsewhere herein.

Said pharmaceutical composition may comprise a therapeutically effective amount of one or a plurality of the immune-checkpoint inhibitors, CAR T cells and/or antibody construct described herein together with a pharmaceutically effective diluents, carrier, solubilizer, emulsifier, preservative, and/or adjuvant. Pharmaceutical compositions described herein include, but are not limited to, liquid, frozen, and lyophilized compositions. Preferably, formulation materials are nontoxic to recipients at the dosages and concentrations employed.

As used herein, the term "pharmaceutical composition" relates to a composition for administration to a patient, preferably a human patient. Preferably, the pharmaceutical composition comprises suitable formulations of carriers, stabilizers and/or excipients. In a preferred embodiment, the pharmaceutical composition comprises a composition for parenteral, transdermal, intraluminal, intraarterial, intrathecal and/or intranasal administration or by direct injection into tissue. It is in particular envisaged that said composition is administered to a patient via infusion or injection. Administration of the suitable compositions may be effected by different ways, e.g., by intravenous, intraperitoneal, subcutaneous, intramuscular, topical or intradermal administration. In particular, the present invention provides for an uninterrupted administration of the suitable composition. As a non-limiting example, uninterrupted, i.e. continuous administration may be realized by a small pump system worn by the patient for metering the influx of therapeutic agent into the body of the patient as described in WO2015/036583.

The compositions may further comprise a pharmaceutically acceptable carrier. Examples of suitable pharmaceutical carriers are well known in the art and include solutions, e.g. phosphate buffered saline solutions, water, emulsions, such as oil/water emulsions, various types of wetting agents, sterile solutions, liposomes, etc. Compositions comprising such carriers can be formulated by well known conventional methods. Formulations can comprise carbohydrates, buffer solutions, amino acids and/or surfactants. Carbohydrates may be non-reducing sugars, preferably trehalose, sucrose, octasulfate, sorbitol or xylitol. In general, as used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; cosolvents; antioxidants, including ascorbic acid and methionine; chelating agents such as EDTA; metal complexes (e.g., Zn-protein complexes); biodegradable polymers, such as polyesters; salt-forming counter-ions, such as sodium, polyhydric sugar alcohols; amino acids, such as alanine, glycine, asparagine, 2-phenylalanine, and threonine; sugars or sugar alcohols, such as trehalose, sucrose, octasulfate, sorbitol or xylitol stachyose, mannose, sorbose, xylose, ribose, myoinisitose, galactose, lactitol, ribitol, myoinisitol, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Such formulations may be used for continuous administrations which may be intravenuous or subcutaneous with and/or without pump systems. Amino acids may be charged amino acids, preferably lysine, lysine acetate, arginine, glutamate and/or histidine. Surfactants may be detergents, preferably with a molecular weight of >1.2 KD and/or a polyether, preferably with a molecular weight of >3 KD. Non-limiting examples for preferred detergents are Tween 20, Tween 40, Tween 60, Tween 80 or Tween 85. Non-limiting examples for preferred polyethers are PEG 3000, PEG 3350, PEG 4000 or PEG 5000. Buffer systems used in the present invention can have a preferred pH of 5-9 and may comprise citrate, succinate, phosphate, histidine and acetate.

The pharmaceutical compositions can be administered to the subject at a suitable dose which can be determined e.g. by dose escalating studies by administration of increasing doses of the polypeptide described herein exhibiting cross-species specificity described herein to non-chimpanzee primates, for instance macaques. As set forth above, the CD33 targeting composition described herein exhibiting cross-species specificity described herein can be advantageously used in identical form in preclinical testing in non-chimpanzee primates and as drug in humans. The composition or these compositions can also be administered in combination with additional other proteinaceous and non-proteinaceous drugs. These drugs may be administered simultaneously with the composition comprising the polypeptide described herein as defined herein or separately before or after administration of said polypeptide in timely defined intervals and doses. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, inert gases and the like. In addition, the composition might comprise proteinaceous carriers, like, e.g., serum albumin or immunoglobulin, preferably of human origin. It is envisaged that the composition might comprise, in addition to the polypeptide described herein defined herein, further biologically active agents, depending on the intended use of the composition. Such agents might be drugs acting on the gastrointestinal system, drugs acting as cytostatica, drugs preventing hyperuricemia, drugs inhibiting immunoreactions (e.g. corticosteroids), drugs modulating the inflammatory response, drugs acting on the circulatory system and/or agents such as cytokines known in the art. It is also envisaged that the composition comprising the CD33 targeting compound and at least one epigenetic factor in a single or separate formulations is applied in an additional co-therapy, i.e., in combination with another anti-cancer medicament.

The biological activity of the pharmaceutical composition defined herein can be determined for instance by cytotoxicity assays, as described in the following examples, in WO 99/54440 or by Schlereth et al. (Cancer Immunol. Immunother. 20 (2005), 1-12). "Efficacy" or "*in vivo* efficacy" as used herein refers to the response to therapy by the pharmaceutical composition of the invention, using e.g. standardized NCI response criteria. The success or *in vivo* efficacy of the therapy using a pharmaceutical composition according to the invention refers to the effectiveness of the composition for its intended purpose, i.e. the ability of the composition to cause its desired effect, i.e. depletion of pathologic cells, e.g. tumor cells. The *in vivo* efficacy may be monitored by established standard methods for the respective disease entities including, but not limited to white blood cell counts, differentials, Fluorescence Activated Cell Sorting, bone marrow aspiration. In addition, various disease specific clinical chemistry parameters and other established standard methods may be used. Furthermore, computer-aided tomography, X-ray, nuclear magnetic resonance tomography (e.g. for National Cancer Institute-criteria based response assessment [Cheson BD, Horning SJ, Coiffier B, Shipp MA, Fisher RI, Connors JM, Lister TA, Vose J, Grillo-Lopez A, Hagenbeek A, Cabanillas F, Klippensten D, Hiddemann W, Castellino R, Harris NL, Armitage JO, Carter W, Hoppe R, Canellos GP. Report of an international workshop to standardize response criteria for non-Hodgkin's lymphomas. NCI Sponsored International Working Group. J Clin Oncol. 1999 Apr;17(4):1244]), positron-emission tomography scanning, white blood cell counts, differentials, Fluorescence Activated Cell Sorting, bone marrow aspiration, lymph node biopsies/histologies, and various lymphoma specific clinical chemistry parameters (e.g. lactate dehydrogenase) and other established standard methods may be used.

Another major challenge in the development of drugs is the predictable modulation of pharmacokinetic properties. To this end, a pharmacokinetic profile of the drug candidate, i.e. a profile of the pharmacokinetic parameters that affect the ability of a particular drug to treat a given condition, can be established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above. "Half-life" means the time where 50% of an administered drug are eliminated through biological processes, e.g. metabolism, excretion, etc. By "hepatic first-pass metabolism" is meant the propensity of a drug to be metabolized upon first contact with the liver, i.e. during its first pass through the liver. "Volume of distribution" means the degree of retention of a drug throughout the various compartments of the body, like e.g. intracellular and extracellular spaces, tissues and organs, etc. and the distribution of the drug within these compartments. "Degree of blood serum binding" means the propensity of a drug to interact with and bind to blood serum proteins, such as albumin, leading to a reduction or loss of biological activity of the drug.

Pharmacokinetic parameters also include bioavailability, lag time (Tlag), Tmax, absorption rates, more onset and/or Cmax for a given amount of drug administered. "Bioavailability" means the amount of a drug in the blood compartment. "Lag time" means the time delay between the administration of the drug and its detection and measurability in blood or plasma. "Tmax" is the time after which maximal blood concentration of the drug is reached, and "Cmax" is the blood concentration maximally obtained with a given drug. The time to reach a blood or tissue concentration of the drug which is required for its biological effect is influenced by all parameters.

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", "*in vivo* safety" or "tolerability" as used herein defines the administration of a drug without inducing severe adverse events directly after administration (local tolerance) and during a longer period of application of the drug. "Safety", "*in vivo* safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviations to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance hematology, clinical chemistry, coagulation profile and urine analysis and examination of other body fluids such as serum, plasma, lymphoid or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the disease and the general state of the subject's own immune system. The term "patient" includes human and other mammalian subjects that receive either prophylactic or therapeutic treatment. The term "effective and non-toxic dose" as used herein refers to a tolerable dose of a pharmaceutical composition (i.e. a pharmaceutical composition comprising the inhibitor against CD112, CD155, TIGIT, Galectin-9 and/or TIM-3 and a CAR T cell or an antibody construct engaging T cells in a single or separate formulations) which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The above terms are also referred to e.g. in the preclinical safety evaluation of biotechnology-derived pharmaceuticals S6; ICH Harmonised Tripartite Guideline; ICH Steering Committee meeting on July 16, 1997. The appropriate dosage, or therapeutically effective amount, of a pharmaceutical composition comprising the inhibitor according to the present invention and a CAR T cell or an antibody construct as described elsewhere herein will depend on the condition to be treated, the severity of the condition, prior therapy, and the patient's clinical history and response to the therapeutic agent. The proper dose can be adjusted according to the judgment of the attending physician such that it can be administered to the patient one time or over a series of administrations. The pharmaceutical composition can be administered as a sole therapeutic or in combination with additional therapies such as anti-cancer therapies as needed.

The pharmaceutical compositions according this invention are particularly useful for parenteral administration, i.e., subcutaneously, intramuscularly, intravenously, intra-articular and/or intra-synovial. Parenteral administration can be by bolus injection or continuous infusion. If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

It has been surprisingly found in connection with the present invention that a combination of the inhibitor as defined in the claims and the bispecific antibody construct AMG330 significantly increases the cell lysis of AML cells in a dose-dependent manner as compared to the cell lysis solely reduced by blocking antibodies against CD112 and CD155 alone (**Figure 3-13**). This finding supports that the combination of an inhibitor against CD112, CD155, TIGIT, Galectin-9 and/or TIM-3 and a CD33, CD19 or Flt3 directed T cell engager would be synergistically more effective than either therapy administered separately. Accordingly, the described administration of one or more inhibitor against CD112, CD155, TIGIT, Galectin-9 and/or TIM-3 in combination with a CD33, CD19 or Flt3 directed T cell engager described herein may allow for lower doses of a bispecific T cell engager to be effective at a given time point. The redirected lysis of target cells via the recruitment of T cells by a multispecific, at least bispecific, construct involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261 or WO 2008/119567.

The formulations described herein are useful as pharmaceutical compositions for use in the treatment, amelioration and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease. Those "in need of treatment" include those already with the disorder, as well as those in which the disorder is to be prevented. The term "disease" is any condition that would benefit from treatment with the protein formulation described herein. This includes chronic and acute disorders or diseases including those pathological conditions that predispose the mammal to the disease in question. Non-limiting examples of diseases/disorders to be treated herein include the herein described blood-borne cancers, particularly myeloid leukemia such as AML.

The pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine, proline, or lysine); antimicrobials; antioxidants (such as ascorbic acid, sodium sulfite or sodium hydrogensulfite); buffers (such as borate, bicarbonate, Tris-HCI, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. See, REMINGTON'S PHARMACEUTICAL SCIENCES, 18" Edition, (A. R. Genrmo, ed.), 1990, Mack Publishing Company.

The optimal pharmaceutical composition will be determined by one skilled in the art depending upon, for example, the intended route of administration, delivery format and desired dosage. See, for example, REMINGTON'S PHARMACEUTICAL SCIENCES, supra. In certain embodiments, such compositions may influence the physical state, stability, rate of in vivo release and rate of *in vivo* clearance of the antigen binding proteins described herein. In certain embodiments, the primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. In specific embodiments, pharmaceutical compositions comprise Tris buffer of about pH 7.0-8.5, or acetate buffer of about pH 4.0-5.5, and may further include sorbitol or a suitable substitute therefore. In certain embodiments of the invention, human antibody or antigen binding fragment thereof described herein or the antibody construct described herein compositions may be prepared for storage by mixing the selected composition having the desired degree of purity with optional formulation agents (REMINGTON'S PHARMACEUTICAL SCIENCES, supra) in the form of a lyophilized cake or an aqueous solution. Further, in certain embodiments, the inhibitor against CD112, CD155, TIGIT, Galectin-9 and/or TIM-3 and a CAR T cell or an antibody construct engaging T cells may be formulated as a lyophilizate using appropriate excipients such as sucrose.

The pharmaceutical compositions described herein can be selected for parenteral delivery. Alternatively, the compositions may be selected for inhalation or for delivery through the digestive tract, such as orally. Preparation of such pharmaceutically acceptable compositions is within the skill of the art. The formulation components are present preferably in concentrations that are acceptable to the site of administration. In certain embodiments, buffers are used to maintain the composition at physiological pH or at a slightly lower pH, typically within a pH range of from about 5 to about 8.

Additional pharmaceutical compositions will be evident to those skilled in the art, including formulations involving the inhibitor against CD112, CD155, TIGIT, Galectin-9 and/or TIM-3 and a CAR T cell or an antibody construct engaging T cells as described herein in sustained- or controlled-delivery formulations. Techniques for formulating a variety of other sustained- or controlled-delivery means, such as liposome carriers, bio-erodible microparticles or porous beads and depot injections, are also known to those skilled in the art. See, for example, International Patent Application No. WO 9315722, which describes controlled release of porous polymeric microparticles for delivery of pharmaceutical compositions. Sustained-release preparations may include semipermeable polymer matrices in the form of shaped articles, e.g., films, or microcapsules. Sustained release matrices may include polyesters, hydrogels, polylactides (as disclosed in U.S. Pat. No. 3,773,919 and European Patent Application Publication No. EP 058481), copolymers of L-glutamic acid and gamma ethyl-L-glutamate (Sidman et al., 1983, Biopolymers 2:547-556), poly (2-hydroxyethyl-methacrylate) (Langer et al., 1981, J. Biomed. Mater. Res. 15:167-277 and Langer, 1982, Chem. Tech. 12:98-105), ethylene vinyl acetate (Langer et al., 1981, supra) or poly-D(-)-3-hydroxybutyric acid (European Patent Application Publication No. EP 133,988). Sustained release compositions may also include liposomes that can be prepared by any of several methods known in the art. See, e.g., Eppstein et al., 1985, Proc. Natl. Acad. Sci. U.S.A. 82:3688-3692; European Patent Application Publication Nos. EP 036,676; EP 088,046 and EP 143,949.

Pharmaceutical compositions used for *in vivo* administration are typically provided as sterile preparations. Sterilization can be accomplished by filtration through sterile filtration membranes. When the composition is lyophilized, sterilization using this method may be conducted either prior to or following lyophilization and reconstitution. Compositions for parenteral administration can be stored in lyophilized form or in a solution. Parenteral compositions generally are placed into a container having a sterile access port, for example, an intravenous solution bag or vial having a stopper pierceable by a hypodermic injection needle. Salts may be used in accordance with certain embodiments of the invention to, for example, adjust the ionic strength and/or the isotonicity of a formulation and/or to improve the solubility and/or physical stability of a protein or other ingredient of a composition in accordance with the invention. As is well known, ions can stabilize the native state of proteins by binding to charged residues on the protein's surface and by shielding charged and polar groups in the protein and reducing the strength of their electrostatic interactions, attractive, and repulsive interactions. Ions also can stabilize the denatured state of a protein by binding to, in particular, the denatured peptide linkages (--CONH) of the protein. Furthermore, ionic interaction with charged and polar groups in a protein also can reduce intermolecular electrostatic interactions and, thereby, prevent or reduce protein aggregation and insolubility.

A number of categorical rankings of ions and their effects on proteins have been developed that can be used in formulating pharmaceutical compositions in accordance with the invention. One example is the Hofmeister series, which ranks ionic and polar non-ionic solutes by their effect on the conformational stability of proteins in solution. Stabilizing solutes are referred to as "kosmotropic." Destabilizing solutes are referred to as "chaotropic." Kosmotropes commonly are used at high concentrations (e.g., >1 molar ammonium sulfate) to precipitate proteins from solution ("salting-out"). Chaotropes commonly are used to denture and/or to solubilize proteins ("salting-in"). The relative effectiveness of ions to "salt-in" and "salt-out" defines their position in the Hofmeister series.

Free amino acids can be used in the formulations in accordance with various embodiments of the invention as bulking agents, stabilizers, and antioxidants, as well as other standard uses. Lysine, proline, serine, and alanine can be used for stabilizing proteins in a formulation. Glycine is useful in lyophilization to ensure correct cake structure and properties. Arginine may be useful to inhibit protein aggregation, in both liquid and lyophilized formulations. Methionine is useful as an antioxidant. Polyols include sugars, e.g., mannitol, sucrose, and sorbitol and polyhydric alcohols such as, for instance, glycerol and propylene glycol, and, for purposes of discussion herein, polyethylene glycol (PEG) and related substances. Polyols are kosmotropic. They are useful stabilizing agents in both liquid and lyophilized formulations to protect proteins from physical and chemical degradation processes. Polyols also are useful for adjusting the tonicity of formulations.

The formulations may further comprise one or more antioxidants. To some extent deleterious oxidation of proteins can be prevented in pharmaceutical formulations by maintaining proper levels of ambient oxygen and temperature and by avoiding exposure to light. Antioxidant excipients can be used as well to prevent oxidative degradation of proteins. Among useful antioxidants in this regard are reducing agents, oxygen/free-radical scavengers, and chelating agents. Antioxidants for use in therapeutic protein formulations in accordance with the invention preferably are water-soluble and maintain their activity throughout the shelf life of a product. EDTA is a preferred antioxidant in accordance with the invention in this regard.

Antioxidants can damage proteins. For instance, reducing agents, such as glutathione in particular, can disrupt intramolecular disulfide linkages. Thus, antioxidants for use in the invention are selected to, among other things, eliminate or sufficiently reduce the possibility of themselves damaging proteins in the formulation. Formulations in accordance with the invention may include metal ions that are protein co-factors and that are necessary to form protein coordination complexes, such as zinc necessary to form certain insulin suspensions. Metal ions also can inhibit some processes that degrade proteins. However, metal ions also catalyze physical and chemical processes that degrade proteins.

As might be expected, development of liquid formulations containing preservatives are more challenging than lyophilized formulations. Freeze-dried products can be lyophilized without the preservative and reconstituted with a preservative containing diluent at the time of use. This shortens the time for which a preservative is in contact with the protein, significantly minimizing the associated stability risks. With liquid formulations, preservative effectiveness and stability should be maintained over the entire product shelf-life (about 18 to 24 months). An important point to note is that preservative effectiveness should be demonstrated in the final formulation containing the active drug and all excipient components.

Once the pharmaceutical composition has been formulated, it may be stored in sterile vials as a solution, suspension, gel, emulsion, solid, crystal, or as a dehydrated or lyophilized powder. Such formulations may be stored either in a ready-to-use form or in a form (e.g., lyophilized) that is reconstituted prior to administration. The disclosure also provides kits for producing a single-dose administration unit. In certain aspects, kits containing single and multi-chambered pre-filled syringes (e.g., liquid syringes and lyosyringes) are provided. The therapeutically effective amount of the pharmaceutical composition comprising an inhibitor against CD112, CD155, TIGIT, Galectin-9 and/or TIM-3 and a CAR T cell or an antibody construct engaging T cells will depend, for example, upon the therapeutic context and objectives. One skilled in the art will appreciate that the appropriate dosage levels for treatment will vary depending, in part, upon the molecule delivered, the indication for which the composition described herein is being used, the route of administration, and the size (body weight, body surface or organ size) and/or condition (the age and general health) of the patient. In certain embodiments, the clinician may titer the dosage and modify the route of administration to obtain the optimal therapeutic effect. A typical dosage may range from about 0.1 µg/kg to up to about 30 mg/kg or more, depending on the factors mentioned above. In specific embodiments, the dosage may range from 1.0 µg/kg up to about 20 mg/kg, optionally from 10 µg/kg up to about 10 mg/kg or from 100 µg/kg up to about 5 mg/kg.

A therapeutic effective amount of pharmaceutical composition comprising the inhibitor against CD112, CD155, TIGIT, Galectin-9 and/or TIM-3 and a CAR T cell or an antibody construct engaging T cells as described elsewhere herein preferably results in a decrease in severity of disease symptoms, in increase in frequency or duration of disease symptom-free periods or a prevention of impairment or disability due to the disease affliction. For treating CD112, CD155 and or Galectin-9-expressing tumors, a therapeutically effective amount of the composition disclosed herein preferably inhibits cell growth or tumor growth by at least about 20%, at least about 40%, at least about 50%, at least about 60%, at least about 70%, at least about 80%, or at least about 90% relative to untreated patients. The ability of a compound to inhibit tumor growth may be evaluated in an animal model predictive of efficacy in human tumors.

The pharmaceutical compositions may be administered using a medical device. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

Of note, the immune-checkpoint inhibitors described herein as well as all embodiments pertaining thereto as described herein may be for use in methods of treating of a blood-borne cancer, such as leukemia or lymphoma, particularly AML, comprising administering a therapeutically effective amount of said inhibitor to a subject in need thereof.

Similarly, the immune-checkpoint inhibitors described herein may be used for the preparation of a pharmaceutical composition for use in the treatment of a blood-borne cancer, such as leukemia or lymphoma, particularly AML.

### EXAMPLES

The following examples are provided for the purpose of illustrating specific embodiments or features of the present invention. These examples should not be construed as to limit the scope of this invention. The examples are included for purposes of illustration, and the present invention is limited only by the claims.

Samples from 140 treatment naive patients with newly diagnosed AML (AMLSG 07-04, NCT00151242) were analyzed by RT-qPCR for expression of the immune checkpoint molecules PVR, PVRL2 and Galectin-9 (Gal-9). Expression was correlated with patient demographics (age, karyotype, FLT3 mutation status) and clinical survival data by multivariate cox regression. The majority of patients showed mRNA expression of PVR (94%), PVRL2 (95%) and Gal-9 (92%). In a multivariate stepwise cox regression for overall survival, an unfavorable karyotype, high PVR and high Gal-9 expression were identified as independent prognostic markers (p<0.001, HR: 2.10, CI 1.39-3.15 for the karyotype; p=0.001, HR: 1.64, CI 1.21-2.21 for PVR and p<0.001, HR: 0.67, CI 0.54-0.84 for Gal-9). Due to a high correlation between PVR and PVRL2 (Pearson's rho=0.827, p<0.001), PVRL2 was removed during the stepwise process. Nevertheless, if PVR was excluded from the multivariate cox regression, PVRL2 remained as significant term in the stepwise procedure in addition to the karyotype and Gal-9 (p=0.003, HR: 1.58, CI 1.17-2.13 for PVRL2). In a second, independent patient cohort containing microarray-based gene expression and clinical data of 291 AML patients (Verhaak *et. al.,* Haematologica 2009;94) a high PVR and PVRL2 expression in contrast to expression of CD80, CD86 or PD-L1 was associated with poor overall survival (log-rank test p=0.003 and p=0.032, respectively). In *in vitro* killing assays the therapeutic effect of PVR and PVRL2 blockade was studied by FACS using 7-AAD staining. AML cell lines MV4-11, Kasumi-1 and Molm-13 were preincubated with blocking antibodies against PVR, PVRL2 or both and co-cultured for 24h with peripheral blood mononuclear cells (PBMCs) of healthy donors in the presence or absence of AMG 330.

In the absence of AMG 330, the cell kill of MV4-11 increased from 12.6 ±4.7% (control) to 33.0 ±8.8% (PVR), to 40.4 ±10.4% (PVRL2) and to 56.0 ±12.0% (both PVR + PVRL2). In the presence of suboptimal concentration of AMG 330 (0.1 ng/ml MV4-11 cell lysis was 29.4±9.0% (AMG 330 alone), 49.7 ±12.6% (AMG 330 + PVR), 57.9 ±11.3% (AMG 330 + PVRL2) and 70.0 ±9.8% (AMG 330 + PVR + PVRL2; n=4, p<0.05 for all comparisons). Comparable results were found for Kasumi-1 and Molm-13 with blockade of both checkpoint inhibitors being the most effective treatment, although additive effects of antibodies against PVR and PVRL2 could not be verified in all cases (data not shown). To confirm specificity of the approach and to exclude effects caused by antibody dependent cellular cytotoxicity (ADCC), PVR and PVRL2 double knockouts of the cell line MV4-11 were generated by CRISPR/Cas-9. Significantly increased killing was observed in PVR and PVRL2 double knockout cells compared to wild-type cells (40.5 ±8.1 % vs. 25.9 ±9.1; n=3, p<0.001). Further experiments using an irrelevant antibody against CD117 or Fcy receptor blockade by purified IgG antibodies excluded ADCC confirming the functional relevance of PVR/PVRL2 blockade.

The expression of immune checkpoint ligands PVR and PVRL2 confers a negative prognosis to AML patients possibly due to immune evasion. We could further show that the killing of AML cells by PBMCs could be augmented by blockade of these novel checkpoint inhibitors. Furthermore, addition of PVR and/or PVRL2 blocking antibodies to AMG 330 could enhance cytotoxicity. Therefore, blockade of PVR and PVRL2 represents a promising target for the treatment of AML.

### Prognostic impact of PVR, PVRL2 and Galectin-9 in AML

The PVR, PVRL2 and Galectin-9 mRNA expression of 140 patients with de-novo AML was analyzed in quantitative RT-PCR using the LightCycler 96 (Roche, Basel, Switzerland). Glyceraldehyde 3-phosphate dehydrogenase (GAPDH) served as reference gene. The following primers were used: PVR forward 5'-agcaggagcgtggatatctg-3' (SEQ ID No: 1), PVR reverse 5'-gactgtgccagacaggaacc-3'(SEQ ID No: 2), PVRL2 forward 5'-gaggacgagggcaactacac-3' (SEQ ID No: 3), PVRL2 reverse 5'-agggatgagagccaggagat-3' (SEQ ID No: 4), Galectin-9 forward 5'-gtctccaggacggacttcag-3' (SEQ ID No: 5), Galectin-9 reverse 5'-caggaagcagaggtcaaagg-3' (SEQ ID No: 6), GAPDH forward 5'- gtcagtggtggacctgacct-3' (SEQ ID No: 7), GAPDH reverse 5'- tgctgtagccaaattcgttg-3' (SEQ ID No: 8). A cut-off was defined for each gene dividing the AML patient cohort into low versus high expressors. Gene expression was correlated to patient's demographic (age, karyotype, FLT3 mutation status) and clinical survival data using multivariate cox regression. Statistical analyses were done with SPSS 17 (SPSS Inc, Chicago, IL).

### Protein expression of PVR, PVRL2 and Galectin-9

Protein expression of PVR and PVRL2 was analyzed in AML cell lines and primary AML cells in flow cytometry (FACSCalibur and CellQuestPro Software, BD Biosciences) using the following antibodies: mouse anti-PVR clone D171 (Thermo Scientific™ Lab Vision, Waltham, MA) and mouse anti-PVRL2 clone L14 (Bottino et al, J Exp Med 2003;198:557-567) as primary antibodies and anti-mouse APC antibody as secondary antibody. Galectin-9 was stained with the directly APC-labelled mouse anti-human antibody (clone 9M1-3; Biozol, Eching, Germany).

### T-cell induced AML cell lysis

Buffy coats from healthy donors were used as T-cell source. The mononuclear cell (MNC) fraction was isolated using Ficoll-Paque centrifugation. AML cells were pre-stained with Cell Tracker™ Green CMFDA Dye (LifeTechnologies) for one hour and washed twice with cell culture medium. The pre-stained AML target cells were mixed with the T-cell containing MNC fraction in a ratio of 1:6 and plated in a 96-well plate (200.000 cells per well).
The cell mixture was pre-incubated with the PVR blocking antibody clone D171 (4-20 µg/ml; Thermo Scientific™ Lab Vision), the PVRL2 blocking antibody clone L14 (5-20 µl cell culture supernatant; Bottino et al, J Exp Med 2003;198:557-567), the Galectin-9 blocking antibody 9M3-1 (10-50 µg/ml; Biozol, Eching, Germany) or without antibody addition for 2 hours. After 2 hours, 100-500 pg/ml AMG330 were added to the culture.
After 24 hours, the cell mixture was stained with 7-AAD and analyzed via flow cytometry using the FACSCalibur and CellQuestPro Software (BD Biosciences). The AML target cells were gated based on the CellTracker™ Green CMFDA Dye staining. The killing ratio was determined as proportion of 7-AAD positive cells within the target cell gate.

## Claims

1. A pharmaceutical composition comprising an inhibitor against CD112 (Nectin-2, PVRL2), CD155 (PVR), and/or TIGIT for use in the treatment of acute myeloid leukemia (AML), wherein the inhibitor is an antibody which specifically binds to CD112, CD155 and/or TIGIT, and wherein the pharmaceutical composition further comprises a bispecific single chain Fv (scFv) antibody construct that engages T cells and comprises a CD3epsilon binding domain and a further binding domain targeting a surface molecule selected from the group consisting of CD33, CD19 and Flt3.

2. The pharmaceutical composition for use according to claim 1, wherein said inhibitor against CD112 inhibits the interaction between CD112 and TIGIT or wherein said inhibitor against CD155 inhibits the interaction between CD155 and TIGIT, or wherein said inhibitor against TIGIT inhibits the interaction between TIGIT and CD112, or wherein said inhibitor against TIGIT inhibits the interaction between TIGIT and CD155.

3. The pharmaceutical composition for use of according to claim 1, wherein said inhibitor against CD112 modulates intracellular signaling of CD112.

4. The pharmaceutical composition for use of according to claim 1, wherein said inhibitor against CD155 modulates intracellular signaling of CD155.

5. The pharmaceutical composition for use of according to claim 1, wherein said inhibitor against TIGIT modulates intracellular signaling of TIGIT.

6. The pharmaceutical composition for use of according to claim 1, wherein the antibody construct that engages T cells and comprises a CD3epsilon binding domain and a further binding domain targeting a surface molecule selected from the group consisting of CD33, CD19 and Flt3 is selected from the molecules depicted in SEQ ID Nos.: 15 to 37.

## Patentansprüche

1. Pharmazeutische Zusammensetzung, umfassend einen Inhibitor gegen CD112 (Nectin-2, PVRL2), CD155 (PVR) und/oder TIGIT, zur Verwendung bei der Behandlung von akuter myeloischer Leukämie (AML), wobei es sich bei dem Inhibitor um einen Antikörper handelt, der an CD112, CD155 und/oder TIGIT spezifisch bindet, und wobei die pharmazeutische Zusammensetzung ferner ein bispezifisches Einzelkette-Fv(scFv)-Antikörperkonstrukt umfasst, das T-Zellen verpflichtet und eine CD3epsilon-Bindungsdomäne und eine weitere Bindungsdomäne, die auf ein Oberflächenmolekül, ausgewählt aus der Gruppe bestehend aus CD33, CD19 und Flt3 zielt, umfasst.

2. Pharmazeutische Zusammensetzung zur Verwendung nach Anspruch 1, wobei der Inhibitor gegen CD112 die Wechselwirkung zwischen CD112 und TIGIT hemmt oder wobei der Inhibitor gegen CD155 die Wechselwirkung zwischen CD155 und TIGIT hemmt oder wobei der Inhibitor gegen TIGIT die Wechselwirkung zwischen TIGIT und CD112 hemmt oder wobei der Inhibitor gegen TIGIT die Wechselwirkung zwischen TIGIT und CD155 hemmt.

3. Pharmazeutische Zusammensetzung zur Verwendung nach Anspruch 1, wobei der Inhibitor gegen CD112 intrazelluläre Signalgebung von CD112 moduliert.

4. Pharmazeutische Zusammensetzung zur Verwendung nach Anspruch 1, wobei der Inhibitor gegen CD155 intrazelluläre Signalgebung von CD155 moduliert.

5. Pharmazeutische Zusammensetzung zur Verwendung nach Anspruch 1, wobei der Inhibitor gegen TIGIT intrazelluläre Signalgebung von TIGIT moduliert.

6. Pharmazeutische Zusammensetzung zur Verwendung nach Anspruch 1, wobei das Antikörperkonstrukt, das T-Zellen verpflichtet und eine CD3epsilon-Bindungsdomäne und eine weitere Bindungsdomäne, die auf ein Oberflächenmolekül, ausgewählt aus der Gruppe bestehend aus CD33, CD19 und Flt3 zielt, umfasst, aus den unter SEQ ID Nr.: 15 bis 37 dargestellten Molekülen ausgewählt ist.

## Revendications

1. Composition pharmaceutique comprenant un inhibiteur contre la CD112 (nectine-2, PVRL2), la CD155 (PVR), et/ou la TIGIT pour une utilisation dans le traitement de la leucémie myéloïde aiguë (LMA), l'inhibiteur étant un anticorps qui se lie spécifiquement à la CD112, à la CD155 et/ou à la TIGIT, et la composition pharmaceutique comprenant en outre un construit d'anticorps Fv à chaîne unique (scFv) bispécifique qui engage des cellules T et comprend un domaine de liaison CD3epsilon et un domaine de liaison supplémentaire ciblant une molécule de surface choisie dans le groupe constitué par CD33, CD19 et Flt3.

2. Composition pharmaceutique pour une utilisation selon la revendication 1, ledit inhibiteur contre la CD112 inhibant l'interaction entre la CD112 et la TIGIT ou ledit inhibiteur contre la CD155 inhibant l'interaction entre la CD155 et la TIGIT, ou ledit inhibiteur contre la TIGIT inhibant l'interaction entre la TIGIT et la CD112, ou ledit inhibiteur contre la TIGIT inhibant l'interaction entre la TIGIT et la CD155.

3. Composition pharmaceutique pour une utilisation selon la revendication 1, ledit inhibiteur contre la CD112 modulant la signalisation intracellulaire de la CD112.

4. Composition pharmaceutique pour une utilisation selon la revendication 1, ledit inhibiteur contre la CD155 modulant la signalisation intracellulaire de la CD155.

5. Composition pharmaceutique pour une utilisation selon la revendication 1, ledit inhibiteur contre la TIGIT modulant la signalisation intracellulaire de la TIGIT.

6. Composition pharmaceutique pour une utilisation selon la revendication 1, le construit d'anticorps qui engage des cellules T et comprend un domaine de liaison CD3epsilon et un domaine de liaison supplémentaire ciblant une molécule de surface choisie dans le groupe constitué par CD33, CD19 et Flt3 étant choisi parmi les molécules décrites dans les SEQ ID n° : 15 à 37.
